# EUROPÄISCHE PATENTSCHRIFT

(11) **EP 1 681 565 B1**
(45) Veröffentlichungstag und Bekanntmachung des Hinweises auf die Patenterteilung: **06.07.2011**
(21) Anmeldenummer: 06000726.7
(22) Anmeldetag: 13.01.2006
(51) Int. Cl.: G01N 33/50, C07K 14/47, C12N 15/62

(54) **Zellulärer RhoGTPase Aktivierungs-Assay**
Cellular RhoGTPase activation assay
Essai d'activation cellulaire de la GTPase Rho

(30) Priorität: 14.01.2005 DE 102005001885; 14.01.2005 US 644312 P
(43) Veröffentlichungstag der Anmeldung: 19.07.2006
(73) Patentinhaber: Abbott GmbH & Co. KG, 65205 Wiesbaden (DE)
(72) Erfinder: Teusch, Nicole, 64683 Einhausen (DE); Mezler, Mario, 67146 Deidesheim (DE)
(74) Vertreter: Reitstötter - Kinzebach

(56) Entgegenhaltungen:
- WO-A-2004/085648
- WO-A-2004/087744
- MI S ET AL: "LINGO-1 IS A COMPONENT OF THE NOGO-66 RECEPTOR/P75 SIGNALING COMPLEX" NATURE NEUROSCIENCE, NATURE AMERICA, INC, US, Bd. 7, Nr. 3, März 2004 (2004-03), Seiten 221-228, XP001183275 ISSN: 1097-6256
- YAMASHITA TOSHIHIDE ET AL: "Neurotrophin binding to the p75 receptor modulates Rho activity and axonal outgrowth" NEURON, Bd. 24, Nr. 3, November 1999 (1999-11), Seiten 585-593, XP002381427 ISSN: 0896-6273
- YAMASHITA TOSHIHIDE ET AL: "The p75 receptor transduces the signal from myelin-associated glycoprotein to Rho" THE JOURNAL OF CELL BIOLOGY, ROCKEFELLER UNIVERSITY PRESS, US, Bd. 157, Nr. 4, 13. Mai 2002 (2002-05-13), Seiten 565-570, XP002295646 ISSN: 0021-9525
- WANG K C ET AL: "P75 INTERACTS WITH THE NOGO RECEPTOR AS A CO-RECEPTOR FOR NOGO, MAG AND OMGP" NATURE, NATURE PUBLISHING GROUP, LONDON, GB, Bd. 420, Nr. 6911, 7. November 2002 (2002-11-07), Seiten 74-78, XP001183135 ISSN: 0028-0836
- SCHWEIGREITER R ET AL: "Versican V2 and the central inhibitory domain of Nogo-A inhibit neurite growth via p75<NTR>/NgR-independent pathways that converge at RhoA" MOLECULAR AND CELLULAR NEUROSCIENCES, SAN DIEGO, US, Bd. 27, Nr. 2, 1. Oktober 2004 (2004-10-01), Seiten 163-174, XP004599339 ISSN: 1044-7431
- MCGEE A W ET AL: "The Nogo-66 receptor: focusing myelin inhibition of axon regeneration" TRENDS IN NEUROSCIENCE, ELSEVIER, AMSTERDAM, NL, Bd. 26, Nr. 4, April 2003 (2003-04), Seiten 193-198, XP004418152 ISSN: 0166-2236
- HU F ET AL: "Regulating axon growth within the postnatal central nervous system" SEMINARS IN PERINATOLOGY, W.B. SAUNDERS, GB, Bd. 28, Nr. 6, Dezember 2004 (2004-12), Seiten 371-378, XP004689705 ISSN: 0146-0005
- HUNT D ET AL: "The Nogo receptor, its ligands and axonal regeneration in the spinal cord; a review" JOURNAL OF NEUROCYTOLOGY, CHAPMAN AND HALL, GB, Bd. 31, Nr. 2, Februar 2002 (2002-02), Seiten 93-120, XP002321234 ISSN: 0300-4864
- FOURNIER A ET AL: "Identification of a receptor mediating Nogo-66 inhibition of axonal regeneration" NATURE, NATURE PUBLISHING GROUP, LONDON, GB, Bd. 409, Nr. 6818, 18. Januar 2001 (2001-01-18), Seiten 341-346, XP000926532 ISSN: 0028-0836
- DATABASE UniProt [Online] 1. Juni 2001 (2001-06-01), "Reticulon-4 receptor precursor (Nogo receptor) (NgR) (Nogo-66 receptor)." XP002381774 gefunden im EBI accession no. UNIPROT:Q9BZR6 Database accession no. Q9BZR6

## Beschreibung

Die Erfindung betrifft einen zellulären RhoGTPase Aktivierungs-Assay, basierend auf der Bestimmung von Veränderungen im Actin-Cytoskelett (Actin Cytoskeletal Rearrangement (AcR)-Assay) geeignet zum Nachweis von Effektoren der Nogo-Rezeptor (NgR)-abhängigen Signaltransduktion unter Beteiligung der RhoGTPase wird, insbesondere zur Bestimmung von Effektoren der neuronalen Regeneration.

### Stand der Technik

Rho ist Mitglied der Ras-Superfamilie kleiner GTP-bindender Proteine. Wie Ras so dient auch Rho als molekularer Schalter, der zwischen einem inaktiven GDP-gebundenen und einem aktiven, GTP-gebundenen Zustand pendelt. Diese Poteine, auch GTPasen genannt, binden und hydrolysieren Guanin-Nukleotide. Der Austausch8 von hydrolysiertem GDP durch GTP resultiert in einer Konformationsänderung der GTPasen, die die Bindung an spezifische Zielproteine, die sogenannten Effektoren erlaubt. Dieser Mechanismus ermöglicht den Rho GTPasen, zwischen einer biochemisch aktiven, GTP-gebundenen und einer inaktiven, GDP-gebundenen Konformation zu zyklisieren. Der Austausch von GDP zu GTP wird von Guaninnukleotid-Austauschfaktoren (engl.: guanine nucleotide exchance factors, GEFs) katalysiert, indem diese die Freisetzung von GDP erleichtern und die nukleotid-freie Form der GTPase transient stabilisieren. Auf der anderen Seite fördern sogenannte GTPasenaktivierende Proteine (engl.: GTPase-activating proteins, GAPs) die GTP-Hydrolyseaktivität von Rho Proteinen und führen somit zu einer raschen Umwandlung in die inaktive, GDP-gebundene Form. Aufgrund dieses empfindlich regulierten Mechanismus sind Rho GTPasen im Sinne "molekularer Schalter" in der Lage, in Gegenwart extrazellulärer Stimuli spezifische Signalantworten gezielt einzuleiten.

Insgesamt ermöglicht die spezifische Interaktion aktivierter GTPasen mit unterschiedlichen zellulären Downstream-Targets die Ausbildung eines kooperativen Signalnetzwerkes, das die Kontrolle komplexer biologischer Prozesse auf unterschiedlichen Ebenen erlaubt.

Bisher sind vierzehn verschiedene RhoGTPasen beschrieben worden (Rac1 bis 3; Cdc42; TC10; RhoA bis E, G, H und Rnd1,2; vgl. auch Übersichtsartikel von Hall und Bar-Sagi Cell 2000, 103 (2), S. 227 ff. worauf hiermit ausdrücklich Bezug genommen wird), wobei die Familienmitglieder Rac1, Cdc42 und RhoA am besten charakterisiert sind. Diese GTPasen regulieren ein breites Spektrum zellulärer Funktionen. Der Einfluss von Rac, Rho und Cdc42 auf stimulusabhängige Veränderungen des Aktin-Cytoskeletts, der die Grundlage vieler dynamischer Prozesse, wie z.B. Chemotaxis und Phagozytose bildet, stellt eine Hauptfunktion der Rho GTPasen dar.

Beispielsweise induziert aktiviertes RhoA eine Signalkaskade, welche zu signifikanten Veränderungen im Cytoskelett führt. So ist beispielsweise die Bildung von Stressfasern, eine Migration oder Zellkontraktion bei verschiedenen Zelltypen zu beobachten (vgl. Kaibuchi, K. et al., Annu. Rev. Biochem. (1999), 68, 459-486). Ein hypothetisches Modell für den diesen Veränderungen zugrundeliegenden biochemischen Mechanismus, der schließlich zu einer Aktin-Reorganisation führt, wird von Narumiya, S. et al., in FEBS Letters, 410 (1997), 68-72 beschrieben

Im zentralen Nervensystem eines Säugers scheint RhoA eine wichtige Rolle bei der Inhibition des axonalen Nervenwachstums und beim Kollabieren des neuronalen Wachstumskegels. ("growth cone collapse") zu spielen (vgl. z.B. Lee et al Nature Reviews, November 2003, Volume 2, 1-7; Schwab, M.E. Current Opinion in Neurobiology (2004), 14, 118-124; Niederöst, B. et al., J. Neurosci. (2002), 22 (23), 10368-10376). Die genauen zellulären Signalübertragungsmechanismen, mit deren Hilfe Rho aktiviert und die axonale Regeneration im adulten Gehirn beeinträchtigt wird, werden derzeit noch nicht vollständig verstanden. Neuere Befunde weisen aber darauf hin, dass bestimmte Komponenten der Myelinscheide, wie z.B. Nogo66 (ein 66 Aminosäuren umfassendes Fragment des Membranproteins NogoA), MAG (Myelin-assiziiertes Glycoprotein) oder OMgp (Oligodendrocyten Myelin Glycoprotein) das axonale Wachstum inhibieren, indem sie an ein neuronales, auf der Axonmembran verankertes Protein mit der Bezeichnung Nogo-Rezeptor (NgR) binden. Da NgR an die extrazelluläre Oberfläche über einen Lipidanker gebunden ist, ist ein Co-Rezeptor zur Kopplung an intrazelluläre Signalwege erforderlich. Kürzlich wurde der Neurotrophin-Rezeptor p75, von dem bekannt ist, dass er die Rho-Aktivierung in verschiedenen Geweben reguliert, als möglicher Co-Rezeptor für NgR identifiziert (Wang, K.C. et al., Nature (2002) 420, 74-78).

Da das adulte menschliche Gehirn nur eine begrenzte Kapazität zur neuronalen Regeneration und zur strukturellen Plastizität (d.h. die Fähigkeit einer Gruppe von Nervenfasern, die Funktion einer anderen, geschädigten Gruppe von Nervenfasem zu übernehmen) besitzt, besteht ein zunehmendes pharmazeutisches Interesse an einer Blockade der NgR-induzierten Rho-vermittelten Aktivierung der Signalkaskade, welche diese neuronale Regeneration unterbindet. Um potentielle NgR-Antagonisten zu evaluieren, wäre es wünschenswert, RhoGTP (d.h. aktiviertes Rho) nach Stimulation von NgR mit Myelin-Proteinen, wie Nogo66, zu bestimmen. Entscheidende Voraussetzung für einen derartigen Test ist aber die Erzeugung von Zelllinien, welche Rho stabil überexprimieren, da die meisten Zellen nur eine geringe endogene Expression dieser GTPase zeigen (vgl. N. Teusch, Dissertation, Die Funktion von RhoGTPasen in den von Toll-Rezeptoren induzierten Signaltransduktionswegen, 2002).

Aus dem Stand der Technik ist der RBD-Assay bekannt, welcher die Detektion von aktivem Rho durch Affinitätspräzipitation mit der Rho-bindenden Domäne des Effektorproteins Rhotekin (RBD) ermöglicht. Dieser Assay ist für einen hohen Durchsatz nicht geeignet, da zahlreiche zeitaufwändige Arbeitsschritte, wie die Herstellung eines Zellhomogenats nach Aktivierung, Präzipitation von aktiviertem Rho und Nachweis des präzipitierten Proteins, umfasst. Außerdem sind die damit erzielbaren Ergebnisse schwer reproduzierbar und schlecht quantifizierbar; wodurch eine pharmakologische Charakterisierung von chemischen Leitstrukturen oder Antikörpern praktisch unmöglich wird.

Mi, S. et al beschreiben in Nature Neuroscience, Bd. 7, Nr. 3, 2004, 221 - 228 eine rekombinante Zelllinie COS-7, transfiziert mit p75, NgR1 und Lingo-1.

Yamashita, T. et al beschreiben in Neuron, Bd. 24, 1999, 585 - 593 mit Rho und p75 cotransfizierte 293 Zellen.

Es ist deshalb Aufgabe der vorliegenden Erfindung, ein verbessertes Testverfahren zur Bestimmung von Effektoren der NgR-abhängigen Rho-Aktivierung bereitzustellen. Insbesondere sollte das neue Testverfahren schneller und zuverlässiger (reproduzierbarer) durchführbar sein und damit einen höheren Probendurchsatz ermöglichen und gegebenenfalls auch standardisierbar und/oder quantifizierbar sein.

### Kurze Beschreibung der Erfindung

Obige Aufgabe konnte überraschenderweise durch Bereitstellung von rekombinanten Zelllinier gemäß der Definition in den Patentansprüchen gelöst werden, welche die Fähigkeit besitzen, einen funktionalen intrazellulären Faktor, der an einem eine Veränderung im Cytoskelett der Zelle induzierenden Signalweg beteiligt ist, stabil überzuexprimieren, und außerdem nach Coexpression eines mit dem intrazellulären Faktor wechselwirkenden zellulären Rezeptors reproduzierbare und quantitativ analysierbare Veränderungen im Actin-Cytoskelett der rekombinanten Zellen zeigen.

Erfindungsgemäß werden insbesondere verschiedene, die RhoGTPase stabil exprimierende Zelllinien auf der Basis von humanen embryonalen Nierenzellen (z.B. HEK293) verwendet.

Auf dieser Basis wurde ein neuartiger RhoGTPase-Aktivierungstest (ACR-Assay) entwickelt, der auf NgR-induzierten Veränderungen im Cytoskelett basiert.

Eine überraschende Beobachtung war dabei eine erhöhte Anzahl rekombinanter Rhoexprimierender Zellen, die Veränderungen im Actin-Cytoskelett nach NgR-abhängiger Stimulation zeigten. Die Erzeugung stabil überexprimierender Rho-Zelllinien war dazu Voraussetzung, da die meisten Epithelzellen nur eine niedrige endogene Expression der GTPase zeigen.

Im Gegensatz zu herkömmlichen biochemischen Assays erlaubt der ACR-Assay Screening und Charakterisierung von chemischen Verbindungen und/ oder Biomolekülen im multi-well-Platten-Format, wobei diese Tests sowohl auf das high through-putals auch das high-content-Screening ausgelegt sein können. Insbesondere die Verwendung von high-content Screening Systemen mit Bildanalyse erlaubt die Eliminierung der aus dem Stand der Technik bekannten technischen Nachteile.

Zelllinien, welche eine RhoGTPase in Gegenwart oder Abwesenheit des nogo Rezeptors und / oder des Neurotrophin Rezeptors p75 stabil überexprimieren, waren bisher nicht bekannt. Außerdem wurde bisher die NgR-induzierte, Rho-abhängige Aktin-Cytoskelett-Umordnung, wie eine Zell-Kontraktion und -Schrumpfung in Epithelzellen nicht beschrieben, ebenso wenig wie die Implementierung eines Screening Assays auf der Grundlage dieser beobachteten zellulären Veränderungen zum Zweck des Screenings und/ oder der Charakterisierung von Wirkstoffen.

### Figurenbeschreibung

Die Erfindung wird nun unter Bezugnahme auf beiliegende Figuren näher beschrieben, dabei zeigt:

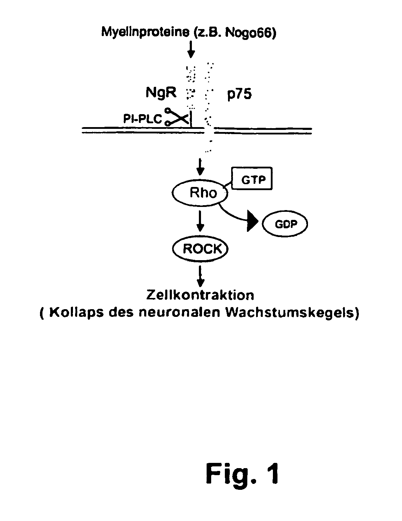

Figur 1 ein Modell der an der Inhibition des neuronalen Wachstums beteiligten Rhoabhängigen Signalkaskade, die durch den NgR/p75-Rezeptorkomplex induziert wird. Die Stimulation des NgR/p75-Rezeptorkomplexes mit Myelin-Proteinen, wie Nogo66, aktiviert die GTPase Rho welche GTP (Guanosintriphosphat) bindet. Aktivierte RhoGTPase aktiviert ihrerseits die Serin-Threonin-Kinase ROCK (Rho-assoziierte Kinase), ein spezifisches Downstream-Target von Rho. Die Aktivierung von ROCK führt zur Phosphorylierung von mehreren Cytoskelettproteinen (z.B. Myosin-light chain kinase (MLCK), CRMP2, Cofilin.; hier nicht gezeigt), resultierend in einer Veränderung der Zellmorphologie, wie insbesondere einer Zellkontraktion. Wie für ein GPI (Glycan-Phosphatidylinositol)-verknüpftes Protein zu erwarten, kann NgR von der Zellmembran durch Behandlung mit Phosphatidylinositol-spezifischer Phospholipase C (PI-PLC) freigesetzt werden.

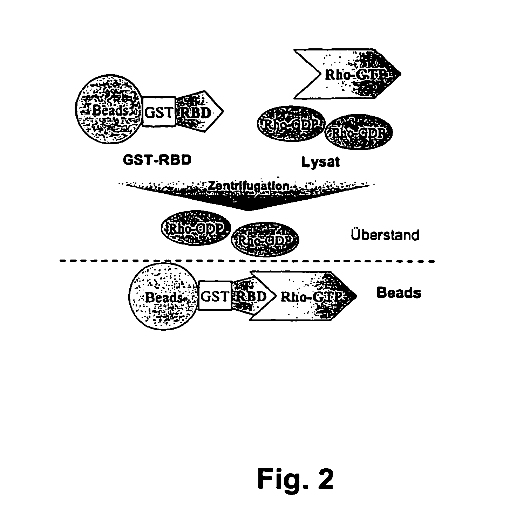

Figur 2 das Prinzip des RBD-Assays zum Nachweis der Aktivierung von niedermolekularen GTPasen durch extrazelluläre Stimuli. Dabei nutzt man aus, dass nur die GTPgebundene Form der GTPase (Rho-GTP) mit downstream-Effektoren wechselwirkt. Im Falle von Rho wird die Rho-bindende Domäne (RBD) des Effektorproteins Rhotekin zur Präzipitation von aktivem, GTP-gebundenem Rho aus Zelllysaten verwendet. GST-RBD-Beads (Polymerbeads mit über Glutathion S-Transferase verankertem RBD) werden mit Rho-haltigem Zelllysat inkubiert, nach Abzentrifugieren findet man bei Vorliegen von aktiviertem Rho-GTP im Lysat RBD-gebundenes Rho-GTP im Zentrifugat. Nicht-aktiviertes Rho (Rho-GDP) verbleibt dagegen im Überstand.

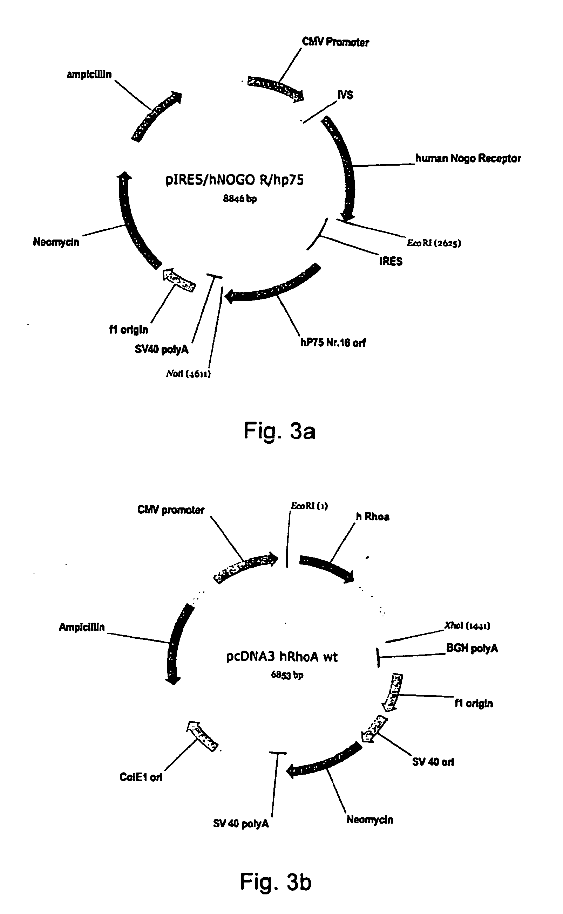

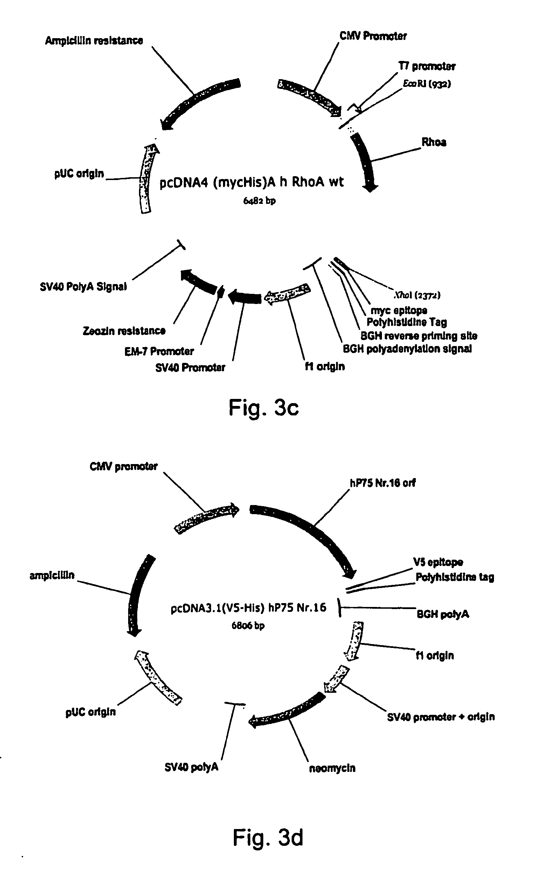

Figur 3 die Plasmidkarten von verschiedenen erfindungsgemäß verwendeten Plasmiden:
   pIRES hNgR hp75 (SEQ ID NO:10) (Fig. 3a)
   pcDNA3 hRhoA wt (SEQ ID NO:15) (Fig. 3b)
   pcDNA4 (mycHis)A hRhoA wt (SEQ ID NO:13) (Fig. 3c)
   pcDNA3.1 (V5-His)hp75 Nr. 16 (SEQ ID NO:8) (Fig. 3d)

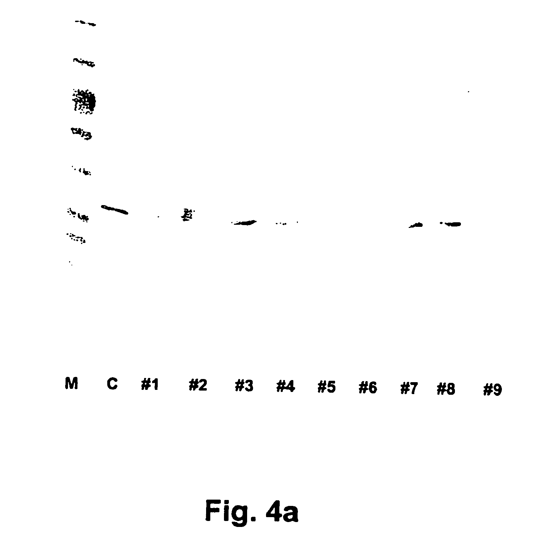

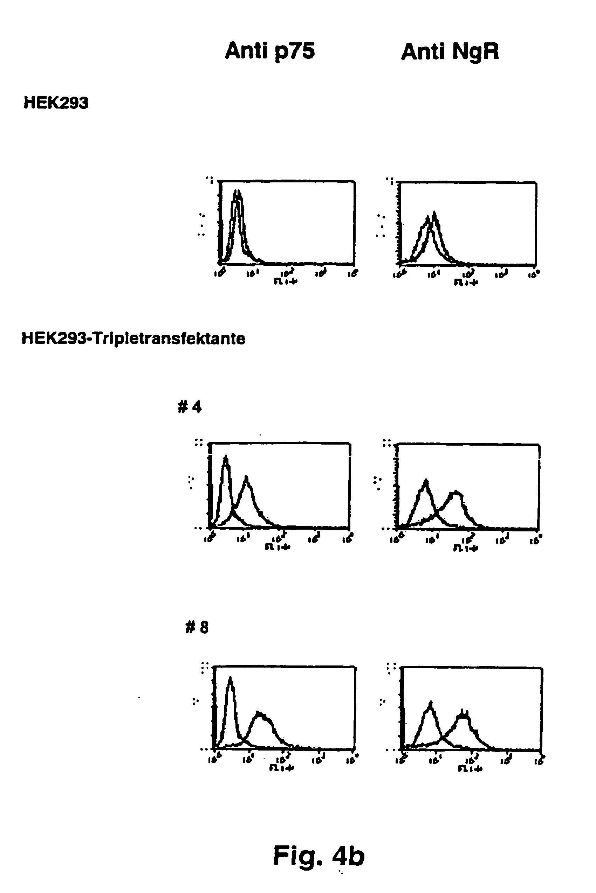

Figur 4a den immunologischen Nachweis von exprimiertem Rho durch erfindungsgemäß isolierte positive Klone (#1 bis #8) der Tripletransfektante HEK293 RhoA/NgR/p75 (Klone 1 bis 9), positive Kontrolle (C) erhalten durch transiente Transfektion mit 100 ng pcDNA4(mycHis)A hRhoA wt; als Marker (M) wurde verwendet: Benchmark Prestain Protein Ladder, Invitrogen, Bestellnummer #10748-010; Figur 4b das Ergebnis einer FACS-Analyse auf exprimierte Zelloberflächenrezeptoren hNgR und hp75 einer erfindungemäß hergestellten HEK293-Tripletransfektante (Klon #4 und #8) sowie von nichttransformierten HEK293-Zellen (oben), jeweils für Analysen mit Anti-p75- bzw. Anti-NgR- Antikörpern.

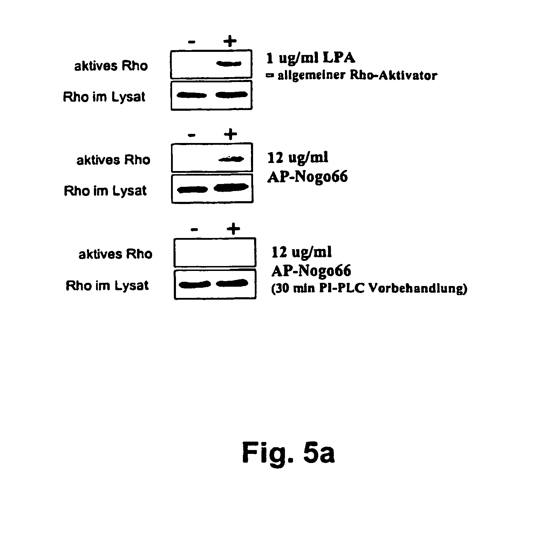

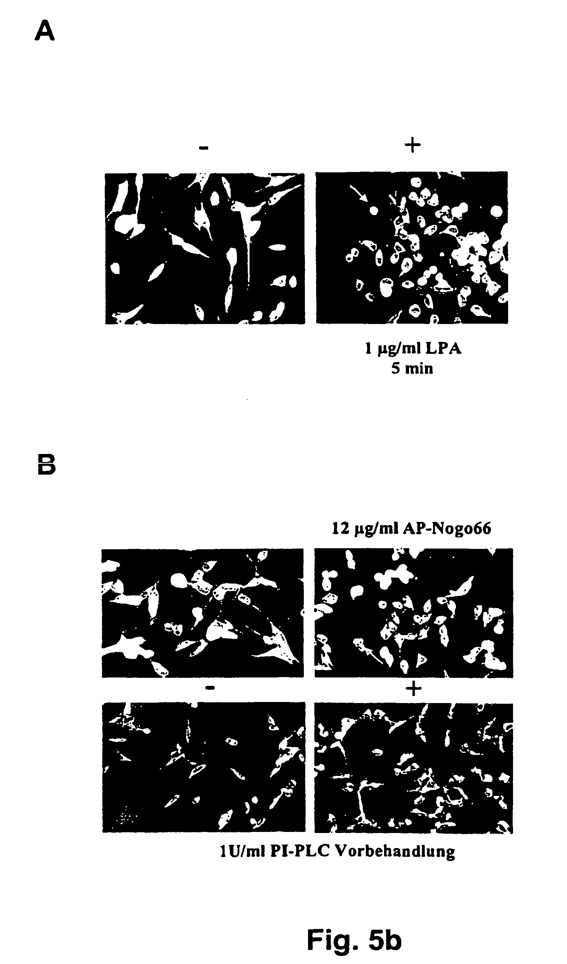

Figur 5a das Ergebnis eines RBD-Assays mit erfindungsgemäßen rekombinanten HEK-Zelllinien; Figur 5b das Ergebnis eines ACR-Assays mit erfindungsgemäßen rekombinanten HEK-Zelllinien

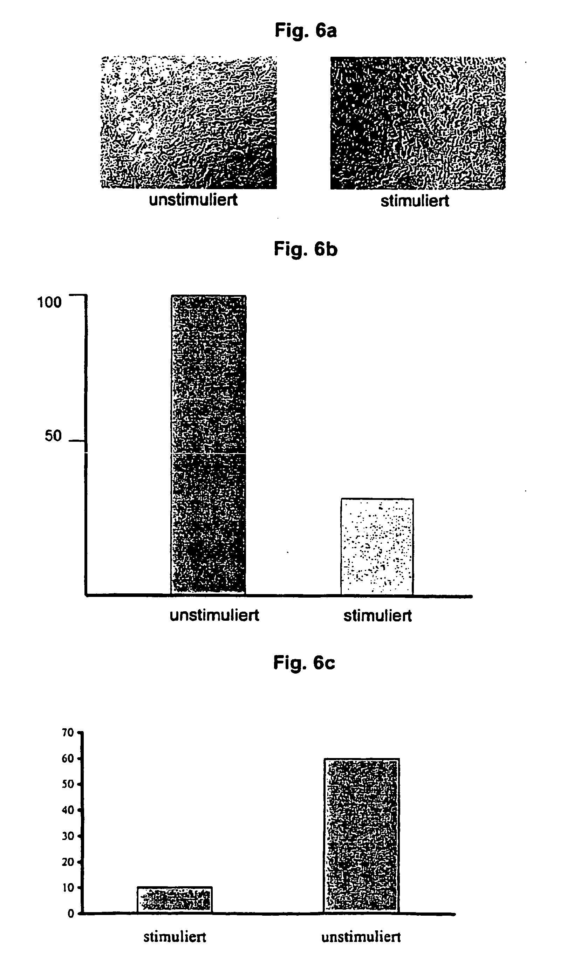

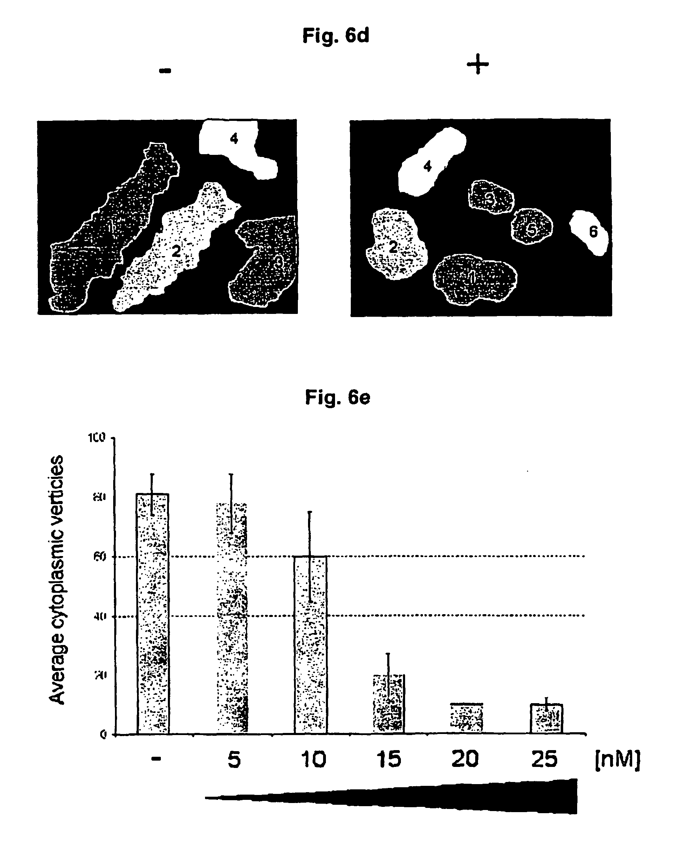

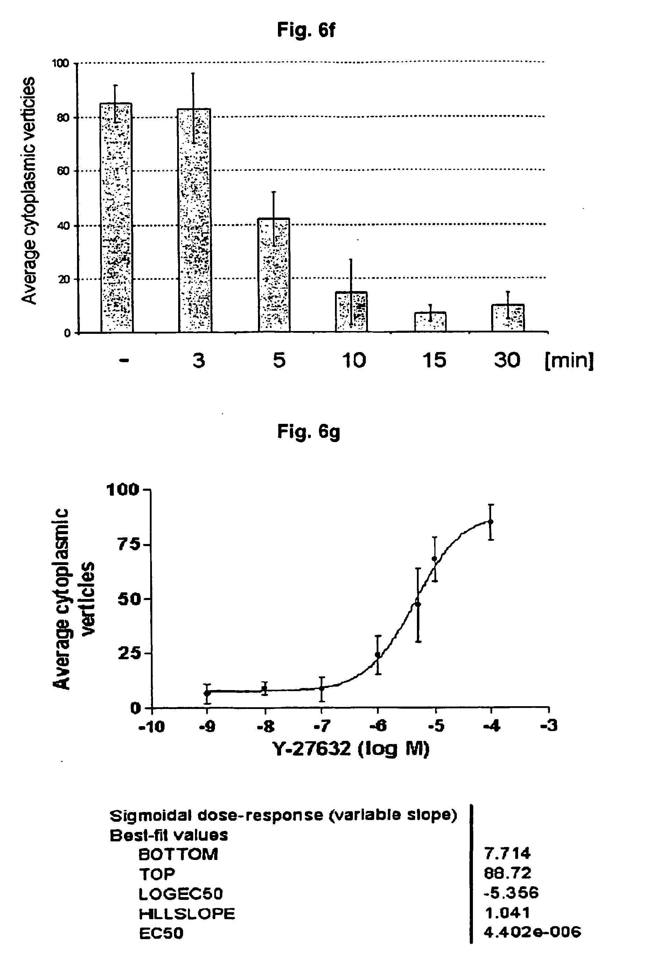

Figur 6 Ergebnisse von Auswertungen eines erfindungsgemäßen ACR-Assays durch verschiedene automatische Bildanalyseverfahren. In Figur 6a sind Fluoreszensmikrogramme stimulierter und nichtstimulierter HEK293-RhoA/p75/NgR-Zellen zu sehen. Darunter sind die zugehörigen Balkendiagramme angegeben, welches das Bildanalyse-Ergebnis darstellen, und zwar in Figur 6b die durchschnittliche prozentuale Zelllänge ermittelt mit Hilfe des Discovery-1 Systems von Molecular Devices und Analyse mit der Bildbearbeitungssoftware Metamorph, Molecular Devices; und in Figur 6c die Anzahl von "cytoplasmic verticies" (diese sind ein Maß für bzw. repräsentieren den Umfang des Aktinz-Cytoskeletts einer Zelle; dies entspricht aber nicht dem Zellumfang) ermittelt mit Hilfe des Atto Pathway HT Systems von BD Bioimages (Becton Dickinson). Figur 6 d zeigt repräsentative Abbildungen, welche eine Algorhythmus-Detektion veranschaulichen. Die Bilder wurden mit Hilfe von Pathway HT aufgenommen und die Segmentierung erfolgte mit Hilfe von Attovision. HEK293-RhoA/NgR/p75-Zellen waren entweder unstimuliert (-) oder mit 20 nM AP-Nogo66 stimuliert (+). Die angegebenen Zahlen geben die einzelnen interessierenden Bereiche (regions of interest (ROIs)) an, welche nach der Segmentierung detektiert werden. Figur 6e zeigt das Dosis-Ansprechverhalten für AP-Nogo66. HEK293-RhoA/NgR/p75-Zellen waren entweder nicht stimuliert (-) oder wurden 10 Minuten bei verschiedenen AP-Nogo66-Konzentrationen stimuliert. Aus einer Segmentierung gemäß Figur 6d wurden die Mittelwerte der cytoplasmic verticies mit Hilfe von Attovision bestimmt und deren Abhängigkeit von der AP-Novo66-Konzentration dargestellt. In ähnlicher Weise wurde der zeitliche Verlauf der Aktin-Cytoskelett-Umordnung, induziert durch AP-Novo66 dargestellt. HEK293-RhoA/NgR/p75-Zellen waren entweder unstimuliert (-) oder wurden mit 20 nM AP-Novo66 unterschiedlich lange stimuliert. Die Ergebnisse sind in Figur 6f dargestellt. Figur 6g zeigt das Dosis-Ansprechverhalten des ROCK-Inhibitors Y-27632. HEK293-RhoA/NgR/p75-Zellen wurden entweder nicht behandelt (-) oder wurden 15 Minuten bei verschiedenen Konzentrationen von Y-27632 vorbehandelt, bevor sie mit 20 nM AP-Nogo66 stimuliert wurden. Die dargestellten Ergebnisse stellen wenigstens drei unabhängige Experimente dar.

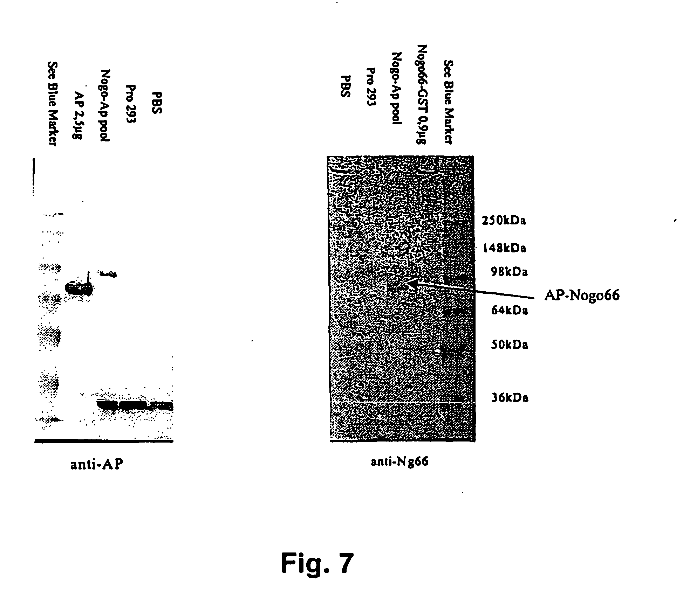

Figur 7 die Western-Analyse von AP-Nogo66 mit verschiedenen Antiseren (anti-AP; Anti-Nogo66) nach SDS-Gelelektrophorese. Pro 293 steht für die Negativkontrolle für Blot und Antikörper-Behandlung, da hier nur das verwendete Serum aufgetragen wurde.

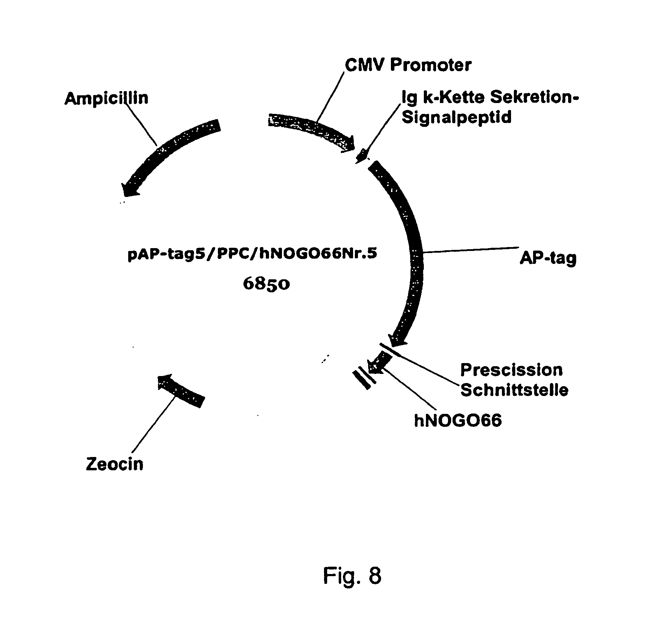

Figur 8 die Plasmidkarte des verwendeten Plasmids pAP-tag5/PPC/hNOG066Nr.5

### Detaillierte Beschreibung der Erfindung

### I. Spezielle Gegenstände der Erfindung

Spezielle Gegenstände sind in den Patentansprüchen beschrieben.

Ein erster Gegenstand betrifft die Verwendung rekombinanter Zelllinien, gemäß der Definition in Anspruch 1 zur Bestimmung von Effektoren der NgR-abhängigen Signaltransduktion über Veränderungen im Aktin-Cytoskelett von Zellen der Zelllinie.

Es werden insbesondere Zelllinien eingesetzt, welche stabil exprimieren
a) eine funktionale, intrazelluläre RhoGTPase; und /oder
b) einen mit der RhoGTPase wechselwirkenden membrangebundenen Rezeptor, wie insbesondere einen Rezeptorkomplex, umfassend:
   einen funktionalen, menbrangebundenen Neurotrophin Rezeptor p75; und
   einen funktionalen, menbrangebundenen Nogo Rezeptor (NgR);

Beispielsweise ist dabei die Rho GTPase eine RhoA GTPase.

Insbesondere werden Zelllinien eingesetzt, wobei
a) die funktionale RhoGTPase eine Aminosäuresequenz gemäß SEQ ID NO: 16,
b) der funktionale p75 eine Aminosäuresequenz gemäß SEQ ID NO:9 , und
c) der funktionale NgR eine Aminosäuresequenz gemäß SEQ ID NO:11 umfasst;
oder Zelllinien, die funktionale Äquivalente dieser konkreten Proteinfaktoren exprimeren.

Zur Herstellung erfindungsgemäßer rekombinanter Zelllinien sind insbesondere solche geeignet, die zu einer Reorganisation ihres Aktin-Cytoskeletts, induziert durch die Bindung eines Liganden an NgR, befähigt sind, wobei die Ligandenbindung z.B. eine Aktivierung von Rho induziert.

Ohne darauf beschränkt zu sein, kann ein brauchbarer Ligand Nogo-A oder ein davon abgeleitetes NgR bindendes Fragment oder Derivat umfassen.

Erfindungsgemäß Verwendete Zelllinien sind insbesondere von einer adhärenten Säugerzelllinie mit ausgespreiteter Zellmorphologie ableitbar. Beispiele hierfür sind humane Epithelial- oder Fibroblastenzelllinien.

In einer speziellen Ausführungsform sind die Verwendeten Zelllinien von einer humanen embryonalen Nieren (HEK)-Zeillinie abgeleitet.

Ein weiterer Gegenstand der Erfindung betrifft die Verwendung einer erfindungsgemäßen rekombinanten Zelllinie zur Bestimmung von Effektoren der Aktivierung der GTPase Rho.

Gegenstand der Erfindung sind weiterhin Testmethoden zur Bestimmung von Veränderungen im Cytoskelett einer Zelllinie wobei man eine rekombinante Zelllinie gemäß obiger Definition mit einem mit den Zellen wechselwirkenden Liganden kultiviert, der gegebenenfalls die Veränderung im Cytoskelett über die Induktion der NgR-abhängigen Signal transdeletion bewirkt, und Veränderungen in der Reorganisation des Cytoskeletts der so behandelten Zelllinien bestimmt. Insbesondere wird dabei ein Ligand verwendet, der mit einem membrangebunden Rezeptor wechselwirkt.

Unter einer "korrespondierende Primärzellline" versteht man in diesem Zusammenhand eine nicht rekombinante ansonsten funktional äquivalente Zelllinie, in welcher über einen der oben beschriebenen Mechanismen eine bestimmbare Veränderung in ihrem Cytoskelett induzierbar ist.

Als Primärzellen werden z.B. folgende Zellen genannt Zellen aus dem Hinterwurzelganglion der Ratte (Li & Strittmatter J Neurosci. 2003 15; 23(10):4219-27) oder Körnerzellen aus dem Cerebellum der Ratte (Madura et al. EMBO Rep. 2004; 5(4):412-7).

In einer bevorzugten Ausführungsform dieser Testmethode wird die Zelllinie in Gegenwart oder Abwesenheit eines Analyten kultiviert, von dem man vermutet, dass er einen Effektor enthält, der die Wirkung des Liganden auf die Zelle moduliert. Die Kultivierung mit dem Analyten kann dabei gleichzeitig oder zeitlich versetzt (d.h. früher oder später) zur Kultivierung mit dem rezeptorbindenen Liganden erfolgt.

Insbesondere eignet sich diese Methode als Test für Effektoren der NgR-abhängigen Aktivierung der GTPase Rho, wobei man eine rekombinante Zelllinie gemäß obiger Definition in Gegenwart eines NgR bindenden Liganden und in Gegenwart oder Abwesenheit eines Analyten kultiviert, von dem man vermutet, dass er den Effektor enthält, und Unterschiede in der Reorganisation des Cytoskeletts der so behandelten Zelllinien bestimmt.

Die erfindungsgemäße Testmethode basiert insbesondere auf einer Testauswertung, wobei man den Mittelwert wenigstens eines der folgenden Parameter in einer oder für eine repräsentative Zellpopulation bestimmt:
a) Zellumfang
b) Zelllänge
c) Zellbreite
d) Zellfläche
e) Länge der Zellausläufer
f) Zelldichte
g) cytoplasmatische Fläche
h) cytoplasmic verticies (bestimmbar nach Visualisierung des Cytoskeletts und ein von a) verschiedenes Maß für den Zellumfang)
i) Brechungsindex
j) zellfreie Fläche einer Messfläche
k) mit Zellen belegte Fläche einer Messfläche
l) eine mathematische Verknüpfung von wenigstens zwei der Parameter a) bis k), oder einen von wenigstens einem der Parameter a) bis k) ableitbaren Parameter, wie insbesondere das Verhältnis von Zelllänge zu Zellbreite, oder das Verhältnis von Zellumfang im Quadrat zu Zellfläche; oder
m) einen entsprechenden reziproken Wert eines der Parameter a) bis I).

Vorzugsweise erfolgt die Bestimmung auf der Grundlage optischen Methoden, falls erforderlich nach Durchführung einer geeigneten Anfärbung der Zellen.

Der Einsatz automatischer Bildanalyseverfahren ist dabei besonders vorteilhaft.

Derartige Testverfahren dienen vorteilhaft zur Bestimmung von Effektoren der neuronalen Regeneration.

So können z.B. Effektoren getestet werden, die die Rho-Aktivierung teilweise oder vollständig inhibieren und/oder die neuronale Regeneration fördern.

Erfindungsgemäß bevorzugt ist insbesondere die Verwendung solcher Testmethoden zur Bestimmung eines Effektors, der eine krankheits- oder verletzungsbedingte Inhibition der axonalen Regeneration und/oder einen krankheits- oder verletzungsbedingten Kollaps der des neuronalen Wachstumskegels teilweise oder vollständig neutralisiert. Derartige Effektoren sind insbesondere NgR-Antagonisten.

Das Offenbarte Zellsystem findet auch Anwendung in einem Screeningverfahren, insbesondere High-Content-Screeningverfahren für Effektoren, insbesondere NgR-Antagonisten, der neuronalen Regeneration. Erfindungsgemäß nachweisbare NgR-Antagonisten sind z.B. einsetzbar bei der Therapie folgender Erkrankungen: Rückenmarksvertetzung, Hirnverletzung, Schädel-Hirn-Trauma, Schlaganfall, mechanisch bedingter Verletzung des Zentralnervensystems, ischämisch bedingter Verletzung des Zentralnervensystems, infektionsbedingter Verletzung des Zentralnervensystems, Morbus Parkinson, Chorea Huntington, amyotropher Lateralsklerose und anderen Motoneuronenerkrankungen, Morbus Alzheimer, Poliomyelitis, Hirschsprung's Krankheit, Paraplegie, Diplegie, Tetraplegie, infantiler neuroaxonaler Dystrophie (Seitelberg-Krankheit), lokalisierter neuroaxonaler Dystrophie (Hallervorden-Spatz-Krankheit), Waller-Degeneration, viraler Meningitis, bakterieller Meningitis und Axon-Ischämie. und neurodegenerativen Erkrankungen, wie insbesondere Multiple Sklerose, post-Polio Syndrom, Spina bifida, spinale Muskelatrophie, Tumoren , wie Hirntumor und Rückenmarkstumor, und transverser Myelitis.

Gegenstand der Erfindung sind außerdem High Content Screening-Vefahren zur Bestimmung der Aktivierung der GTPase Rho, wobei man eine Testmethode nach obiger Definition anwendet.

Schließlich betrifft die Erfindung die Verwendung von Testkits, umfassend eine rekombinante Zelllinie nach obiger Defintion oder eine korrespondierende Primärzelllinie sowie einen Liganden für den zellulären Rezeptor, zur Bestimmung von Effektoren gemäß obiger Definition.

### II. Erläuterung allgemeiner Begriffe

,,Funkionale" Proteinfaktoren sind im Rahmen der Erfindung solche, die an einem Signalweg beteiligt sind, der in einer Veränderung des Cytoskeletts einer den Faktor exprimierenden eukaryotischen Zelle mündet. Ein Fehlen oder Blockieren dieses funktionalen Proteinfaktors würde die Inhibition des betreffenden Signalwegs bewirken.

Die Begriffe "Expression" oder ,,Überexpression" beschreiben im Kontext der Erfindung die Produktion bzw. Erhöhung der intrazellulären oder zellmembangebundenen Aktivität eines oder mehrerer erfindungsgemäß verwendeter Proteine, wie z.B. Rho GTPase, NgR oder p75. Der Grad der Überexpression ist z.B. durch die Kopienzahl der verwendeten Gene oder durch die Stärke des verwendeten Promotors steuerbar.

Eine ,,stabile" Expression oder Überexpression ist erreicht, wenn die rekombinante eukaryotische Zelle die Fähigkeit zur Expression oder Überexpression des Proteins dauerhaft besitzt.

,,Intrazelluläre Faktoren" sind solche, welche als nicht-membrangebundene, translozierbare, lösliche Faktoren an dem eine Veränderung im Cytoskelett der Zelle induzierenden Signalweg beteiligt sind.

,,Zelluläre Faktoren" oder "zelluläre Rezeptoren" sind solche, welche als membrangebundene, gegebenenfalls nicht-lösliche Faktoren an dem eine Veränderung im Cytoskelett der Zelle induzierenden Signalweg beteiligt sind. Sie können an der Zellmembran oberflächlich (i.e. reversibel oder irreversibel) gebunden oder über eine Transmembranregion (i.e. irreversibel) verankert sein. Sie können nach außen oder ins Zellinnere oder bidirektional orientiert sein. Ein zellulärer Faktor soll für sich oder in Kombination (im Komplex) mit einem oder mehreren weiteren gegebenenfalls membrangebundenen Faktoren dazu in der Lage sein, mit einem extrazellulären Liganden in Wechselwirkung zu treten und als Folge davon ein Bindungssignal auf den intrazellulären Teil des, eine Veränderung im Cytoskelett der Zelle induzierenden Signalwegs zu übertragen.

,,Liganden" im Sinnen der vorliegenden Erfindung sind hoch- oder niedermolekulare Moleküle, wie z.B. Proteine, Glycoproteine, Nukleinsäuren oder natürliche oder künstlich erzeugte Fragmente davon, welche zur Wechselwirkung, wie z.B. unter Ausbildung einer nicht-kovalenten Bindung, mit wenigstens einem der erfindungsgemäß verwendeten oder als brauchbar angegebenen zellulären Rezeptoren oder Corezeptoren befähigt sind.

,,Effektoren" im Sinne der vorliegenden Erfindung sind insbesondere solche, welche die Rezeptor-abhängige, die Veränderung des Cytoskeletts der Zellen bewirkende Signaltransduktion, modulieren. Effektoren können dabei insbesondere auch obige ,,Liganden" sein. ,,Modulation" ist dabei insbesondere als Abschwächung oder vollständige Unterbindung der Veränderungen in der Zellmorphologie zu verstehen. Der Effektor kann dabei ein Agonist, ein Antagonist ein inverser Agonist oder ein Partialagonist sein. Der Effektor kann dabei eine hoch- oder niedermolekulare chemische Verbindung sein die aus einer natürlichen Quelle isoliert oder chemisch oder biochemisch synthetisiert wurde. Der Effektor kann aber auch ein Biomolekül, wie z.B. ein Antikörper oder ein antigenbindendes Fragmente davon, sein. Weitere Effektoren können auch Membranproteine oder lösliche Fragmente davon sein. Außerdem sind Polysaccharide, Wachstumsfaktoren, Proteoglykane, Semaphorine und Ephrine potentielle Effektoren.

Ein ,,Analyt" ist eine zu untersuchende Probe, enthaltend ein natürliches oder künstliches Stoffgemisch oder eine Reinsubstanz. In dem Analyten vermutet man des Vorliegen eines Effektors des oben beschriebenen Typs.

,,High-content screening" (HCS) steht für Nachweisverfahren das analytische Informationen mit große Dichte oder Komplexität liefert. Im allgemeinen sind hiermit funktionelle, vor allem aber zelluläre Screening-Verfahren gemeint, die mit Hilfe eines automatisierten Mikroskopiersystems durchgeführt und mit Hilfe von automatisierten Bildbearbeitungssystemen/Algorithmen analysiert und ausgewertet werden.

### III. Weitere Angaben zur Ausführung der Erfindung

### a) Proteine

Als bevorzugte, im Rahmen der Erfindung angewendete Proteine sind zu nennen: hNgR (SEQ ID NO:11) (membrangebundener Rezeptor) (NM_023004) hp75 (SEQ ID NO:9 oder 12) (membrangebundener Co-Rezeptor) (NM_002507) hRhoAGTPase (SEQ ID NO:14 oder 16) (intrazellulärer Faktor) (NM_001664) AP-Nogo66 (Rezeptorligand) (NogoA: NM_020532, NP_065393) Als Alternativen zu RhoA sind zu nennen die GTPasen Rac1 bis 3 (Brandtlow C Exp Gerontol. 2003;38(1-2):79-86) ; Cdc42; TC10; RhoB bis E, G, H. (Tanaka et al. EMBO J. 2004 10;23(5):1075-88)

Als Alternativen zu NgR sind zu nennen Neogenin (Rezeptor für RGM = Repulsive Guidance Molecule), alle Semaphorinrezeptoren, wie z.B. Plexin, Neuropilin, alle Ephrinrezeptoren, wie z.B. Eph A4 (Rajagopalan et al. Nat Cell Biol. 2004 Aug;6(8):756-62), (Moreno-Flores et al. Mol Cell Neurosci. 2002;20(3):429-46)..( Hanbali et al. J Neurochem. 2004;90(6):1423-31).

Als Alternativen zu p75 sind zu nennen:
- die Nervenwachstumsfaktorrezeptoren TrkA, TrkB, TrkC; (Williams et al. J Biol Chem. 2004 30)
- Purinrezeptoren, die RhoGTPasen aktivieren können, wie z.B. P2X7 (Wang et al. Nat Med. 2004;10(8):821-7)
- Sortilin, (Bronfman et al. EMBO Rep. 2004;5(9):867-71.)
- alle Proteine der Lingo Familie, wie z.B. Lingo1, Lingo 2, Lingo3 (Mi et al. Nat Neurosci. 2004;7(3):221-8).

Als Alternativen zu AP-Nogo66 sind Faktoren zu nennen, welche zur Bindung an NgR befähigt sind: Nogo-A, MAG, Omgp und Fragmente davon, wie NEP1-40; anti-NgR-Antikörper, anti-p75-Antikörper und bindene Fragmente davon; anders getaggtes Nogo66 wie z.B. GST-Nogo66, His-Nogo66, Semaphorine, Ephrine, Chrondoitin Sulfat Proteoglykane (CSPGs), RGM, Tenascine, Nervenwachstumsfaktoren (z.B. NGF, BDNF, NT-3/4, GDNF).

Die vorliegende Erfindung ist aber nicht auf die Verwendung der konkret unter Angabe von Sequenzen beschriebenen Proteinfaktoren (zur Etablierung einer rekombinanten Zelllinie als Grundlage für die Durchführung eines ACR-Assays) oder Liganden (zur Induzierung der intrazellulären Signalkaskade in der etablierten Zelllinie) beschränkt.

Erfindungsgemäß mit umfasst sind ebenfalls ,,funktionale Äquivalente" der konkret offenbarten Proteine/Polypeptide.

,,Funktionale Äquivalente" oder Analoga der konkret offenbarten Polypeptide sind im Rahmen der vorliegenden Erfindung davon verschiedene Polypeptide, welche weiterhin die gewünschte biologische Aktivität, wie z.B. Rezeptor- oder Ligandenbindungsvermögen oder GTPase-Aktivität, besitzen.

Unter "funktionalen Äquivalenten" versteht man erfindungsgemäß insbesondere Mutanten, welche in wenigstens einer der Sequenzpositionen der oben genannten konkreten Sequenzen eine andere als die konkret genannte Aminosäure aufweisen aber trotzdem noch die gewünschte biologische Aktivität besitzen. "Funktionale Äquivalente" umfassen somit die durch eine oder mehrere, wie z.B. 1 bis 10, Aminosäure-Additionen, - Substitutionen, -Deletionen und/oder -Inversionen erhältlichen Mutanten, wobei die genannten Veränderungen in jeglicher Sequenzposition auftreten können, solange sie zu einer Mutante mit dem erfindungsgemäß gewünschten Eigenschaftsprofil führen. Funktionale Äquivalenz ist insbesondere auch dann gegeben, wenn die Aktivitätsmuster zwischen Mutante und unverändertem Polypeptid qualitativ übereinstimmen, d.h. beispielsweise gleiche Substrate mit unterschiedlicher Geschwindigkeit umgesetzt werden oder gleiche Liganden unterschiedlich stark binden. Beispielsweise können N-terminale Met-Gruppen der oben genannten Sequenzen deletiert oder durch eine andere Aminosäure substituiert sein.

"Funktionale Äquivalente" im obigen Sinne sind auch Präkursoren der beschriebenen Polypeptide sowie funktionale Derivate und Salze der Polypeptide. Unter dem Ausdruck ,,Salze" versteht man sowohl Salze von Carboxylgruppen als auch Säureadditionssalze von Aminogruppen der offenbarten Proteinmoleküle. Salze von Carboxylgruppen können in an sich bekannter Weise hergestellt werden und umfassen anorganische Salze, wie zum Beispiel Natrium-, Calcium-, Ammonium-, Eisen- und Zinksalze, sowie Salze mit organischen Basen, wie zum Beispiel Aminen, wie Triethanolamin, Arginin, Lysin, Piperidin und dergleichen. Säureadditionssalze, wie zum Beispiel Salze mit Mineralsäuren, wie Salzsäure oder Schwefelsäure und Salze mit organischen Säuren, wie Essigsäure und Oxalsäure sind ebenfalls Gegenstand der Erfindung.

,,Funktionale Derivate" offenbarter Polypeptide können an funktionellen Aminosäure-Seitengruppen oder an deren N- oder C-terminalen Ende mit Hilfe bekannter Techniken ebenfalls hergestellt werden. Derartige Derivate umfassen beispielsweise aliphatische Ester von Carbonsäuregruppen, Amide von Carbonsäuregruppen, erhältlich durch Umsetzung mit Ammoniak oder mit einem primären oder sekundären Amin; N-Acylderivate freier Aminogruppen, hergestellt durch Umsetzung mit Acylgruppen; oder O-Acylderivate freier Hydroxygruppen, hergestellt durch Umsetzung mit Acylgruppen.

"Funktionale Äquivalente" umfassen natürlich auch Polypeptide welche aus anderen Organismen zugänglich sind, sowie natürlich vorkommende Varianten. Beispielsweise lassen sich durch Sequenzvergleich Bereiche homologer Sequenzregionen festlegen und in Anlehnung an die konkreten Vorgaben der Erfindung äquivalente Proteine ermitteln.

"Funktionale Äquivalente" umfassen ebenfalls Fragmente, vorzugsweise einzelne Domänen oder Sequenzmotive, der offenbarten Polypeptide, welche z.B. die gewünschte biologische Funktion aufweisen.

,,Funktionale Äquivalente" sind außerdem Fusionsproteine, welche ein der oben genannten Polypeptidsequenzen oder davon abgeleitete funktionale Äquivalente und wenigstens eine weitere, davon funktionell verschiedene, heterologe Sequenz in funktioneller, N- oder C-terminaler Verknüpfung (d.h. ohne gegenseitigen wesentliche funktionelle Beeinträchtigung der Fusionsproteinteile) aufweisen. Nichtlimitiernde Beispiele für derartige heterologe Sequenzen sind z.B. Signalpeptide, Enzyme, Immunoglobuline, Oberflächenantigene, Rezeptoren oder Rezeptorliganden.

Erfindungsgemäß mit umfasste "funktionale Äquivalente" können wenigstens 60 %, wie wenigstens 75% ins besondere wenigsten 85 %, wie z.B. 90%, 95, 96, 97, 98 oder 99%, Homologie zu einer der konkret offenbarten Sequenzen, berechnet nach dem Algorithmus von Pearson und Lipman, Proc. Natl. Acad, Sci. (USA) 85(8), 1988, 2444-2448 aufweisen. Eine prozentuale Homologie eines offenbarten homologen Polypeptids bedeutet insbesondere prozentuale Identität der Aminosäurereste bezogen auf die Gesamtlänge einer der hierin konkret beschriebenen Aminosäuresequenzen.

Im Falle einer möglichen Proteinglykosylierung umfassen offenbarte funktionale Äquivalente auch Proteine des oben bezeichneten Typs in deglykosylierter bzw. glykosylierter Form sowie durch Veränderung des Glykosylierungsmusters erhältliche, abgewandelte Formen.

Homologe der erfindungsgemäß verwendeten Proteine oder Polypeptide können durch Mutagenese erzeugt werden, z.B. durch Punktmutation, Verlängerung oder Verkürzung des Proteins.

Homologe der erfindungsgemäß verwendeten Proteine können durch Screening kombinatorischer Banken von Mutanten, wie z.B. Verkürzungsmutanten, identifiziert werden. Beispielsweise kann eine variegierte Bank von Protein-Varianten durch kombinatorische Mutagenese auf Nukleinsäureebene erzeugt werden, wie z.B. durch enzymatisches Ligieren eines Gemisches synthetischer Oligonukleotide. Es gibt eine Vielzahl von Verfahren, die zur Herstellung von Banken potentieller Homologer aus einer degenerierten Oligonukleotidsequenz verwendet werden können. Die chemische Synthese einer degenerierten Gensequenz kann in einem DNA-Syntheseautomaten durchgeführt werden, und das synthetische Gen kann dann in einen geeigneten Expressionsvektor ligiert werden. Die Verwendung eines degenerierten Gensatzes ermöglicht die Bereitstellung sämtlicher Sequenzen in einem Gemisch, die den gewünschten Satz an potentiellen Proteinsequenzen codieren. Verfahren zur Synthese degenerierter Oligonukleotide sind dem Fachmann bekannt (Z.B. Narang, S.A. (1983) Tetrahedron 39:3; Itakura et al. (1984) Annu. Rev. Biochem. 53:323; Itakura et al., (1984) Science 198:1056; lke et al. (1983) Nucleic Acids Res. 11:477).

### b) Nukleinsäuren

Alle hierin erwähnten Nukleinsäuresequenzen (einzel- und doppelsträngige DNA- und RNA-Sequenzen, wie z.B. cDNA und mRNA) sind in an sich bekannter Weise durch chemische Synthese aus den Nukleotidbausteinen, wie beispielsweise durch Fragmentkondensation einzelner überlappender, komplementärer Nukleinsäurebausteine der Doppelhelix herstellbar. Die chemische Synthese von Oligonukleotiden kann beispielsweise, in bekannter Weise, nach der Phosphoamiditmethode (Voet, Voet, 2. Auflage, Wiley Press New York, Seiten 896-897) erfolgen. Die Anlagerung synthetischer Oligonukleotide und Auffüllen von Lücken mit Hilfe des Klenow-Fragmentes der DNA-Polymerase und Ligationsreaktionen sowie allgemeine Klonierungsverfahren werden in Sambrook et al. (1989), Molecular Cloning: A laboratory manual, Cold Spring Harbor Laboratory Press, beschrieben.

Die offenbarten Nukleinsäuremoleküle können zudem untranslatierte Sequenzen vom 3'- und/oder 5'-Ende des kodierenden Genbereichs enthalten

Der Begriff ,,isolierte Nukleinsäuresequenz " steht für eine Gensequenz in einem nicht natürlich vorkommenden Zustand. Allgemein bedeutet dies, dass sie aus ihrem natürlichen Zustand isoliert wurde oder in einer nicht natürlich vorkommenden Umgebung synthetisiert oder hergestellt wurde. Insbesondere umfasst dies Nukleinsäuremoleküle, die *in vitro* gebildet oder erhalten wurden; einschließlich genomische DNA-Fragmente, rekombinante oder synthetische Moleküle sowie Nukleinsäuren in Kombination mit heterologen Nukleinsäuren. Der Begriff erstreckt sich auch auf die genomische DNA , cDNA, RNA oder Teile davon. Die hier betrachteten Nukleinsäuresequenzen umfassen auch Oligonukleotide, die sich als genetische Sonden eignen und z.B. zur Identifizierung kodierender Sequenzen funktional äquivalenter, erfindungsgemäß verwendbarer Proteinfaktoren geeignet sind.

Ein "isoliertes" Nukleinsäuremolekül wird von anderen Nukleinsäuremolekülen abgetrennt, die in der natürlichen Quelle der Nukleinsäure zugegen sind und kann überdies im wesentlichen frei von anderem zellulären Material oder Kulturmedium sein, wenn es durch rekombinante Techniken hergestellt wird, oder frei von chemischen Vorstufen oder anderen Chemikalien sein, wenn es chemisch synthetisiert wird.

Der Begriff "Gen" oder "Gensequenzen" wird hier in seiner allgemeinsten Bedeutung verwendet und umfasst alle aufeinander folgenden Reihen von Nukleotidbasen, die direkt oder über eine komplementäre Basenreihe für die Aminosäuresequenz eines erfindungsgemäß verwendbaren Proteins, insbesondere RhoGTPasen und damit wechselwirkende zelluläre Rezeptoren, kodieren. Solche Aminosäuresequenzen können ein full-length Protein oder eine aktive verkürzte Form davon sein oder sie können einem bestimmten Bereich, wie einem N-terminalen, C-terminalen oder einem inneren Bereich des Proteins entsprechen.

Ein offenbartes Nukleinsäuremolekül kann mittels molekularbiologischer Standard-Techniken und der erfindungsgemäß bereitgestellten Sequenzinformation isoliert werden. Beispielsweise kann cDNA aus einer geeigneten cDNA-Bank isoliert werden, indem eine der konkret offenbarten vollständigen Sequenzen oder ein Abschnitt davon als Hybridisierungssonde und Standard-Hybridisierungstechniken (wie z.B. beschrieben in Sambrook, J., Fritsch, E.F. und Maniatis, T. Molecular Cloning: A Laboratory Manual. 2. Aufl., Cold Spring Harbor Laboratory, Cold Spring Harbor Laboratory Press, Cold Spring Harbor, NY, 1989) verwendet werden. Überdies lässt sich ein Nukleinsäuremolekül, umfassend eine der offenbarten Sequenzen oder ein Abschnitt davon, durch Polymerasekettenreaktion isolieren, wobei die Oligonukleotidprimer, die auf der Basis dieser Sequenz erstellt wurden, verwendet werden. Die so amplifizierte Nukleinsäure kann in einen geeigneten Vektor kloniert werden und durch DNA-Sequenzanalyse charakterisiert werden. Die erfindungsgemäß verwendeten Oligonukleotide können ferner durch Standard-Syntheseverfahren, z.B. mit einem automatischen DNA-Synthesegerät, hergestellt werden.

Offenbart sind weiterhin die zu den konkret beschriebenen Nukleotidsequenzen komplementären Nukleinsäuremoleküle oder einen Abschnitt davon.

Die offenbarten Nukleotidsequenzen ermöglichen die Erzeugung von Sonden und Primern, die zur Identifizierung und/oder Klonierung von homologer Sequenzen in anderen Zelltypen und Organismen verwendbar sind. Solche Sonden bzw. Primer umfassen gewöhnlich einen Nukleotidsequenzbereich, der unter stringenten Bedingungen an mindestens etwa 12, vorzugsweise mindestens etwa 25, wie z.B. etwa 40, 50 oder 75 aufeinanderfolgende Nukleotide eines Sense-Stranges einer erfindungsgemäßen Nukleinsäuresequenz oder eines entsprechenden Antisense-Stranges hybridisiert.

Weitere erfindungsgemäß verwendbare Nukleinsäuresequenzen sind abgeleitet von den hierin beschriebenen Sequenzen und unterscheiden sich davon durch Addition, Substitution, Insertion oder Deletion einzelner oder mehrerer Nukleotide, kodieren aber weiterhin für Polypeptide mit dem gewünschten Eigenschaftsprofil.

Offenbart sind auch solche Nukleinsäuresequenzen, die sogenannte stumme Mutationen umfassen oder entsprechend der Codon-Nutzung eins speziellen Ursprungs- oder Wirtsorganismus, im Vergleich zu einer konkret genannten Sequenz verändert sind, ebenso wie natürlich vorkommende Varianten, wie z.B. Spleißvarianten oder Allelvarianten, davon. Gegenstand sind ebenso durch konservative Nukleotidsubstutionen (d.h. die betreffende Aminosäure wird durch eine Aminosäure gleicher Ladung, Größe, Polarität und/oder Löslichkeit ersetzt) erhältliche Sequenzen.

Offenbart sind auch die durch Sequenzpolymorphismen von den konkret offenbarten Nukleinsäuren abgeleiteten Moleküle. Diese genetischen Polymorphismen können zwischen Individuen innerhalb einer Population aufgrund der natürlichen Variation existieren. Diese natürlichen Variationen bewirken üblicherweise eine Varianz von 1 bis 5 % in der Nukleotidsequenz eines Gens.

Weiterhin umfasst die auch Nukleinsäuresequenzen, welchen mit oben genannten kodierenden Sequenzen hybridisieren oder dazu komplementär sind. Diese Polynukleotide lassen sich bei Durchmusterung von genomischen oder cDNA-Banken auffinden und gegebenenfalls daraus mit geeigneten Primern mittels PCR vermehren und anschließend beispielsweise mit geeigneten Sonden isolieren. Eine weitere Möglichkeit bietet die Transformation geeigneter Mikroorganismen mit offenbarten Polynukleotiden oder Vektoren, die Vermehrung der Mikroorganismen und damit der Polynukleotide und deren anschließende Isolierung. Darüber hinaus können die offenbarten Polynukleotide auch auf chemischem Wege synthetisiert werden.

Unter der Eigenschaft, an Polynukleotide "hybridisieren" zu können, versteht man die Fähigkeit eines Poly- oder Oligonukleotids unter stringenten Bedingungen an eine nahezu komplementäre Sequenz zu binden, während unter diesen Bedingungen unspezifische Bindungen zwischen nicht-komplementären Partnern unterbleiben. Dazu sollten die Sequenzen zu 70-100%, vorzugsweise zu 90-100%, komplementär sein. Die Eigenschaft komplementärer Sequenzen, spezifisch aneinander binden zu können, macht man sich beispielsweise in der Northern- oder Southern-Blot-Technik oder bei der Primerbindung in PCR oder RT-PCR zunutze. Üblicherweise werden dazu Oligonukleotide ab einer Länge von 30 Basenpaaren eingesetzt. Unter stringenten Bedingungen versteht man beispielsweise in der Northern-Blot-Technik die Verwendung einer 50 - 70 °C, vorzugsweise 60 - 65 °C warmen Waschlösung, beispielsweise 0,1x SSC-Puffer mit 0,1% SDS (20x SSC: 3M NaCl, 0,3M Na-Citrat, pH 7,0) zur Elution unspezifisch hybridisierter cDNA-Sonden oder Oligonukleotide. Dabei bleiben, wie oben erwähnt, nur in hohem Maße komplementäre Nukleinsäuren aneinander gebunden. Die Einstellung stringenter Bedingungen ist dem Fachmann bekannt und ist z:B. in Ausubel et al., Current Protocols in Molecular Biology, John Wiley & Sons, N.Y. (1989), 6.3.1-6.3.6. beschrieben.

### c) Expressionskonstrukte und Vektoren:

Gegenstand der Offenbarung sind außerdem Expressionskonstrukte, enthaltend unter der genetischen Kontrolle regulativer Nukleinsäuresequenzen eine für ein offenbarungsgemäß verwendetes Polypeptid kodierende Nukleinsäuresequenz; sowie Vektoren, umfassend wenigstens eines dieser Expressionskonstrukte. Vorzugsweise umfassen solche erfindungsgemäßen Konstrukte 5'-stromaufwärts von der jeweiligen kodierenden Sequenz einen Promotor und 3'-stromabwärts eine Terminatorsequenz sowie gegebenenfalls weitere übliche regulative Elemente, und zwar jeweils operativ verknüpft mit der kodierenden Sequenz. Unter einer "operativen Verknüpfung" versteht man die sequentielle Anordnung von Promotor, kodierender Sequenz, Terminator und gegebenenfalls weiterer regulativer Elemente derart, dass jedes der regulativen Elemente seine Funktion bei der Expression der kodierenden Sequenz bestimmungsgemäß erfüllen kann. Beispiele für operativ verknüpfbare Sequenzen sind Targeting-Sequenzen sowie Enhancer, Polyadenylierungssignale und dergleichen. Weitere regulative Elemente umfassen selektierbare Marker, wie Resistenzgene, Amplifikationssignale, Replikationsursprünge und dergleichen. Geeignete regulatorische Sequenzen sind z.B. beschrieben in Goeddel, Gene Expression Technology: Methods in Enzymology 185, Academic Press, San Diego, CA (1990).

Zusätzlich zu den artifiziellen Regulationssequenzen kann die natürliche Regulationssequenz vor dem eigentlichen Strukturgen noch vorhanden sein. Durch genetische Veränderung kann diese natürliche Regulation gegebenenfalls ausgeschaltet und die Expression der Gene erhöht oder erniedrigt werden. Das Genkonstrukt kann aber auch einfacher aufgebaut sein, das heißt es werden keine zusätzlichen Regulationssignale vor das Strukturgen insertiert und der natürliche Promotor mit seiner Regulation wird nicht entfernt. Statt dessen wird die natürliche Regulationssequenz so mutiert, dass keine Regulation mehr erfolgt und die Genexpression gesteigert oder verringert wird. Die Nukleinsäuresequenzen können in einer oder mehreren Kopien im Genkonstrukt enthalten sein.

Beispiele für brauchbare Promotoren sind: cos-, tac-, trp-, tet-, trp-tet-, lpp-, lac-, Ipplac-, laclq-, T7-, T5-, T3-, gal-, trc-, ara-, SP6-, lambda-PR- oder im lambda-PL-Promotor, die vorteilhafterweise in gram-negativen Bakterien Anwendung finden; sowie die gram-positiven Promotoren amy und SPO2, die Hefepromotoren ADC1, MFalpha , AC, P-60, CYC1, GAPDH oder die Pflanzenpromotoren CaMV/35S, SSU, OCS, lib4, usp, STLS1, B33, not oder der Ubiquitin- oder Phaseolin-Promotor. Zu nenne sind auch induzierbare Promotoren, wie z.B. licht- und insbesondere temperaturinduztierbarer Promotoren, wie der PᵣPₗ-Promotor. Prinzipiell können alle natürlichen Promotoren mit ihren Regulationssequenzen verwendet werden. Darüber hinaus können auch synthetische Promotoren vorteilhaft verwendet werden.

Die regulatorischen Sequenzen bzw. Faktoren können dabei vorzugsweise die Expression positiv beeinflussen und dadurch erhöhen oder erniedrigen. So kann eine Verstärkung der regulatorischen Elemente vorteilhafterweise auf der Transkriptionsebene erfolgen, indem starke Transkriptionssignale wie Promotoren und/oder "Enhancer" verwendet werden. Daneben ist aber auch eine Verstärkung der Translation möglich, indem beispielsweise die Stabilität der mRNA verbessert wird.

Die Herstellung einer Expressionskassette erfolgt durch Fusion eines geeigneten Promotors mit einer geeigneten kodierenden Nukleotidsequenz sowie einem Terminator- oder Polyadenylierungssignal. Dazu verwendet man gängige Rekombinations- und Klonierungstechniken, wie sie beispielsweise in T. Maniatis, E.F. Fritsch und J. Sambrook, Molecular Cloning: A Laboratory Manual, Cold Spring Harbor Laboratory, Cold Spring Harbor, NY (1989) sowie in T.J. Silhavy, M.L. Berman und L.W. Enquist, Experiments with Gene Fusions, Cold Spring Harbor Laboratory, Cold Spring Harbor, NY (1984) und in Ausubel, F.M. et al., Current Protocols in Molecular Biology, Greene Publishing Assoc. and Wiley Interscience (1987) beschrieben sind.

Das rekombinante Nukleinsäurekonstrukt bzw. Genkonstrukt wird zur Expression in einem geeigneten Wirtsorganismus vorteilhafterweise in einen Vektor insertiert, der eine optimale Expression der Gene im Wirt ermöglicht. Vektoren sind dem Fachmann wohl bekannt und können beispielsweise aus "Cloning Vectors" (Pouwels P. H. et al., Hrsg, Elsevier, Amsterdam-New York-Oxford, 1985) entnommen werden. Unter Vektoren sind außer Plasmiden auch alle anderen dem Fachmann bekannten Vektoren, wie beispielsweise Phagen, Viren, wie SV40, CMV, Baculovirus und Adenovirus, Transposons, IS-Elemente, Phasmide, Cosmide, und lineare oder zirkuläre DNA zu verstehen. Diese Vektoren können autonom im Wirtsorganismus repliziert oder chromosomal repliziert werden.

Als Beispiele für geeignete Expressionsvektoren können genannt werden:

Übliche Fusionsexpressionsvektoren, wie pGEX (Pharmacia Biotech Inc; Smith, D.B. und Johnson, K.S. (1988) Gene 67:31-40), pMAL (New England Biolabs, Beverly, MA) und pRIT 5 (Pharmacia, Piscataway, NJ), bei denen Glutathion-S-Transferase (GST), Maltose E-bindendes Protein bzw. Protein A an das rekombinante Zielprotein fusioniert wird.

Nicht-Fusionsprotein-Expressionsvektoren wie pTrc (Amann et al., (1988) Gene 69:301-315) und pET 11d (Studier et al. Gene Expression Technology: Methods in Enzymology 185, Academic Press, San Diego, Kalifornien (1990) 60-89).

Hefe-Expressionsvektor zur Expression in der Hefe *S*. *cerevisiae*, wie pYepSec1 (Baldari et al., (1987) Embo J. 6:229-234), pMFa (Kurjan und Herskowitz (1982) Cell 30:933-943), pJRY88 (Schultz et al. (1987) Gene 54:113-123) sowie pYES2 (Invitrogen Corporation, San Diego, CA). Vektoren und Verfahren zur Konstruktion von Vektoren, die sich zur Verwendung in anderen Pilzen, wie filamentösen Pilzen, eignen, umfassen diejenigen, die eingehend beschrieben sind in: van den Hondel, C.A.M.J.J. & Punt, P.J. (1991) "Gene transfer systems and vector development for filamentous fungi, in: Applied Molecular Genetics of Fungi, J.F. Peberdy et al., Hrsg., S. 1-28, Cambridge University Press: Cambridge.

Baculovirus-Vektoren, die zur Expression von Proteinen in gezüchteten Insektenzellen (bspw. Sf9-Zellen) verfügbar sind, umfassen die pAc-Reihe (Smith et al., (1983) Mol. Cell Biol.. 3:2156-2165) und die pVL-Reihe (Lucklow und Summers (1989) Virology 170:31-39).

Pflanzen-Expressionsvektoren, wie solche, die eingehend beschrieben sind in: Becker, D., Kemper, E., Schell, J. und Masterson, R. (1992) "New plant binary vectors with selectable markers located proximal to the left border", Plant Mol. Biol. 20:1195-1197; und Bevan, M.W. (1984) "Binary Agrobacterium vectors for plant transformation", Nucl. Acids Res. 12:8711-8721.

Säugetier-Expressionsvektoren, wie pCDM8 (Seed, B. (1987) Nature 329:840) und pMT2PC (Kaufman et al. (1987) EMBO J. 6:187-195).

Weitere geeignete Expressionssysteme für prokaryontische und eukaryotische Zellen sind in Kapitel 16 und 17 von Sambrook, J., Fritsch, E.F. und Maniatis, T., Molecular cloning: A Laboratory Manual, 2. Auflage, Cold Spring Harbor Laboratory, Cold Spring Harbor Laboratory Press, Cold Spring Harbor, NY, 1989 beschrieben

### d) Rekombinante Wirte:

Mit Hilfe der offenbarten Vektoren sind rekombinante Mikroorganismen/Zelliinien herstellbar, welche beispielsweise mit wenigstens einem offenbarten Vektor transformiert sind.

Vorteilhafterweise werden die oben beschriebenen offenbarten rekombinanten Konstrukte in ein geeignetes Wirtssystem eingebracht und exprimiert. Dabei werden vorzugsweise dem Fachmann bekannte geläufige Klonierungs- und Transfektionsmethoden, wie beispielsweise Co-Präzipitation, Protoplastenfusion, Elektroporation, retrovirale Transfektion und dergleichen, verwendet, um die genannten Nukleinsäuren im jeweiligen Expressionssystem zur Expression zu bringen. Geeignete Systeme werden beispielsweise in Current Protocols in Molecular Biology, F. Ausubel et al., Hrsg., Wiley Interscience, New York 1997, oder Sambrook et al. Molecular Cloning: A Laboratory Manual. 2. Aufl., Cold Spring Harbor Laboratory, Cold Spring Harbor Laboratory Press, Cold Spring Harbor, NY, 1989 beschrieben.

Als Wirtsorganismen sind prinzipiell alle Organismen geeignet, die eine Expression der offenbarten Nukleinsäuren, ihrer Allelvarianten, ihrer funktionellen Äquivalente oder Derivate ermöglichen.

Die für die Etablierung des offenbarten ACR-Testsystems geeigneten Zelllinien sind solche, in denen eine mikroskopisch beobachtbare Reorganisation des Aktin-Cytosteletts induzierbar ist. Solche Zelllinien sind insbesondere von einer adhärenten Säugerzelllinie mit ausgespreiteter Zellmorphologie abgeleitet, wie insbesondere, vorzugsweise humanen, Epithelial- oder Fibroblastenzelllinie. Es können aber auch Säuger-Zelllinien nicht-humanen Ursprungs, wie z.B. Zelllinien von Maus, Ratte, Affen, Ziege, Schaft, Rind, Hamster und dergleichen verwendet werden. Als Beispiele können genannt werden: COS1, COS7, SWISS oder NIH 3T3, CHO, PC12. Als weitere Beispiele sind primäre Zellen (z.B. kortikale Neuronen) zu nennen, für welche ein Nervenauswachsen im Zusammenhang mit Rho vielfach beschrieben wurde (vgl. z.B. Dergham et al. 2003, J. Neuroscience 22(15), S. 6570).

Besonders geeignet sind humanen embryonalen Nieren-Zelllinien: Als Beispiele können insbesondere HEK293-zellen genannt werden.. Weiterhin sind alle adhärenten Epithel- und Fibroblastenzelllinien, auch Krebszelllinien, wie z.B. die Neurogliomazelllinie U87MG (Liao et al. 2004, J. Neurochem. 90 (5), S. 1156), SH-SY5Y (=Neuroblastoma), HeLa, CACO-2 grundsätzlich geeignet.

Die Selektion erfolgreich transformierter Organismen kann durch Markergene erfolgen, die ebenfalls im Vektor oder in der Expressionskassette enthalten sind. Beispiele für solche Markergene sind Gene für Antibiotikaresistenz und für Enzyme, die eine farbgebende Reaktion katalysieren, die ein Anfärben der transformierten Zelle bewirkt. Diese können dann mittels automatischer Zellsortierung selektiert werden. Erfolgreich mit einem Vektor transformierte Mikroorganismen, die ein entsprechendes Antibiotikaresistenzgen (z.B. G418 oder Hygromycin) tragen, lassen sich durch entsprechende Antibiotika-enthaltende Medien oder Nährböden selektieren. Markerproteine, die an der Zelloberfläche präsentiert werden, können zur Selektion mittels Affinitätschromatographie genutzt werden.

### e) Durchführung des erfindungsgemäßen Assays

Das erfindungsgemäße Nachweisverfahren kann als Einzeluntersuchung oder im Rahmen groß angelegter Screenings eingesetzt werden. Grundsätzlich wird dabei aber nach im Wesentlichen gleichem Schema verfahren.

Es wird eine definierte Anzahl (z.B. 10² bis 10⁶) von rekombinanten, vorzugsweise frisch kultivierten Zellen in einem geeigneten Testgefäß, z.B. Vertiefung (Well) einer Mikrotiterplatte, in Kulturmedium vorgelegt. Die Zellen werden dann mit einer wirksamen Menge rezeptorbindenen Liganden (für AP-Nogo66 z.B. 1 bis 50, ng/ml, wie insbesondere etwa 10 ng/ml) inkubiert, wie z.B. 1 bis 30 Minuten, bei einer geeigneten Temperatur, wie z.B. im Bereich von etwa 20 bis 40 °C. Optimale Testbedingungen sind vom Fachmann durch wenige Vorversuche ohne weiteres feststellbar.

Die Stimulation erfolgt in Gegenwart oder Abwesenheit eines zu untersuchenden Analyten, der einen Effektor der Ligand-Rezeptor-Wechselwirkung enthält. Gewöhnlich werden verschiedene Konzentrationen des Analyten eingesetzt. Die Zugabe des Analyten kann dabei zeitlich vor oder nach der Ligandenzugabe oder gleichzeitig damit erfolgen.

Zur Auswertung werden die stimulierten Zellen, vorzugsweise nach Fixierung auf einem Träger, wie z.B. der verwendeten Mikrotiterplatte, angefärbt und visualisiert. Geeignete Methoden zur Fixierung, Färbung und Visualisierung der Zellen sind dem Fachmann bekannt (vgl. z.B. Zell- und Gewebekultur, 4. Aufl. Sektrum akademischer Verlag, Fischer).

Die Veränderungen im Cytoskelett sind in der Regel so signifikant, dass eine qualitative Aussage zum Testergebnis bereits durch Vergleich mit einer visualisierten Referenzprobe getroffen werden kann.

Quantitative Aussagen können unter Verwendung automatischer Bildanalysesysteme gewonnen werden, die für die untersuchte Zellpopulation geeignete Messparameter bestimmen und zwischen den einzelnen Proben vergleichen. Derartige Parameter sind z.B. Zellumfang, Zelllänge, Zellbreite, Zellfläche, Länge der Zellausläufer, eine mathematische Verknüpfung von wenigstens zwei dieser Parameter oder ein entsprechender reziproken Wert eines solchen Parameters.

Screeningverfahren können außerdem hinsichtlich Probenvorbereitung, Durchführung und Auswertung der Messung automatisiert werden, so dass der Zeitaufwand je Einzelanalyse signifikant verringert werden kann. Geeignete Vorrichtungen werden von verschiedenen Herstellern angeboten, wie z.B. von den Firmen Becton Dickinson, Molecular Devices, Cellomics, Amersham Biosciences.

Die vorliegende Erfindung wird nun unter Bezugnahme auf die folgenden nichtlimitierenden Ausführungsbeispiele näher beschrieben.

### Experimenteller Teil

### I. Allgemeine Angaben

### a) Bestandteile der Kulturmedien

RPMI-Glutamax (Invitrogen, Carlsbad, USA)
Geneticin (G418); (Antibiotikum; Selektionsmarker) (Invitrogen)
Fetal Bovine Serum (FBS) Dialyzed, (Invitrogen)
Antibiotic-Antimycotic (Invitrogen)
Zeocin (Antibiotikum; Selektionsmarker) (Invitrogen)
LIPOFECTAMINE (Invitrogen)

### b) Zellen

Menschliche embryonale Nierenzellen (HEK293) (ATCC = American Tissue Culture Collection, Bestellnr.: CRL-1573) wurden mit Rho (HEK293-RhoA) oder mit Rho in Kombination mit dem Rezeptorkomplex NgR/p75 (HEK293-RhoA/NgR/p75) stabil transfiziert. Die Zellen wurden in RPMI-Glutamax Medium, ergänzt mit 5 % hitzeinaktiviertem fötalem Rinderserum (FBS), HEPES (10 mM), Penicillin (100 U/ml), Streptomycin (100 mM) und entweder Neomycin (HEK293-RhoA) oder Neomycin in Kombination mit Zeocin (HEK293-RhoA/NgR/p75), gehalten.

### c) Reagenzien

| | |
|---|---|
| Nogo66, enthaltend ein N-terminales alkalische Phosphatase-Tag (AP-Nogo66) | |
| L-alpha-Lysophosphatitsäure (LPA) | Sigma, München, DE |
| Phosphatidylinositol-spezifische Phospholipase C (PI-PLC) | Sigma |
| Mit Rho-Bindungsdomäne (RBD) von Rhotekin (Aminosäuren 7-89) beschichtete | Upstate Biotechnology, Lake Placid, USA |
| Beads | |
| Anti-RhoA-Antikörper | Santa Cruz Biotechnology, Santa Cruz, USA |
| Anti-Nogo66 Antikörper (NogoA12-A) | Alpha Diagnostics International, San Antonio, Texas, USA |
| Alexa Phalloidin 568 oder 488 (Farbstoff zur Fluoreszenz-Färbung von F-Aktin) | Molecular Probes, Eugene, USA |
| ECL (Western Blotting Detection Reagent, Elektocheminumileszenz), | Pierce, Rockford, USA |
| DAPI (Hoechst 33342, Trihydrochlorid, trihydrat) | Molecular Probes Inc., Eugene, USA |

### II. Allgemeine Testmethoden

### a) RBD-Assay (vgl. auch Figur 2)

Die HEK-Zellen blieben entweder unstimuliert oder wurden (z.B. mit LPA oder AP-Nogo66) für die Dauer von 5 bis 10 Minuten nach einer 2-stündigen Hungerperiode (durch Inkubation mit serumfreiem RPMI-Glutamax) stimuliert. HEK-Zellen (1x 10⁶ pro Well) wurden in 6-well-Platten (Ansatzvolumen 1000 µl/well) kultiviert. Vor der Stimulation wurde entsprechend der Konzentration der eingesetzten AP-Nogo66 Batch pro Well ein bestimmtes Volumen (z.B. 100 µl) an Medium entnommen und mit AP-Nogo66 entsprechend wieder bis auf 1000 µl aufgefüllt, so dass sich eine definierte Endkonzentration von AP-Nogo66 auf den Zellen ergab. Im Anschluss an die Stimulation wurde die AP-Nogo66-Medium Lösung komplett abgesaugt. Die Zellen wurden anschließend mit eiskalter PBS (phosphatgepufferter Kochsalzlösung) gewaschen und in 50 mM Tris, pH 7.5 (enthaltend NaCl (500 mM), EDTA (1 mM), MgCl₂ (10 nM), 10 % Glycerin, NaF (5 mM), DTT (1 mM), 1 % Nonidet P-40 in Kombination mit den Protease Inhibitoren PMSF (1 mM), Leupeptin (1 µg/ml) und Aprotinin (1 µg/ml)) lysiert.

Zelllysate (z.B. von HEK293-RhoA und HEK293-RhoA/NgR/p75; jeweils 400 µg Gesamtprotein) wurden 1 Stunde bei 4 °C mit 40 µg der GST-RBD Beads (enthaltend rekombinantes Fusionsprotein aus Glutathion S-Transferase und RBD mit den Aminosäuren 7 bis 89 von humanem Rhotekin) inkubiert. Nach 3-maligem Waschen der Beads mit Lysepuffer wurde das gebundene Protein (GTP-gebundenes, biochemisch aktives Rho) durch SDS-PAGE und Immunblotting analysiert. Das Immunoblotting wurde mit anti-Rho-Antikörper durchgeführt und die Visualisierung erfolgte durch Inkubation mit einem Meerettichperoxidase-konjugiertem sekundären Antikörper und Chemilumineszenzverstärker (ECL).

### b) ACR-Assay mit lmmunofluoreszenz-Messung:

1 × 10⁴ Zellen wurden in Mikrotiterplatten mit 96 Vertiefungen zwei Tage vor der Stimulierung überimpft. Die Stimulation der Zellen erfolgte mit einem geeigneten Reagens (z.B. LPA oder AP-Nogo66) in Kulturmedium (RPMI-Glutamax) enthaltend 5% FCS. Nach einer 5- bis 10-minütigen Stimulationsperiode wurde die Aktivierung mit kalter PBS unterbrochen. Die Zellen wurden mit 3 - 4 %-iger Paraformaldehydlösung fixiert, mit PBS, enthaltend 0,2 % Triton X-100 permeabilisiert und 30 - 45 Minuten mit Phalloidin Alexa 568 oder 488 inkubiert. Zusätzlich erfolgte eine 5 minütige Inkubation mit DAPI zur Kernfärbung. Die Zellen wurden mit hilfe eines Epifluoreszenzmikroskops (Axiovert 25) visualisiert. Fluoreszenzmikrogramme wurden mit einer gekühlten CCD Kamera von Zeiss aufgenommen.

### c) FACS Analyse

### Reagenzien:

| | |
|---|---|
| Maus-anti-Human p75; IgG Monoklonaler Antikörper | Sigma N-5408 |
| Anti-Maus-FITC | Sigma F-5262 |
| Ziege-anti-Human NogoR | R8D Systems; AF1208 |
| Anti-Ziege-FITC | Sigma F7367 |

### Durchführung:

Zunächst wurden die HEK293-Zellen mit PBS gewaschen. Adhärente Zellen wurden mit PBS, enthaltend 5 mM EDTA abgelöst. Anschließend wurden 2 × 10⁶ Zellen in ein Eppendorff-Gefäß überführt und 2 Minuten bei 1300 Upm zentrifugiert. Die Zellen wurden in PBS, enthaltend 1 % BSA (1 ml/Gefäß) resuspendiert und 2 Minuten bei 1300 Upm zentrifugiert.

Die Pellets wurden erneut mit 0,1 ml PBS/1 % BSA einschließlich Primärantikörper (Maus-anti-Human p75 1:100, oder Ziege-anti-Human NogoR 1:50) resuspendiert und 90 Minuten bei 4°C inkubiert. Anschließend gab man 0,1 ml PBS/1 % BSA hinzu, mischte und zentrifugierte 2 Minuten bei 1300 Upm.

Die Pellets wurden in PBS, enthaltend 1 % BSA (0,1 ml/Gefäß) und Sekundärantikörper (anti-Maus-FITC 1:100; oder anti-Ziege-FITC 1:100); resuspendiert und 60 Minuten bei 4°C inkubiert.

Man gab PBS, enthaltend 1 % BSA (1 ml/Gefäß) hinzu, mischte und zentrifugierte 2 Minuten bei 1300 Upm. Nach Abzentrifugation wurden die Pellets erneut in PBS/ 0,1 % Propidiumjodid zur Detektion toter Zellen resuspendiert. Nach Resuspendieren erfolgte eine FACS-Analyse (FACScan, Firma Becton Dickinson, Heidelberg, Deutschland).

### III. Ausführungsbeispiele

### Beispiel 1: Herstellung von AP-Nogo66:

### a) Klonierung des Nogo66 Fragments von hNogoA

Es wurde ausgegangen von der publizierten hNogoA Sequenz (NCBI): AF148537. Die beiden folgenden synthetischen Oligonukleotide wurden von der publizierten Sequenz abgeleitet:
Mez 402: GCCACCATGAGGATATACAAGGGTGTGATCC (SEQ ID NO:1); Oligo ab Aminosäure R1055 (kursiv) mit Start ATG (unterstrichen) und Kozak Sequenz (fett)
Mez 404: CTTCAGAGAATCAACTAAATCATC (SEQ ID NO:2); Oligo bis Aminosäure K1120 (fett)

Mit diesen beiden Oligos wurde in frontal cortex cDNA als Template (hergestellt mit Superscript first strand synthesis system for RT-PCR; Invitrogen, Carlsbad, CA) (Novak et al., Brain Res. Mol. Brain, 2002, 107(2): 183-189) eine PCR durchgeführt. Die Reaktion wurde nach Standardmethode, wie Innis et al. (PCR Protocols. A Guide to Methods and Applications, Academic Press (1990)) mit Herculase (Stratagene, La Jolla, USA), durchgeführt. Das erhaltene DNA Fragment mit einer Grösse von 207 bp wurde mit dem QIAEX II Gel Extraction Kit (QIAGEN GmbH, Hilden, Germany) nach Angaben des Herstellers gereinigt. Das amplifizierte, gereinigte Fragment wurde in pcDNA3.1V5-His TOPO (SEQ ID NO:6) (pcDNA3.1/V5-His TOPO TA Expression Kit, #K4800-01) gesetzt. Mit dem so erhaltenen Konstrukt pcDNA3.1V5-His hNOGO66 wurden E.coli TOP10 Zellen (Invitrogen, Carlsbad, CA) nach Standardmethoden wie in Sambrook et al. (Molecular Cloning. A Laboratory Manual, Cold Spring Harbor, (1989)) beschrieben, transformiert. Eine Selektion auf Plasmid tragende Zellen wurde durch das Antibiotikum Ampicillin erreicht.

Die Identifikation von neun Klonen mittels PCR zeigte bei drei Klonen eine Bande in der richtigen Größe von 207 bp. Die Richtigkeit der Sequenz wurde Sequenzanalyse überprüft.

Die kodierende Nogo66-Sequenz wurde mit Xbal aus obigem Plasmid pcDNA3.1V5-His hNOGO66 herausgeschnitten und in das Plasmid pAP-tag5 (GenHunter Cooperation, Nashville, TN, USA) über Xbal einkloniert. Man erhält das Plasmid pAP-tag5/PPC/hNOGO66 Nr.5. (SEQ ID NO:7) dargestellt in Figur 8.

### b) Herstellung der stabilen Zelllinie:

HEK293 Zellen wurden in Wachstumsmedium (DMEM mit 10% fötalem Rinderserum unter Zusatz von Penicillin/Streptomycin) bei 37°C und 5% CO₂ kultiviert. Für die Transfektion wurden die Zellen in Platten mit 6 Kavitäten (1x 10⁶ pro Well) ausgesät und über Nacht inkubiert. Die Zellen wurden mit einem Cocktail, der 1µg Plasmid DNA (pAP-tag5/PPC/hNOGO66 Nr.5) und 3µl Fugene6 (ROCHE Diagnostics, Mannheim) enthielt, nach Angaben der Herstellers transfiziert. Zwei Tage später wurden die Zellen durch Behandlung mit Trypsin abgelöst, in eine Zellkulturflasche mit 175cm² Grundfläche überführt und die Selektion durch Zugabe von 150µg/ml Zeocin in Wachstumsmedium gestartet. Heranwachsende Zeocin-resistente Zellkolonien wurden nach etwa 4 Wochen mit Trypsin abgelöst und mit einem Zellsorter in 96-well Platten vereinzelt. Nach etwa drei Wochen wurde ein Aliquot des Mediumüberstands der herangewachsenen Einzellzellkolonien, deren Konfluenz zu diesem Zeitpunkt zwischen 5 und 85% abgeschätzt wurde, auf Nitrozellulose pipettiert. Durch Zugabe einer Lösung von NBT (Nitroblau-Tetrazoliumsalz) und BCIP (5-Brom-4-chlor-3-indolylphosphat) wurde die Proteinexpression über die Aktivität der Alkalischen Phosphatase anhand der entstehenden Färbung nachgewiesen.

### c) Expression von AP-Nogo66:

Ein HEK293 Klon (Klon Nr. 5), welcher AP-Nogo66 produziert, wurde in Kulturmedium (RPMI Glutamax +10% FCS, 150 µg/ml Zeocin, Antibiotic / Antimycotic) durch mehrmalige Passage (ca. 20) in Kulturflaschen vermehrt. Die AP-Nogo66-Expression wurde nach jeder zweiten oder dritten Passage im Dot Blot Test (mit NBT/BCIP Reagens) überprüft.

Um AP-Nogo66 in Serum-freiem Medium zu produzieren wurden die Zelle zunächst bis fast zur Konfluenz in Triple-Flasks kultiviert, dann erfolgte ein Mediumwechsel zu Expressionsmedium (Pro293a - CDM Medium (Biowhittaker; # 12-764Q),2mM Glutamin, Antibiotic / Antimycotic). Daraufhin wurden die Zellen 3 - 4 Tage bei 37 °C kultiviert, die Überstände wurden abgenommen und abzentrifugiert (1500 Upm, 5 min). Die Überstände wurden bei -80°C gesammelt. Zur weiteren Verarbeitung wurden sie aufgetaut, mit Proteinaseinhibitoren (PMSF 0,1 mM, Pefabloc SC 1 mM) versetzt und in Amicon Tubes (Millipore) aufkonzentriert und die AP-Nogo66- Konzentration wurde bestimmt (ca. 3 µg/ml) (Mw von monomerer AP: 67 kDa, und monomerem AP-Nogo66 etwa 75 kDa in SDS Gelen).

Der AP-Nogo66-Pool wurde mit anti-AP-Agarose immunopräzipitiert. Das Präzipitat wurde durch 10-minütiges Erhitzen bei 95°C in Gegenwart von 5% Mercaptoethanol denaturiert und so von den Agarose Beads entfernt, schließlich durch Western-Analyse mittels Antikörpern mit Spezifität für AP und Nogo66 verifiziert. Ein SDS-Gel ist in Figur 7 dargestellt.

Durch Gelchromatographie (Superose 12, Amersham Biosciences. Laufmittel: 20 mM Natriumphosphat, 140 mM Natriumchlorid, pH 7.4) wurde bestimmt, dass AP-Nogo66 als Dimer läuft und ein Mw von etwa 140 kDa aufweist.

In Vorversuchen wurde festgestellt, dass AP-Nogo66, nicht aber GST-Nogo66 funktionell aktiv war (Versuche nicht gezeigt). Es wird daher vermutet, dass NgR-Liganden womöglich als Dimer vorliegen müssen, um funktionell zu sein. Wie die Kristallstruktur der Alkalischen Phosphatase zeigt, wird die Dimerisierung vom AP-Tag induziert.

### Beispiel 2: Herstellung von Konstrukten zur Generierung von rekombinanten HEK Zelllinien

### a) Klonierung von hp75; Herstellung von pcDNA3.1(V5-His)hp75 Nr.16 (Fig. 3d)

Es wurde von den publizierten Sequenzen für humanes p75 ausgegangen: NM_002507; M14764 (die beide 100% identisch sind ). Die folgenden Oligonukleotide wurden von der publizierten Sequenz abgeleitet:
Mey 36: GCCACC**ATG**GGGGCAGGTGCCACC (SEQ ID NO:4) ; Oligo mit Start-Codon (fett) mit Kozak Sequenz (kursiv)
Mey 35: **TCA**CACTGGGGATGTGGCAG (SEQ ID NO:3); Oligo mit Stop Codon (fett) ein Basenaustausch T statt C (unterstrichen) da das Oligo von der publizierten Ratten Sequenz abgeleitet ist
Mey 71: GCAGCCCCATCAGTCCGC (SEQ ID NO:5); Oligo beginnend 64 Basenpaare vor Start ATG

Anschließend wurde eine PCR (analog zur Nogo66-Klonierung, Beispiel 1) mit dem Primerpaar Mey 35/71 in cDNA aus der Zelllinie SH-SY5Y (humane Neuroblastomazelllinie ATCC # CRL-2266) durchgeführt. Die PCR mit Mey35/71 lieferte ein 1348 bp Fragment. Dieses wurde gereinigt.

Eine anschließende PCR mit dem Primerpaar Mey 35/36 im Mey 35/71-Fragment ergab eine deutliche Bande (Fragmentgröße: 1284 bp). Diese wurde gereinigt. Dann wurde diese Bande in pcDNA3.1/V5-His TOPO Vector ( pcDNA3.1(V5-His)TOPO TA Expression Kit Invitrogen #K4800-01) ungeschnitten eingesetzt (Prinzip Topoisomerase). Mit dem so erhaltenen Konstrukt (pcDNA3.1 hp75) wurden TOP10 Zellen nach Standardmethoden (analog zur Nogo66-Klonierung, Beispiel 1) transformiert.

13 Klone dieser Transformation wurden überprüft und vier Klone mit der richtigen Orientierung ( Nr. 13 , 15 , 16 und 18 ) konnten identifiziert werden. Klon Nr. 16 wurde weiterverwendet. Die Sequenz des enthaltenen Konstruktes (pcDNA3.1(V5-His)hp75 Nr.16 ; vgl. auch Figur **3d**) ist in SEQ ID NO:8 dargestellt. Weitere Informationen zu dieser Sequenz sind im folgenden Abschnitt zusammengefasst.

| | | |
|---|---|---|
| Human p75 | start: 40 | stop:1320 |
| Neomycin | start:2596 | stop: 3390 |
| Ampicillin | start:4897 | stop:5757 compl. |
| Bgh pA | start:1487 | stop:1701 |
| SV40 pA | start:3416 | stop:3654 |
| His tag | start:1441 | stop:1458 |
| V5 Epitope | start:1397 | stop:1438 |
| F1 ori | start:1764 | stop:2177 |
| SV40 promoter + ori | start:2242 | stop:2567 |
| pUC ori | start:4086 | stop:4759 |

### b) Herstellung von pIRES hp75

Plasmid pIRES (Clontech, Palo Alto, USA) wurde mit Xba I und Not I (Roche Diagnostics) unter Verwendung eines Restriktionsansatzes (40 µl;1,5 h bei 37 °C) enthaltend 5 µg DNA, 2 µl Xba 1, 2 µl Not 1, 4 µl 10x Puffer H (Roche) und 32 µl Wasser bidest. geschnitten: Das ausgeschnittene Fragment wurde mit Hilfe des Qiagen Gelextraktionskit aus einem DNA-Gel in herkömmlicher Weise gewonnen.

Plasmid pcDNA3.1 hp75, hergestellt wie oben beschrieben, wurde mit Spe I und und Not I (Roche Diagnostics) unter Verwendung eines Restriktionsansatzes (40 µl;1,5 h bei 37 °C) enthaltend 5 µg DNA, 2 µl Spe 1, 2 µl Not I, 4 µl 10x Puffer H (Roche), 32 µl Wasser bidest geschnitten. Das ausgeschnittene Fragment wurde mit Hilfe des Qiagen Gelextraktionskit aus einem DNA-Gel in herkömmlicher Weise gewonnen.

Die auf diese Weise hergestellten Restriktionsfragmente wurden unter Verwendung des folgenden Ansatzes über Nacht bei 16 °C ligiert: 5 µl hp75-Konstrukt, 2 µl geschnittener pIRES, 1,5 µl T4-DNA Ligase (Roche) 3 µl 10x Puffer T4 (Roche), 20,5 µl Wasser bidest. Diese Ligation mit der Bezeichnung pIRES hp75 wurde dann in den Bakterienstamm SuperComp XL2 Blue (Stratagene) transformiert.

### c) Herstellung von pIRES hNgR hp75 (Fig. 3a)

pcDNA hNgR CDS1, kommerziell erworben von RZPD (Deutsches Resourcenzentrum für Genomforschung GmbH, Klon.Nr.: IRAL-p962L1427Q2), enthaltend die kodierende Sequenz für humanen Nogo-Rezeptor, wurde unter Verwendung eines Restriktionsansatzes 13 µl;1 h bei 37 °C) enthaltend 5 µg DNA, 1,5 µl Hind III (Roche), 1,5 µl 10x Puffer B (Roche) und 10 µl Wasser bidest verdaut (blunt end). Das ausgeschnittene Fragment wurde mit Hilfe des Qiagen Gelextraktionskit aus einem DNA-Gel in herkömmlicher Weise gewonnen.

Zum Auffüllen der Enden wurden 20 µl des geschnittenes pcDNA h NgR CDS1-Konstrukt in einem Ansatz (100µl; 30 min 11 °C), enthaltend 2 µl T4-DNA Polymerase (Roche), 10 µl 10x Puffer T4 (Roche), 1µl 100 mM DTT (Gibco), 10 µl 20 mM NTP Mix (Stratagene) und 77 µl Wasser bidest. inkubiert. Das T4-fill-in wurde mit Hilfe des Qiagen Gelextraktionskit aus einem DNA-Gel gewonnen.

Zur Dephosphorylierung, um eine Re-Ligierung der blunt ends zu vermeiden, wurden 50 µl des T4 fill-ins des h NgR CDS1-Konstruktes mit 2 µl Alkalische Phosphatase (Roche), 10 µl 10x Puffer (Roche) und 38 µl Wasser bidest. zunächst 30 min bei 37 °C und anschließend 15 min bei 65 °C inkubiert. Das so erhaltenen Dephosphorylierungskonstrukt wurde schließlich mit Qiagen Hit clean-up aufgereinigt.

Das gereinigte pcDNA h NgR Hind II blunt Fragment wurde dann mit EcoR1 unter Verwendung eines Restriktionsansatzes (15 µl,1 h bei 37 °C), enthaltend 10 µl Eluat (aus Qiagen Hit clean-up), 1,5 µl EcoR I (Roche), 1,5 µl 10x Puffer H (Roche) und 2,0 µl Wasser bidest., verdaut und mit Hilfe des Qiagen Gelextraktionskit aus einem DNA-Gel nach Restriktion gewonnen.

pIRES hp75 (wie oben hergestellt) wurde mit EcoR I und Hind III unter Verwendung eines Restriktionsansatzes (27 µl, 1,5 h bei 37 °C), enthaltend 5 µg DNA, 1,5 µl EcoR I, 1,5 µl Hind III, 4 µl 10x Puffer H (Roche) und 20 µl Wasser bidest. geöffnet. Das gewünschte hP75 Fragment wurde mit Hilfe des Qiagen Gelextraktionskit aus einem DNA-Gel nach Restriktion gewonnen und mit dem oben hergestellten hNgR-Konstrukt unter Verwendung eines Ligationsansatzes, enthaltend 5 µNgR-Konstrukt, 2 µl geschnittener pIRES hp75, 1,5 µl T4-DNA Ligase (Roche), 3 µl 10x Puffer T4 (Roche) und 20,5 µl Wasser bidest. über Nacht 16 °C ligiert. Das erhaltene Konstrukt pIRES hNgR hp75 (Fig 3a; SEQ ID NO:10) wurde dann in den Bakterienstamm SuperComp XL2 Blue (Stratagene) transformiert.

Weitere Sequenzinformationen zu pIRES hNgR hp75 sind in folgendem Abschnitt zusammengefasst:

| | |
|---|---|
| CMV prom. | 108 - 857 |
| NOGO R | 1202-2620 |
| IRES | 2658-3238 |
| hp75 | 3294-4574 |
| SV40pA | 4648-4869 |
| f1 ori | 4964-5419 |
| Neo | 5483-6856 |
| amp | 7261-8121 |
| pUC ori | 8266-93 compl. |

### d) Herstellung von pcDNA4(mycHis)A hRhoA wt (Fig. 3c)

Das Plasmid pOTB7 RhoA (kommerziell erworben von RZPD, Deutsches Resourcenzentrum für Genomforschung GmbH, Klon.Nr.: IRAL-p962A174), das die kodierende Sequenz für humane RhoA GTPase enthält, wurde unter Verwendung des folgenden Restriktionsansatzes (25 µl, 1,5 h bei 37 °C), enthaltend 5 µg DNA, 1,5 µl EcoR I, 1,5 µl Xho I (Roche), 3 µl 10x Puffer H (Roche) und 19 µl Wasser bidest. verdaut.

In einem zweiten Ansatz wurde pcDNA4(mycHis) (Invitrogen, Carlsbad, USA) unter Verwendung des folgenden Restriktionsansatzes (25 µl, 1,5 h bei 37 °C), enthaltend 5 µg DNA, 1,5 µl EcoR I, 1,5 µl Xho I (Roche), 3 µl 10x Puffer H (Roche) und 19 µl Wasser bidest. ebenfalls verdaut.

Anschließend wurden die geschnittenen Fragmente, wie oben beschrieben gereinigt und ligiert, wobei man das Plasmid pcDNA4(mycHis)A h RhoA wt (vgl. Fig. 3c; SEQ ID NO:13.) erhält. Das erhaltene Konstrukt wurde dann in den Bakterienstamm SuperComp XL2 Blue (Stratagene) transformiert.

Weitere Sequenzinformationen zu pcDNA4(mycHis)A hRhoA wt sind in folgendem Abschnitt zusammengefasst:

| | |
|---|---|
| CMV prom. | 197-851 |
| RhoA | 1058-1636 |
| His | 2437-2454 |
| myc | 2392-2421 |
| Bgh pA | 2480-2707 |
| SV40 pA | 4143-4273 |
| f1ori | 2753-3181 |
| amp | 5477-6334 compl. |
| pUC ori | 4656-5329 |
| Zeo | 3639-4010 |
| EM7 prom. | 3565-3620 |
| SV40 prom. | 3209-3517 |

### d) Herstellung von pcDNA3 hRhoA wt (Fig. 3b)

Die Herstellung erfolgt in Analogie zu pcDNA4(mycHis)A hRhoA wt, wobei man aber anstelle von pcDNA4(mycHis) das oben bereits beschriebenen Plasmid pcDNA3.1V5-His TOPO verwendet.

Weitere Sequenzinformationen zu pcDNA3 hRhoA wt (vgl. Fig. 3b; SEQ ID NO15) sind in folgendem Abschnitt zusammengefasst:

| | |
|---|---|
| CMV prom. | 6124-6778 |
| h RhoA | 127-705 |
| Bgh pA | 1478-1718 |
| SV40 pA | 3402-3597 |
| SV40 ori | 2259-2584 |
| ColE1 ori | 4141-4515 |
| f1 ori | 1788-2201 |
| Neo | 2620-3411 |
| amp | 4919-5779 compl. |

### Beispiel 3: Generierung stabiler rekombinanter HEK Zelllinien

### a) Herstellung der Doppel-Transfektante HEK293 NgR/p75

HEK293 Wildtyp- Zellen (kultiviert in RPMI -Glutamax + 10% dial. FCS + 1% Antibiotic-Antimycotic) wurden in einem ersten Transfektionsschritt mit dem Plasmid piRES hNgR hp75 transfiziert. Dazu wurden in Kulturschalen mit 10 Vertiefungen je Ansatz 2 µg Plasmid-DNA in 100 µl serumfreien RPMI-Gutamax Medium und 2 x 10⁶ Zellen in 12 µl LIPOFECTAMINE in 100 µl serumfreien RPMI-Gutamax Medium vermischt, und 15 bis 20 Minuten bei Raumtemperatur inkubiert. Man füllte dann mit serumfreien Medium auf ein Gesamtvolumen von 1 ml je Transfektionsansatz auf. Anschließend gab man zu jeder Schale 2 ml serumfreies RPMI-Gutamax Medium und man inkubierte 6 h bei 37 °C. Dann erfolgte ein Mediumwechsel auf RPMI-Glutamax + 5% dialysiertes FCS und inkubierte 1 Tag bei 37 °C. Anschließend wurden die Schaleninhalte gesplittet (in unterschiedlichen Verdünnungen: 1:10; 1:50, 1:100, 1:250, 1:500, 1:1000; 1 Ansatz/ Schale pro Verdünnung) (in RPMI-Glutamax + 10% dialysiertes FCS + 1% Antibiotic-Antimycotic + G418; 800 µg/ml)

Klone wurden aus derjenigen Verdünnung isoliert, die die ersten separierten Klone ergab. Sterile Mini-Glaszylinder (BASF) wurden mit einer sterilen Pinzette mit einem Ende in sterile Vaseline (BASF) vorsichtig eingetaucht. Das mit der Vaseline benetzte Ende der Mini-Glaszylinder wurde auf die zuvor ausgewählten Einzelklone vorsichtig aufgesetzt. Der Einzelklon sollte vollständig vom Mini-Glaszylinder umschlossen sein. In die Mini-Glaszylinder wurden nun mit einer Eppendorf-Pipette mit steriler Spitze 40 µl Trypsin (Gibco, Trypsin-EDTA) zugegeben. Das Trypsin ließ man 1-2 Minuten auf die Zellen einwirken. Durch mehrmaliges (3-4 Mal) Anziehen und Abgeben des Trypsins mit einer Eppendorf-Pipette (sterile Spitze) wurden die Zellen resuspendiert. Die resuspendierten Zellen wurden mit der Eppendorf-Pipette vollständig vom Mini-Glaszylinder in eine 24-well Platte (Tissue culture plate, Falcon, Becton Dickinson, jedes well enthielt 1 ml Medium RPMI-Glutamax + 5% dialysiertes FCS) überführt.

Der Nachweis von positiven Klonen erfolgte mittels FACS wie oben beschrieben. Dabei wurde die Überexpression der Rezeptoren NgR und p75 an der Zelloberfläche bestimmt (Ergebnisse nicht gezeigt).

### b) Herstellung der Triple-Transfektante HEK293 RhoA/NgR/p75

Ein gemäß a) hergestellter positiver Klon (Klon 5) der Zelllinie HEK293 NgR/p75 wird in analoger Weise mit dem Plasmid pcDNA4(mycHis)A hRhoA transfiziert. Im letzten Schritt erfolgte eine Splittung der Ansätze in RPMI-Glutamax (+ 10% dialysiertes. FCS + 1% Antibiotic-Antimycotic + G418; 800 µg/ml, + Zeocin 125 µg/ml)

Zum Nachweis positiver Klone wurde diese durch Immunoblotting auf RhoA Expression und durch FACS Analyse auf Expression der Rezeptoren NgR und p75 getestet.

Zum RhoA-Nachweis wurde ein von dem jeweiligen Klon abgeleitetes Zellhomogenat durch SDS-PAGE-Gelelektrophorese (NuPAGE Polyacrylamid Gel 4-12%, 1,5 mm stark (Invitrogen Carlsbad, USA); die Proteinproben wurden mit 5% Mercaptoethanol denaturiert) aufgetrennt und nach Immunoblotting mit monoklonalem Maus-anti-RhoA Antikörper nach Standardmethode getestet. Ein typisches Ergebnis ist in Figur 4a dargestellt. Bei positiven Klonen beobachtet man eine RhoA-Bande mit einem Molekulargewicht von etwa 21 kD.

Zum Nachweis der Expression von NgR und p75 wurde eine FACS-Analyse wie oben beschrieben durchgeführt. Ein typisches Ergebnis ist in Figur 4b dargestellt.

### c) Herstellung der Einfach-Transfektante HEK293 RhoA

HEK293 wt Zellen werden mit dem Plasmid pcDNA3 hRhoA wt transfiziert. Im letzten Schritt erfolgte eine Splittung der Ansätze in RPMI-Glutamax (+ 10% dialysiertes. FCS + 1% Antibiotic-Antimycotic + G418; 800 µg/ml). Ansonsten wurde in Analogie zu a) und b) verfahren.

### Beispiel 4: Stimulation von NgR mit AP-Nogo66 aktiviert Rho

### a) RBD-Assay

Unter Verwendung des oben beschriebenen RBD-Assays wurde untersucht, ob Rho nach einer Stimulation von NgR mit Myelinproteinen, wie Nogo66, in HEK293-Rho-Zellen, die NgR und p75 exprimieren (HEK293-Rho/NgR/p75), aktiviert wird. Die Ergebnisse sind in beiliegender Figur 5a dargestellt

**Ansatz I:** HEK293-RhoA/NgR/p75-Zellen wurden entweder nicht stimuliert (-) oder 5 Minuten mit L-alpha-Lysophosphatidsäure (LPA) stimuliert (+). LPA ist ein allgemeiner Rho-Aktivator, der als positive Kontrolle der Aktivität der GTPase in verschiedenen Zellen dient. Eine Stimulation mit LPA führte zu einer raschen Aktivierung von Rho in HEK293-RhoA/NgR/p75-Zellen sowie HEK-Wildtypzellen (Daten nicht gezeigt). Dies zeigt, dass die LPA-induzierte Rho-Aktivierung im erfindungsgemäßen Zellsystem nicht von der NgR-Expression abhängig ist.

**Ansatz II:** HEK293-RhoA/NgR/p75-Zellen wurden entweder nicht stimuliert (-) oder 5 Minuten mit AP-Nogo66 stimuliert (+). Im Vergleich zur LPA-Behandlung induzierte eine Stimulation mit Nogo66 Rho-Aktivität mit ähnlichem zeitlichen Verlauf.

**Ansatz III:** HEK293-RhoA/NgR/p75-Zellen wurden mit 1 U/ml PI-PLC 30 Minuten vor Beginn des Experiments behandelt. Anschließend wurden die Zellen entweder nicht stimuliert (-) oder mit AP-Nogo66 (+) wie oben beschrieben stimuliert. Eine Stimulation mit AP-Nogo66 nach Freisetzung von NgR aus der Zellmembran führte zu keiner Rho-Aktivierung. Dies veranschaulicht, dass diese GTPase Teil einer Signalkaskade ist, die spezifisch über den Ligand-aktivierten NgR induziert wird.

Jedes Experiment wurde wenigstens 3 Mal wiederholt.

### b) ACR-Assay

HEK293-Rho/NgR/p75-Zellen wurden in Mikrotiterplatten mit 96 Vertiefungen (Biocoat Poly D-Lysine Black Clear Plates von Becton Dickinson, Heidelberg, Bestellnummer 356640) überimpft. Nach einer 2-stündigen Hungerperiode (vgl. oben) wurden die Zellen 30 Minuten mit PI-PLC (1 U/ml) vorinkubiert oder nicht behandelt. Anschließend wurden die Zellen mit LPA oder AP-Nogo66 stimuliert oder blieben unstimuliert. Fluoreszenzmikrogramme wurden wie oben beschrieben erhalten. Repräsentative Abbildungen sind in Figur 5b dargestellt.

Die Abbildungen gemäß Figur 5b belegen den überraschenden Befund, dass eine signifikante Anzahl von Rho-exprimierenden HEK293-RhoA/NgR/p75-Zellen nach Stimulation Veränderungen im Cytoskelett zeigen, wie zum Beispiel eine Abrundung der Zellen, begleitet von einer Zellschrumpfung und der Ausbildung dynamischer Zellvorsprünge. Beispiele repräsentativer Zellen sind in der Figur 5b mit Pfeilen gekennzeichnet.

Wie die obigen Versuche belegen, sind diese morphologischen Veränderungen abhängig von der Rho-Aktivierung und sind nicht in unstimulierten, Rezeptorkompetenten Zellen zu beobachten (Figur 5b II, linke Abbildung). Auch sind diese Veränderungen nicht in NgR-exprimierenden Zellen nach PI-PLC-Behandlung und AP-Nogo66-Stimulation zu beobachten (Figur 5b III, rechtes Bild).

### Beispiel 5: Bildanalyse mikroskopischer Aufnahmen aus erfindungsgemäßen ACR-Assays

Wie durch beiliegende Figuren 6a bis 6g veranschaulicht, eröffnet eine Analyse der erfindungsgemäßen Daten mit Bildanalysentechniken die Möglichkeit, rasche Information über potentielle NgR-Antagonisten zu erhalten. Die Verwendung von High Content Image Analysis Screening Systemen ermöglicht die Umgehung von Problemen, wie mangelnder Reproduzierbarkeit und hohem Zeitaufwand, mit welchem typischerweise der klassische RBD-Test verknüpft ist.

Fluoreszenzmikrogramme (Figur 6a), die aus einem erfindungsgemäß durchgeführten ACR-Assay erhalten wurden (Stimulation von HEK293 RhoA/NgR/p75 Zellen mit 10µg/ml AP-Nogo66), wurden mit unterschiedlichen High content screening Systemen, z.B. mit dem Bildanalysesystem Atto Pathway HT von BD Bioimages (Becton Dickinson) oder dem Discovery-1 von Molecular Devices, auf Veränderungen in der Zellmorphologie untersucht. Figur 6b zeigt das Ergebnis der Bestimmung der durchschnittlichen prozentualen Zelllänge, ermittelt mit Hilfe des Discovery-1-Systems. Mit dem Pathway HT wurde die Anzahl von sogenannten "cytoplasmic verticies" (cytoplasmic verticies sind ein Maß an Veränderungen am Cytoskelett, die u.a. die Kontraktion von Aktinfilamenten und die Bildung von Stressfasem beschreibt.) bestimmt. Das Ergebnis dieser Auswertung ist in Figur 6c dargestellt. Man beobachtet eine signifikante, optisch analysierbare morphologische Veränderung in den erfindungsgemäßen Zellen nach Stimulation mit dem bekannten NgR-Liganden.

Ein weiteres spezifisches Beispiel für einen zellbasierenden Test, der auf einem spezifischen Algorithmus beruht, welcher eine robuste und reproduzierbare Bildanalyse erlaubt, wird nun im Folgenden beschrieben.

Um die bereits genannten morphologischen Änderungen zu analysieren, wurden 1 x 10⁴ Zellen pro Vertiefung in 96er Platten angeimpft und fixiert. Die Aktinfilamente wurden mit FITC-markiertem Alexa Phalloidin angefärbt, und die Kerne wurden mit DAPI visualisiert. Die Platten wurden anschließend dem erfindungsgemäßen High-Content-Screening-(HCS)-System, dem Pathway HT (Becton Dickinson) und der entsprechenden Bildanalyse-Software Attovision unterzogen. Eine Analyse des Umfangs des Aktin-Cytoskeletts ergab die signifikantesten Unterschiede bei Vergleich von stimulierten und nichtstimulierten Proben. Repräsentative Abbildungen, welche die Segmentierung und Algorhythmus-Detektion veranschaulichen, sind in Figur 6d gezeigt. Der Umfang des Aktin-Cytoskeletts wurde über die "cytoplasmic verticies" bestimmt, welche ein Maß für die Anzahl von Richtungsänderungen darstellen.

Unter Verwendung dieses Parameters wurde das Dosis-Ansprechverhalten von AP-Nogo66 in HEK293-RhoA/NgR/p75 untersucht (Figur 6e). Die mittlere Anzahl von cytoplasmic verticies unstimulierter Zellen, die als Kontrolle diente, wurde bestimmt und mit der entsprechenden mittleren Anzahl für Zellen verglichen, welche mit steigenden AP-Nogo66-Konzentrationen stimuliert wurden. Die Attovisionsanalyse der AP-Nogo66-induzierten Zellkontraktion ergab eine maximale Verringerung des mittleren Durchmeters von etwa 80 bis 90 % im Vergleich zu unbehandelten Kontrollzellen nach Stimulation mit 20 nM AP-Nogo66 (Figur 6e). Jeder Balken veranschaulicht den Mittelwert aus wenigstens drei unabhängigen Vertiefungen. Die bestimmte Standardabweichung veranschaulicht die Signifikanz und Robustheit des evaluierten Parameterunterschieds.

Die Zeitabhängigkeit der AP-Nogo66-induzierten Aktin-Cytoskelett-Umordnung wurde ebenfalls untersucht (Figur 6f). Jeder Balken veranschaulicht wiederum den Mittelwert von drei unabhängigen Vertiefungen. Die Stimulation von HEK293-RhoA/NgR/p75-Zellen führte zu raschen morphologischen Veränderungen innerhalb von 5 Minuten und erreichte ein Maximum nach 15 Minuten. Dieser zeitliche Verlauf bestätigt den zeitlichen Verlauf der Rho-Aktivierung, bestimmt mit Hilfe des herkömmlichen Pulldown-Assays mit der gleichen Zelllinie.

Aufgrund der Verfügbarkeit von verschiedenen Rho überexprimierenden Zelllinien wurden Signaltransduktionsuntersuchungen in kontrollierter Weise durchgeführt und wurden zur Validierung des Aktin-Cytoskelett-Rearrangement-(ACR)-Assays verwendet. Aktiviertes GTP-gebundenes Rho ist zur Rekrutierung und Aktivierung der Serin-Threonin-Kinase ROCK, einem spezifischen Downstream-Target von Rho befähigt. Die ROCK-Aktivierung führt zu einer Phosphorylierung mehrerer Cytoskelett-Proteine, was zu einer Zellkontraktion führt (Mills et al., 1998, J. Cell. Biol. 140(3): 627-636). Die Inhibition von ROCK mit Y-27632 (bezogen von Calbiochem, La Jolla, Kalifornien, USA) hob die AP-Nogo66-induzierte, Rho-abhängige Zellabrundung und -schrumpfung in dosisabhängiger Weise auf (Figur 6 g). Dies bestätigt, dass die NgR-induzierten Veränderungen der Zellmorphologie die Aktivität dieses speziellen Effektorproteins erfordern, welche die Rho-Aktivierung direkt mit dem Aktin-Cytoskelett verknüpfen. Darüber hinaus unterstrich die pharmakologische Analyse des inhibitorischen Effektes von Y-27632 auf die Aktin-Cytoskelett-Umordnung die gute Reproduzierbarkeit. Der bestimmte IC50-Wert für Y-27532 lag bei dieser Testanordnung in der Größenordnung von dem, was für diese Verbindung bereits beschrieben worden war (Mills et al., 1998 a.a.O.). Darüber hinaus ergab die Bestimmung eines Z'-Faktors von 0,6 (n = 30 Vertiefungen wurden analysiert) eine zufriedenstellende Robustheit des beschriebenen Tests für die Analyse von Verbindungen.

## Patentansprüche

1. Verwendung einer rekombinanten Zelllinie, welche exprimiert:
a) eine RhoGTPase als funktionalen intrazellulären Faktor, der an einem eine Veränderung im Cytoskelett der Zelle induzierenden Signalweg beteiligt ist; und
b) einen mit der RhoGTPase wechselwirkenden membrangebundenen Rezeptor, umfassend einen funktionalen Neurotrophin Rezeptor p75 und einen funktionalen Nogo Rezeptor (NgR);
wobei die rekombinante Zelllinie mit den kodierenden Sequenzen für die RhoGTPase, p75 und NgR transformiert ist;
zur Bestimmung von Effektoren der NgR-abhängigen Signaltransduktion über Veränderungen im Aktin-Cytoskelett von Zellen der Zelllinie.

2. Verwendung nach Anspruch 1, wobei
a) die funktionale RhoGTPase eine Aminosäuresequenz gemäß SEQ ID NO:16 umfasst;
b) der funktionale p75 eine Aminosäuresequenz gemäß SEQ ID NO:9 umfasst; und
c) der funktionale NgR eine Aminosäuresequenz gemäß SEQ ID NO:11 umfasst.

3. Verwendung nach einem der vorhergehenden Ansprüche, wobei die Zellinie zu einer Reorganisation ihres Aktin-Cytoskeletts, induziert durch die Bindung eines Liganden an NgR befähigt ist.

4. Verwendung nach Anspruch 3, wobei die Ligandenbindung eine Aktivierung von Rho induziert.

5. Verwendung nach Anspruch 3 oder 4, wobei der Ligand Nogo-A oder ein davon abgeleitetes NgR bindendes Fragment oder Derivat umfasst.

6. Verwendung nach einem der vorhergehenden Ansprüche, wobei die Zelllinie abgeleitet ist von einer adhärenten Säugerzelllinie mit ausgespreiteter Zellmorphologie.

7. Verwendung nach Anspruch 6, wobei die Zelllinie abgeleitet ist von einer humanen Epithelial- oder Fibroblastenzelllinie.

8. Verwendung nach Anspruch 7, wobei die Zelllinie abgeleitet ist von einer humanen embryonalen Nieren (HEK)-Zelllinie.

9. Testmethode zur Bestimmung von Veränderungen im Cytoskelett einer zu untersuchenden Zelllinie, wobei man eine rekombinante Zelllinie gemäß der Definition nach einem der Ansprüche 1 bis 8 mit einem mit den Zellen wechselwirkenden Liganden kultiviert, der gegebenenfalls die Veränderung im Cytoskelett über die Induktion der NgR-abhängigen Signaltransduktion bewirkt, und Veränderungen in der Reorganisation des Cytoskeletts der so behandelten Zelllinien bestimmt.

10. Testmethode nach Anspruch 9 wobei der Ligand mit einem membrangebunden Rezeptor wechselwirkt.

11. Testmethode nach Anspruch 9 oder 10, wobei man die Zelllinie in Gegenwart oder Abwesenheit eines Analyten kultiviert, von dem man vermutet, dass er einen Effektor enthält, der die Wirkung des Liganden auf die Zelle moduliert.

12. Testmethode nach Anspruch 11, wobei die Kultivierung mit dem Analyten gleichzeitig oder zeitlich versetzt zur Kultivierung mit dem rezeptorbindenen Liganden erfolgt.

13. Testmethode nach einem der Ansprüche 9 bis 12 zur Bestimmung von Effektoren der NgR-abhängigen Aktivierung der GTPase Rho, wobei man die rekombinante Zelllinie in Gegenwart eines NgR bindenden Liganden und in Gegenwart oder Abwesenheit eines Analyten kultiviert, von dem man vermutet, dass er den Effektor enthält, und Unterschiede in der Reorganisation des Cytoskeletts der so behandelten Zelllinien bestimmt.

14. Testmethode nach einem der Ansprüche 9 bis 13, wobei man den Mittelwert wenigstens eines der folgenden Parameter für eine repräsentative Zellpopulation bestimmt:
a) Zellumfang
b) Zelllänge
c) Zellbreite
d) Zellfläche
e) Länge der Zellausläufer
f) Zelldichte
g) Cytoplasmatische Fläche
h) cytoplasmic verticies
i) Brechungsindex
j) zellfreie Fläche
k) mit Zellen belegte Fläche
l) eine mathematische Verknüpfung von wenigstens zwei der Parameter a) bis k), oder einen von wenigstens einem der Parameter a) bis k) ableitbaren Parameter, wie insbesondere das Verhältnis von Zelllänge zu Zellbreite, oder das Verhältnis von Zellumfang im Quadrat zu Zellfläche; oder
m) einen entsprechenden reziproken Wert eines der Parameter a) bis I).

15. Testmethode nach einem der Ansprüche 9 bis 14, wobei die Bestimmung mittels eines automatisierten Verfahrens, wie insbesondere mittels eines automatischen Bildanalysesystems, erfolgt.

16. Verwendung einer Testmethode nach einem der Ansprüche 9 bis 15 zur Bestimmung von Effektoren der neuronalen Regeneration, wobei diese Effektoren der NgR-abhängigen Signaltransduktion sind.

17. Verwendung nach Anspruch 16, wobei der Effektor die Rho-Aktivierung teilweise oder vollständig inhibiert.

18. Verwendung nach einem der Ansprüche 16 und 17, wobei der Effektor die neuronale Regeneration fördert.

19. Verwendung nach einem der Ansprüche 1 bis 8, wobei der Effektor eine krankheits- oder verletzungsbedingte Inhibition der axonalen Regeneration oder einen krankheits- oder verletzungsbedingten Kollaps des neuronalen Wachstumskegels teilweise oder vollständig neutralisiert.

20. Verwendung nach einem der Ansprüche 16 bis 19, wobei der Effektor ein NgR-Antagonist ist.

21. Verwendung nach einem der Ansprüche 16 bis 20 in einem Screeningverfahren, insbesondere high-content-Screeningverfahren für Effektoren der neuronalen Regeneration.

22. High Content Screening-Vefahren zur Bestimmung von Effektoren der NgR-abhängigen Aktivierung der GTPase Rho, wobei man eine Testmethode nach einem der Ansprüche 9 bis 15 anwendet.

23. Verwendung eines Testkits, umfassend eine rekombinante Zelllinie gemäß der Definition in einem der Ansprüche 1 bis 8 sowie einen Liganden für den zellulären Rezeptor, zur Bestimmung von Effektoren der NgR-abhängigen Signaltransduktion über Veränderungen im Aktin-Cytoskelett von Zellen der Zelllinie.

## Claims

1. The use of a recombinant cell line which expresses:
a) a Rho GTPase as functional intracellular factor which is involved in a signal pathway which induces a change in the cytoskeleton of the cell; and
b) a membrane-bound receptor which interacts with the Rho GTPase, comprising a functional neurotrophin receptor p75 and a functional Nogo receptor (NgR);
where the recombinant cell line is transformed with the coding sequences for RhoGTPase, p75 and NgR;
for determining effectors of NgR-dependent signal transduction via changes in the actin cytoskeleton of cells of the cell line.

2. The use according to claim 1, where
a) the functional RhoGTPase comprises an amino acid sequence as shown in SEQ ID NO: 16,
b) the functional p75 comprises an amino acid sequence as shown in SEQ ID NO: 9; and
c) the functional NgR comprises an amino acid sequence as shown in SEQ ID NO: 11.

3. The use according to either of the preceding claims, where the cell line is able to reorganize its actin cytoskeleton induced by the binding of a ligand to NgR.

4. The use according to claim 3, where the ligand binding induces an activation of Rho.

5. The use according to claim 3 or 4, where the ligand comprises Nogo-A or an NgR-binding fragment or derivative derived therefrom.

6. The use according to any of the preceding claims, where the cell line is derived from an adherent mammalian cell line with extended cell morphology.

7. The use according to claim 6, where the cell line is derived from a human epithelial or fibroblast cell line.

8. The use according to claim 7, where the cell line is derived from a human embryonic kidney (HEK) cell line.

9. An assay method for determining changes in the cytoskeleton of a cell line to be investigated, where a recombinant cell line as defined in any of claims 1 to 8 is cultivated with a ligand which interacts with the cells and which, optionally, brings about the change in the cytoskeleton via induction of NgR-dependent signal transduction, and changes in the reorganization of the cytoskeleton of the cell lines treated in this way are determined.

10. The assay method according to claim 9, where the ligand interacts with a membrane-bound receptor.

11. The assay method according to claim 9 or 10, where the cell line is cultivated in the presence or absence of an analyte which is suspected to comprise an effector which modulates the effect of the ligand on the cell.

12. The assay method according to claim 11, where the cultivation with the analyte takes place simultaneously or sequentially in relation to the cultivation with the receptor-binding ligand.

13. The assay method according to any of claims 9 to 12 for determining effectors of NgR-dependent activation of the GTPase Rho, where the recombinant cell line is cultivated in the presence of an NgR-binding ligand and in the presence or absence of an analyte suspected to comprise the effector, and differences in the reorganization of the cytoskeleton of the cell lines treated in this way are determined.

14. The assay method according to any of claims 9 to 13, where the average of at least one of the following parameters is determined for a representative cell population:
a) cell circumference
b) cell length
c) cell width
d) cell area
e) length of the cell projections
f) cell density
g) cytoplasmic area
h) cytoplasmic vertices
i) refractive index
j) cell-free area
k) area covered with cells
l) a mathematical linkage of at least two of parameters a) to k), or a parameter which can be derived from at least one of the parameters a) to k), such as, in particular, the ratio of cell length to cell width, or the ratio of cell circumference squared to cell area; or
m) an appropriate reciprocal value of one of the parameters a) to l).

15. The assay method according to any of claims 9 to 14, where the determination takes place by means of an automated method such as, in particular, by means of an automatic image analysis system.

16. The use of an assay method according to any of claims 9 to 15 for determining effectors of neuronal regeneration, where these are effectors of NgR-dependent signal transduction.

17. The use according to claim 16, where the effector partly or completely inhibits Rho activation.

18. The use according to either of claims 16 and 17, where the effector promotes neuronal regeneration.

19. The use according to any of claims 1 to 8, where the effector partly or completely neutralizes a disease- or injury-related inhibition of axonal regeneration or a disease- or injury-related collapse of the neuronal growth cone.

20. The use according to any of claims 16 to 19, where the effector is an NgR antagonist.

21. The use according to any of claims 16 to 20 in a screening method, in particular a high content screening method for effectors of neuronal regeneration.

22. A high content screening method for determining effectors of NgR-dependent activation of the GTPase Rho, where an assay method according to any of claims 9 to 15 is used.

23. The use of a test kit comprising a recombinant cell line as defined in any of claims 1 to 8, and a ligand for the cellular receptor, for determining effectors of NgR-dependent signal transduction via changes in the actin cytoskeleton of cells of the cell line.

## Revendications

1. Utilisation d'une lignée cellulaire recombinante exprimant :
a) une GTPase Rho en tant que facteur fonctionnel intracellulaire participant à une voie de signalisation induisant une modification du cytosquelette de la cellule ;
et
b) un récepteur lié à la membrane interagissant avec la GTPase Rho, comprenant un récepteur des neurotrophines fonctionnel p75 et un récepteur Nogo fonctionnel (NgR) ;
où ladite lignée cellulaire recombinante est transformée avec les séquences codantes pour la GTPase Rho, p75 et NgR ;
pour la détection d'effecteurs de la transduction de signal dépendante NgR au moyen de modifications du cytosquelette d'actine de cellules de la lignée cellulaire.

2. Utilisation selon la revendication 1, où :
a) la GTPase Rho fonctionnelle comprend une séquence d'acide aminé décrite dans SEQ ID NO:16 ;
b) le p75 fonctionnel comprend une séquence d'acide aminé décrite dans SEQ ID NO:9 ;
c) le NgR fonctionnel comprend une séquence d'acide aminé décrite dans SEQ ID NO:11.

3. Utilisation selon l'une des revendications précédentes, où la lignée cellulaire est rendue apte à une réorganisation de son cytosquelette d'actine induite par la liaison d'un coordinat à NgR.

4. Utilisation selon la revendication 3, où la liaison de coordinat induit une activation de Rho.

5. Utilisation selon la revendication 3 ou la revendication 4, où le coordinat comprend le Nogo-A, ou un fragment ou un dérivé de celui-ci liant le NgR.

6. Utilisation selon l'une des revendications précédentes, où la lignée cellulaire est dérivée d'une lignée cellulaire de mammifère adhérente à morphologie cellulaire étirée.

7. Utilisation selon la revendication 6, où la lignée cellulaire est dérivée d'une lignée cellulaire épithéliale ou fibroblaste humaine.

8. Utilisation selon la revendication 7, où la lignée cellulaire est dérivée d'une lignée cellulaire rénale embryonnaire (HEK) humaine.

9. Méthode de test pour la détection de modifications du cytosquelette d'une lignée cellulaire à examiner, où une lignée cellulaire recombinante conforme à la définition selon l'une des revendications 1 à 8 est cultivée avec un coordinat interagissant avec les cellules, lequel provoque le cas échéant la modification du cytosquelette par induction de la transduction de signal dépendante NgR, et où des modifications sont détectées dans la réorganisation du cytosquelette des lignées cellulaires ainsi traitées.

10. Méthode de test selon la revendication 9, où le coordinat interagit avec un récepteur lié à la membrane.

11. Méthode de test selon la revendication 9 ou la revendication 10, où la lignée cellulaire est cultivée en présence ou en l'absence d'une analyte dont il est supposé qu'elle contient un effecteur qui module l'effet du coordinat sur la cellule.

12. Méthode de test selon la revendication 11, où la culture avec l'analyte est effectuée simultanément à la culture avec le coordinat liant le récepteur, ou en décalage temporel avec celle-ci.

13. Méthode de test selon l'une des revendications 9 à 12 pour la détection d'effecteurs de l'activation dépendante NgR de la GTPase Rho, où la lignée cellulaire recombinante est cultivée en présence d'un coordinat liant le NgR et en présence ou en l'absence d'une analyte dont il est supposé qu'elle contient un effecteur, et où des différences sont détectées dans la réorganisation du cytosquelette des lignées cellulaires ainsi traitées.

14. Méthode de test selon l'une des revendications 9 à 13, où la moyenne d'au moins un des paramètres suivants est déterminée pour une population cellulaire représentative :
a) périmètre cellulaire
b) longueur cellulaire
c) largeur cellulaire
d) surface cellulaire
e) longueur des prolongements cellulaires
f) densité cellulaire
g) surface cytoplasmique
h) cytoplasmic verticies
i) indice de réfraction
j) surface exempte de cellules
k) surface occupée par des cellules
l) une relation mathématique entre au moins deux des paramètres a) à k), ou un paramètre dérivable d'au moins un des paramètres a) à k), tel que le rapport de la longueur cellulaire à la, largeur cellulaire, ou le rapport du périmètre cellulaire au carré à la surface cellulaire ;
ou
m) une valeur réciproque correspondante d'un des paramètres a) à l).

15. Méthode de test selon l'une des revendications 9 à 14, où la détection est effectuée au moyen d'un procédé automatisé, tel qu'un système d'analyse d'image automatique.

16. Utilisation d'une méthode de test selon l'une des revendications 9 à 15 pour la détection d'effecteurs de la régénération neuronale, ceux-ci étant des effecteurs de la transduction de signal dépendante NgR.

17. Utilisation selon la revendication 16, où l'effecteur inhibe partiellement ou totalement l'activation Rho.

18. Utilisation selon l'une des revendications 16 et 17, où l'effecteur assiste la régénération neuronale.

19. Utilisation selon l'une des revendications 1 à 8, où l'effecteur neutralise partiellement ou totalement une inhibition de la régénération axonale due à la maladie ou à une blessure, ou un collapsus du cône de croissance neuronal du à la maladie ou à une blessure.

20. Utilisation selon l'une des revendications 16 à 19, où l'effecteur est un antagoniste du NgR.

21. Utilisation selon l'une des revendications 16 à 20 dans un procédé d'imagerie, en particulier un procédé d'imagerie haute densité pour des effecteurs de la régénération neuronale.

22. Procédé d'imagerie haute densité pour la détection d'effecteurs de l'activation dépendante NgR de la GTPase Rho, où il est recouru à une méthode de test selon l'une des revendications 9 à 15.

23. Utilisation d'un kit de test, comprenant une lignée cellulaire recombinante conforme à la définition selon l'une des revendications 1 à 8 ainsi qu'un coordinat pour le récepteur cellulaire, pour la détection d'effecteurs de la transduction de signal dépendante NgR au moyen de modifications du cytosquelette d'actine de cellules de la lignée cellulaire.


### SEQUENCE LISTING

<110> Abbott GmbH & CO. KG
<120> ACR-Assay
<130> M/45212
<160> 16
<170> Patent In version 3.1
<210> 1
   <211> 31
   <212> DNA
   <213> Artificial Sequence
<220>
   <223> PCR primer mez402
<400> 1
   gccaccatga ggatatacaa gggtgtgatc c 31
<210> 2
   <211> 24
   <212> DNA
   <213> Artificial Sequence
<220>
   <223> PCR primer mez404
<400> 2
   cttcagagaa tcaactaaat catc 24
<210> 3
   <211> 20
   <212> DNA
   <213> Artificial Sequence
<220>
   <223> PCR primer mey35
<400> 3
   tcacactggg gatgtggcag 20
<210> 4
   <211> 24
   <212> DNA
   <213> Artificial Sequence
<220>
   <223> PCR primer mey36
<400> 4
   gccaccatgg gggcaggtgc cacc 24
<210> 5
   <211> 18
   <212> DNA
   <213> Artificial Sequence
<220>
   <223> PCR primer mey71
<400> 5
   gcagccccat cagtccgc 18
<210> 6
   <211> 5523
   <212> DNA
   <213> Artificial Sequence
<220>
   <223> Plasmid pcDNA3.1V5-HisTOPO
<400> 6
   gacggatcgg gagatctccc gatcccctat ggtcgactct cagtacaatc tgctctgatg 60
   ccgcatagtt aagccagtat ctgctccctg cttgtgtgtt ggaggtcgct gagtagtgcg 120
   cgagcaaaat ttaagctaca acaaggcaag gcttgaccga caattgcatg aagaatctgc 180
   ttagggttag gcgttttgcg ctgcttcgcg atgtacgggc cagatatacg cgttgacatt 240
   gattattgac tagttattaa tagtaatcaa ttacggggtc attagttcat agcccatata 300
   tggagttccg cgttacataa cttacggtaa atggcccgcc tggctgaccg cccaacgacc 360
   cccgcccatt gacgtcaata atgacgtatg ttcccatagt aacgccaata gggactttcc 420
   attgacgtca atgggtggac tatttacggt aaactgccca cttggcagta catcaagtgt 480
   atcatatgcc aagtacgccc cctattgacg tcaatgacgg taaatggccc gcctggcatt 540
   atgcccagta catgacctta tgggactttc ctacttggca gtacatctac gtattagtca 600
   tcgctattac catggtgatg cggttttggc agtacatcaa tgggcgtgga tagcggtttg 660
   actcacgggg atttccaagt ctccacccca ttgacgtcaa tgggagtttg ttttggcacc    720
   aaaatcaacg ggactttcca aaatgtcgta acaactccgc cccattgacg caaatgggcg    780
   gtaggcgtgt acggtgggag gtctatataa gcagagctct ctggctaact agagaaccca    840
   ctgcttactg gcttatcgaa attaatacga ctcactatag ggagacccaa gctggctagt    900
   taagcttggt accgagctcg gatccactag tccagtgtgg tggaattgcc cttaagggca    960
   attctgcaga tatccagcac agtggcggcc gctcgagtct agagggcccg cggttcgaag   1020
   gtaagcctat ccctaaccct ctcctcggtc tcgattctac gcgtaccggt catcatcacc   1080
   atcaccattg agtttaaacc cgctgatcag cctcgactgt gccttctagt tgccagccat   1140
   ctgttgtttg cccctccccc gtgccttcct tgaccctgga aggtgccact cccactgtcc   1200
   tttcctaata aaatgaggaa attgcatcgc attgtctgag taggtgtcat tctattctgg   1260
   ggggtggggt ggggcaggac agcaaggggg aggattggga agacaatagc aggcatgctg   1320
   gggatgcggt gggctctatg gcttctgagg cggaaagaac cagctggggc tctagggggt   1380
   atccccacgc gccctgtagc ggcgcattaa gcgcggcggg tgtggtggtt acgcgcagcg   1440
   tgaccgctac acttgccagc gccctagcgc ccgctccttt cgctttcttc ccttcctttc   1500
   tcgccacgtt cgccggcttt ccccgtcaag ctctaaatcg gggcatccct ttagggttcc   1560
   gatttagtgc tttacggcac ctcgacccca aaaaacttga ttagggtgat ggttcacgta   1620
   gtgggccatc gccctgatag acggtttttc gccctttgac gttggagtcc acgttcttta   1680
   atagtggact cttgttccaa actggaacaa cactcaaccc tatctcggtc tattcttttg   1740
   atttataagg gattttgggg atttcggcct attggttaaa aaatgagctg atttaacaaa   1800
   aatttaacgc gaattaattc tgtggaatgt gtgtcagtta gggtgtggaa agtccccagg   1860
   ctccccaggc aggcagaagt atgcaaagca tgcatctcaa ttagtcagca accaggtgtg   1920
   gaaagtcccc aggctcccca gcaggcagaa gtatgcaaag catgcatctc aattagtcag   1980
   caaccatagt cccgccccta actccgccca tcccgcccct aactccgccc agttccgccc   2040
   attctccgcc ccatggctga ctaatttttt ttatttatgc agaggccgag gccgcctctg   2100
   cctctgagct attccagaag tagtgaggag gcttttttgg aggcctaggc ttttgcaaaa   2160
   agctcccggg agcttgtata tccattttcg gatctgatca agagacagga tgaggatcgt 2220
   ttcgcatgat tgaacaagat ggattgcacg caggttctcc ggccgcttgg gtggagaggc 2280
   tattcggcta tgactgggca caacagacaa tcggctgctc tgatgccgcc gtgttccggc 2340
   tgtcagcgca ggggcgcccg gttctttttg tcaagaccga cctgtccggt gccctgaatg 2400
   aactgcagga cgaggcagcg cggctatcgt ggctggccac gacgggcgtt ccttgcgcag 2460
   ctgtgctcga cgttgtcact gaagcgggaa gggactggct gctattgggc gaagtgccgg 2520
   ggcaggatct cctgtcatct caccttgctc ctgccgagaa agtatccatc atggctgatg 2580
   caatgcggcg gctgcatacg cttgatccgg ctacctgccc attcgaccac caagcgaaac 2640
   atcgcatcga gcgagcacgt actcggatgg aagccggtct tgtcgatcag gatgatctgg 2700
   acgaagagca tcaggggctc gcgccagccg aactgttcgc caggctcaag gcgcgcatgc 2760
   ccgacggcga ggatctcgtc gtgacccatg gcgatgcctg cttgccgaat atcatggtgg 2820
   aaaatggccg cttttctgga ttcatcgact gtggccggct gggtgtggcg gaccgctatc 2880
   aggacatagc gttggctacc cgtgatattg ctgaagagct tggcggcgaa tgggctgacc 2940
   gcttcctcgt gctttacggt atcgccgctc ccgattcgca gcgcatcgcc ttctatcgcc 3000
   ttcttgacga gttcttctga gcgggactct ggggttcgcg aaatgaccga ccaagcgacg 3060
   cccaacctgc catcacgaga tttcgattcc accgccgcct tctatgaaag gttgggcttc 3120
   ggaatcgttt tccgggacgc cggctggatg atcctccagc gcggggatct catgctggag 3180
   ttcttcgccc accccaactt gtttattgca gcttataatg gttacaaata aagcaatagc 3240
   atcacaaatt tcacaaataa agcatttttt tcactgcatt ctagttgtgg tttgtccaaa 3300
   ctcatcaatg tatcttatca tgtctgtata ccgtcgacct ctagctagag cttggcgtaa 3360
   tcatggtcat agctgtttcc tgtgtgaaat tgttatccgc tcacaattcc acacaacata 3420
   cgagccggaa gcataaagtg taaagcctgg ggtgcctaat gagtgagcta actcacatta 3480
   attgcgttgc gctcactgcc cgctttccag tcgggaaacc tgtcgtgcca gctgcattaa 3540
   tgaatcggcc aacgcgcggg gagaggcggt ttgcgtattg ggcgctcttc cgcttcctcg 3600
   ctcactgact cgctgcgctc ggtcgttcgg ctgcggcgag cggtatcagc tcactcaaag 3660
   gcggtaatac ggttatccac agaatcaggg gataacgcag gaaagaacat gtgagcaaaa 3720
   ggccagcaaa aggccaggaa ccgtaaaaag gccgcgttgc tggcgttttt ccataggctc 3780
   cgcccccctg acgagcatca caaaaatcga cgctcaagtc agaggtggcg aaacccgaca 3840
   ggactataaa gataccaggc gtttccccct ggaagctccc tcgtgcgctc tcctgttccg 3900
   accctgccgc ttaccggata cctgtccgcc tttctccctt cgggaagcgt ggcgctttct 3960
   caatgctcac gctgtaggta tctcagttcg gtgtaggtcg ttcgctccaa gctgggctgt 4020
   gtgcacgaac cccccgttca gcccgaccgc tgcgccttat ccggtaacta tcgtcttgag 4080
   tccaacccgg taagacacga cttatcgcca ctggcagcag ccactggtaa caggattagc 4140
   agagcgaggt atgtaggcgg tgctacagag ttcttgaagt ggtggcctaa ctacggctac 4200
   actagaagga cagtatttgg tatctgcgct ctgctgaagc cagttacctt cggaaaaaga 4260
   gttggtagct cttgatccgg caaacaaacc accgctggta gcggtggttt ttttgtttgc 4320
   aagcagcaga ttacgcgcag aaaaaaagga tctcaagaag atcctttgat cttttctacg 4380
   gggtctgacg ctcagtggaa cgaaaactca cgttaaggga ttttggtcat gagattatca 4440
   aaaaggatct tcacctagat ccttttaaat taaaaatgaa gttttaaatc aatctaaagt 4500
   atatatgagt aaacttggtc tgacagttac caatgcttaa tcagtgaggc acctatctca 4560
   gcgatctgtc tatttcgttc atccatagtt gcctgactcc ccgtcgtgta gataactacg 4620
   atacgggagg gcttaccatc tggccccagt gctgcaatga taccgcgaga cccacgctca 4680
   ccggctccag atttatcagc aataaaccag ccagccggaa gggccgagcg cagaagtggt 4740
   cctgcaactt tatccgcctc catccagtct attaattgtt gccgggaagc tagagtaagt 4800
   agttcgccag ttaatagttt gcgcaacgtt gttgccattg ctacaggcat cgtggtgtca 4860
   cgctcgtcgt ttggtatggc ttcattcagc tccggttccc aacgatcaag gcgagttaca 4920
   tgatccccca tgttgtgcaa aaaagcggtt agctccttcg gtcctccgat cgttgtcaga 4980
   agtaagttgg ccgcagtgtt atcactcatg gttatggcag cactgcataa ttctcttact 5040
   gtcatgccat ccgtaagatg cttttctgtg actggtgagt actcaaccaa gtcattctga 5100
   gaatagtgta tgcggcgacc gagttgctct tgcccggcgt caatacggga taataccgcg 5160
   ccacatagca gaactttaaa agtgctcatc attggaaaac gttcttcggg gcgaaaactc 5220
   tcaaggatct taccgctgtt gagatccagt tcgatgtaac ccactcgtgc acccaactga 5280
   tcttcagcat cttttacttt caccagcgtt tctgggtgag caaaaacagg aaggcaaaat 5340
   gccgcaaaaa agggaataag ggcgacacgg aaatgttgaa tactcatact cttccttttt 5400
   caatattatt gaagcattta tcagggttat tgtctcatga gcggatacat atttgaatgt 5460
   atttagaaaa ataaacaaat aggggttccg cgcacatttc cccgaaaagt gccacctgac 5520
   gtc 5523
<210> 7
   <211> 6850
   <212> DNA
   <213> Artificial Sequence
<220>
   <223> Plasmid pAP-tag/PPC/hNOG066 Nr.5
<400> 7
   aattgcatga agaatctgct tagggttagg cgttttgcgc tgcttcgcga tgtacgggcc 60
   agatatacgc gttgacattg attattgact agttattaat agtaatcaat tacggggtca 120
   ttagttcata gcccatatat ggagttccgc gttacataac ttacggtaaa tggcccgcct 180
   ggctgaccgc ccaacgaccc ccgcccattg acgtcaataa tgacgtatgt tcccatagta 240
   acgccaatag ggactttcca ttgacgtcaa tgggtggact atttacggta aactgcccac 300
   ttggcagtac atcaagtgta tcatatgcca agtacgcccc ctattgacgt caatgacggt 360
   aaatggcccg cctggcatta tgcccagtac atgaccttat gggactttcc tacttggcag 420
   tacatctacg tattagtcat cgctattacc atggtgatgc ggttttggca gtacatcaat 480
   gggcgtggat agcggtttga ctcacgggga tttccaagtc tccaccccat tgacgtcaat 540
   gggagtttgt tttggcacca aaatcaacgg gactttccaa aatgtcgtaa caactccgcc 600
   ccattgacgc aaatgggcgg taggcgtgta cggtgggagg tctatataag cagagctctc 660
   tggctaacta gagaacccac tgcttactgg cttatcgaaa ttaatacgac tcactatagg 720
   gagacccaag ctggctagcc accatggaga cagacacact cctgctatgg gtactgctgc 780
   tctgggttcc aggttccact ggtgacgcgg cccagccggc caggcgcgcg cgccgtacgt 840
   acgaagctta cgtaagatct tccggaatca tcccagttga ggaggagaac ccggacttct 900
   ggaaccgcga ggcagccgag gccctgggtg ccgccaagaa gctgcagcct gcacagacag 960
   ccgccaagaa cctcatcatc ttcctgggcg atgggatggg ggtgtctacg gtgacagctg 1020
   ccaggatcct aaaagggcag aagaaggaca aactggggcc tgagataccc ctggccatgg 1080
   accgcttccc atatgtggct ctgtccaaga catacaatgt agacaaacat gtgccagaca 1140
   gtggagccac agccacggcc tacctgtgcg gggtcaaggg caacttccag accattggct 1200
   tgagtgcagc cgcccgcttt aaccagtgca acacgacacg cggcaacgag gtcatctccg 1260
   tgatgaatcg ggccaagaaa gcagggaagt cagtgggagt ggtaaccacc acacgagtgc 1320
   agcacgcctc gccagccggc acctacgccc acacggtgaa ccgcaactgg tactcggacg 1380
   ccgacgtgcc tgcctcggcc cgccaggagg ggtgccagga catcgctacg cagctcatct 1440
   ccaacatgga cattgacgtg atcctaggtg gaggccgaaa gtacatgttt cccatgggaa 1500
   ccccagaccc tgagtaccca gatgactaca gccaaggtgg gaccaggctg gacgggaaga 1560
   atctggtgca ggaatggctg gcgaagcgcc agggtgcccg gtatgtgtgg aaccgcactg 1620
   agctcatgca ggcttccctg gacccgtctg tgacccatct catgggtctc tttgagcctg 1680
   gagacatgaa atacgagatc caccgagact ccacactgga cccctccctg atggagatga 1740
   cagaggctgc cctgcgcctg ctgagcagga acccccgcgg cttcttcctc ttcgtggagg 1800
   gtggtcgcat cgaccatggt catcatgaaa gcagggctta ccgggcactg actgagacga 1860
   tcatgttcga cgacgccatt gagagggcgg gccagctcac cagcgaggag gacacgctga 1920
   gcctcgtcac tgccgaccac tcccacgtct tctccttcgg aggctacccc ctgcgaggga 1980
   gctccatctt cgggctggcc cctggcaagg cccgggacag gaaggcctac acggtcctcc 2040
   tatacggaaa cggtccaggc tatgtgctca aggacggcgc ccggccggat gttaccgaga 2100
   gcgagagcgg gagccccgag tatcggcagc agtcagcagt gcccctggac gaagagaccc 2160
   acgcaggcga ggacgtggcg gtgttcgcgc gcggcccgca ggcgcacctg gttcacggcg 2220
   tgcaggagca gaccttcata gcgcacgtca tggccttcgc cgcctgcctg gagccctaca 2280
   ccgcctgcga cctggcgccc cccgccggca ccaccgacgc cgcgcacccg ggttatctcg 2340
   aggaagcgct ctctctagat ctggaagttc tgttccaggg gcccctggga tctaggatat 2400
   acaagggtgt gatccaagct atccagaaat cagatgaagg ccacccattc agggcatatc 2460
   tggaatctga agttgctata tctgaggagt tggttcagaa gtacagtaat tctgctcttg 2520
   gtcatgtgaa ctgcacgata aaggaactca ggcgcctctt cttagttgat gatttagttg 2580
   attctctgaa gtagtctaga agggcccgaa caaaaactca tctcagaaga ggatctgaat 2640
   agcgccgtcg accatcatca tcatcatcat tgagtttaaa cccgctgatc agcctcgact 2700
   gtgccttcta gttgccagcc atctgttgtt tgcccctccc ccgtgccttc cttgaccctg 2760
   gaaggtgcca ctcccactgt cctttcctaa taaaatgagg aaattgcatc gcattgtctg 2820
   agtaggtgtc attctattct ggggggtggg gtggggcagg acagcaaggg ggaggattgg 2880
   gaagacaata gcaggcatgc tggggatgcg gtgggctcta tggcttctga ggcggaaaga 2940
   accagctggg gctctagggg gtatccccac gcgccctgta gcggcgcatt aagcgcggcg 3000
   ggtgtggtgg ttacgcgcag cgtgaccgct acacttgcca gcgccctagc gcccgctcct 3060
   ttcgctttct tcccttcctt tctcgccacg ttcgccggct ttccccgtca agctctaaat 3120
   cggggcatcc ctttagggtt ccgatttagt gctttacggc acctcgaccc caaaaaactt 3180
   gattagggtg atggttcacg tagtgggcca tcgccctgat agacggtttt tcgccctttg 3240
   acgttggagt ccacgttctt taatagtgga ctcttgttcc aaactggaac aacactcaac 3300
   cctatctcgg tctattcttt tgatttataa gggattttgg ggatttcggc ctattggtta 3360
   aaaaatgagc tgatttaaca aaaatttaac gcgaattaat tctgtggaat gtgtgtcagt 3420
   tagggtgtgg aaagtcccca ggctccccag caggcagaag tatgcaaagc atgcatctca 3480
   attagtcagc aaccaggtgt ggaaagtccc caggctcccc agcaggcaga agtatgcaaa 3540
   gcatgcatct caattagtca gcaaccatag tcccgcccct aactccgccc atcccgcccc 3600
   taactccgcc cagttccgcc cattctccgc cccatggctg actaattttt tttatttatg 3660
   cagaggccga ggccgcctct gcctctgagc tattccagaa gtagtgagga ggcttttttg 3720
   gaggcctagg cttttgcaaa aagctcccgg gagcttgtat atccattttc ggatctgatc 3780
   agcacgtgtt gacaattaat catcggcata gtatatcggc atagtataat acgacaaggt 3840
   gaggaactaa accatggcca agttgaccag tgccgttccg gtgctcaccg cgcgcgacgt 3900
   cgccggagcg gtcgagttct ggaccgaccg gctcgggttc tcccgggact tcgtggagga 3960
   cgacttcgcc ggtgtggtcc gggacgacgt gaccctgttc atcagcgcgg tccaggacca 4020
   ggtggtgccg gacaacaccc tggcctgggt gtgggtgcgc ggcctggacg agctgtacgc 4080
   cgagtggtcg gaggtcgtgt ccacgaactt ccgggacgcc tccgggccgg ccatgaccga 4140
   gatcggcgag cagccgtggg ggcgggagtt cgccctgcgc gacccggccg gcaactgcgt 4200
   gcacttcgtg gccgaggagc aggactgaca cgtgctacga gatttcgatt ccaccgccgc 4260
   cttctatgaa aggttgggct tcggaatcgt tttccgggac gccggctgga tgatcctcca 4320
   gcgcggggat ctcatgctgg agttcttcgc ccaccccaac ttgtttattg cagcttataa 4380
   tggttacaaa taaagcaata gcatcacaaa tttcacaaat aaagcatttt tttcactgca 4440
   ttctagttgt ggtttgtcca aactcatcaa tgtatcttat catgtctgta taccgtcgac 4500
   ctctagctag agcttggcgt aatcatggtc atagctgttt cctgtgtgaa attgttatcc 4560
   gctcacaatt ccacacaaca tacgagccgg aagcataaag tgtaaagcct ggggtgccta 4620
   atgagtgagc taactcacat taattgcgtt gcgctcactg cccgctttcc agtcgggaaa 4680
   cctgtcgtgc cagctgcatt aatgaatcgg ccaacgcgcg gggagaggcg gtttgcgtat 4740
   tgggcgctct tccgcttcct cgctcactga ctcgctgcgc tcggtcgttc ggctgcggcg 4800
   agcggtatca gctcactcaa aggcggtaat acggttatcc acagaatcag gggataacgc 4860
   aggaaagaac atgtgagcaa aaggccagca aaaggccagg aaccgtaaaa aggccgcgtt 4920
   gctggcgttt ttccataggc tccgcccccc tgacgagcat cacaaaaatc gacgctcaag 4980
   tcagaggtgg cgaaacccga caggactata aagataccag gcgtttcccc ctggaagctc 5040
   cctcgtgcgc tctcctgttc cgaccctgcc gcttaccgga tacctgtccg cctttctccc 5100
   ttcgggaagc gtggcgcttt ctcaatgctc acgctgtagg tatctcagtt cggtgtaggt 5160
   cgttcgctcc aagctgggct gtgtgcacga accccccgtt cagcccgacc gctgcgcctt 5220
   atccggtaac tatcgtcttg agtccaaccc ggtaagacac gacttatcgc cactggcagc 5280
   agccactggt aacaggatta gcagagcgag gtatgtaggc ggtgctacag agttcttgaa 5340
   gtggtggcct aactacggct acactagaag gacagtattt ggtatctgcg ctctgctgaa 5400
   gccagttacc ttcggaaaaa gagttggtag ctcttgatcc ggcaaacaaa ccaccgctgg 5460
   tagcggtggt ttttttgttt gcaagcagca gattacgcgc agaaaaaaag gatctcaaga 5520
   agatcctttg atcttttcta cggggtctga cgctcagtgg aacgaaaact cacgttaagg 5580
   gattttggtc atgagattat caaaaaggat cttcacctag atccttttaa attaaaaatg 5640
   aagttttaaa tcaatctaaa gtatatatga gtaaacttgg tctgacagtt accaatgctt 5700
   aatcagtgag gcacctatct cagcgatctg tctatttcgt tcatccatag ttgcctgact 5760
   ccccgtcgtg tagataacta cgatacggga gggcttacca tctggcccca gtgctgcaat 5820
   gataccgcga gacccacgct caccggctcc agatttatca gcaataaacc agccagccgg 5880
   aagggccgag cgcagaagtg gtcctgcaac tttatccgcc tccatccagt ctattaattg 5940
   ttgccgggaa gctagagtaa gtagttcgcc agttaatagt ttgcgcaacg ttgttgccat 6000
   tgctacaggc atcgtggtgt cacgctcgtc gtttggtatg gcttcattca gctccggttc 6060
   ccaacgatca aggcgagtta catgatcccc catgttgtgc aaaaaagcgg ttagctcctt 6120
   cggtcctccg atcgttgtca gaagtaagtt ggccgcagtg ttatcactca tggttatggc 6180
   agcactgcat aattctctta ctgtcatgcc atccgtaaga tgcttttctg tgactggtga 6240
   gtactcaacc aagtcattct gagaatagtg tatgcggcga ccgagttgct cttgcccggc 6300
   gtcaatacgg gataataccg cgccacatag cagaacttta aaagtgctca tcattggaaa 6360
   acgttcttcg gggcgaaaac tctcaaggat cttaccgctg ttgagatcca gttcgatgta 6420
   acccactcgt gcacccaact gatcttcagc atcttttact ttcaccagcg tttctgggtg 6480
   agcaaaaaca ggaaggcaaa atgccgcaaa aaagggaata agggcgacac ggaaatgttg 6540
   aatactcata ctcttccttt ttcaatatta ttgaagcatt tatcagggtt attgtctcat 6600
   gagcggatac atatttgaat gtatttagaa aaataaacaa ataggggttc cgcgcacatt   6660
   tccccgaaaa gtgccacctg acgtcgacgg atcgggagat cgatctcccg atcccctatg   6720
   gtcgactctc agtacaatct gctctgatgc cgcatagtta agccagtatc tgctccctgc   6780
   ttgtgtgttg gaggtcgctg agtagtgcgc gagcaaaatt taagctacaa caaggcaagg   6840
   cttgaccgac                                                          6850
<210> 8
   <211> 13612
   <212> DNA
   <213> Artificial Sequence
<220>
   <223> Plasmid pcDNA3.1(V5-His)hp75 Nr.16
<220>
   <221> CDS
   <222> (40)..(1320)
   <223> human p75 sequence
<400> 8
   gatccactag tccagtgtgg tggaattgcc cttgccacc atg ggg gca ggt gcc       54
                                              Met Gly Ala Gly Ala
                                              1               5
   acc ggc cgc gcc atg gac ggg ccg cgc ctg ctg ctg ttg ctg ctt ctg      102
   Thr Gly Arg Ala Met Asp Gly Pro Arg Leu Leu Leu Leu Leu Leu Leu
                   10                  15                  20
   ggg gtg tcc ctt gga ggt gcc aag gag gca tgc ccc aca ggc ctg tac      150
   Gly Val Ser Leu Gly Gly Ala Lys Glu Ala Cys Pro Thr Gly Leu Tyr
               25                  30                  35
   acg cac agc ggt gag tgc tgc aaa gcc tgc aac ctg ggc gag ggt gtg      198
   Thr His Ser Gly Glu Cys Cys Lys Ala Cys Asn Leu Gly Glu Gly Val
               40                  45                  50
   gcc cag cct tgt gga gcc aac cag acc gtg tgt gag ccc tgc ctg gac      246
   Ala Gln Pro Cys Gly Ala Asn Gln Thr Val Cys Glu Pro Cys Leu Asp
       55                  60                  65
   agc gtg acg ttc tcc gac gtg gtg agc gcg acc gag ccg tgc aag ccg      294
   Ser Val Thr Phe Ser Asp Val Val Ser Ala Thr Glu Pro Cys Lys Pro
   70                  75                  80                  85
   tgc acc gag tgc gtg ggg ctc cag agc atg tcg gcg ccg tgc gtg gag      342
   Cys Thr Glu Cys Val Gly Leu Gln Ser Met Ser Ala Pro Cys Val Glu
                   90                  95                  100
   gcc gac gac gcc gtg tgc cgc tgc gcc tac ggc tac tac cag gat gag      390
   Ala Asp Asp Ala Val Cys Arg Cys Ala Tyr Gly Tyr Tyr Gln Asp Glu
               105                 110                 115
   acg act ggg cgc tgc gag gcg tgc cgc gtg tgc gag gcg ggc tcg ggc      438
   Thr Thr Gly Arg Cys Glu Ala Cys Arg Val Cys Glu Ala Gly Ser Gly
           120                 125                 130
   ctc gtg ttc tcc tgc cag gac aag cag aac acc gtg tgc gag gag tgc      486
   Leu Val Phe Ser Cys Gln Asp Lys Gln Asn Thr Val Cys Glu Glu Cys
       135                 140                 145
   ccc gac ggc acg tat tcc gac gag gcc aac cac gtg gac ccg tgc ctg      534
   Pro Asp Gly Thr Tyr Ser Asp Glu Ala Asn His Val Asp Pro Cys Leu
   150                 155                 160                 165
   ccc tgc acc gtg tgc gag gac acc gag cgc cag ctc cgc gag tgc aca      582
   Pro Cys Thr Val Cys Glu Asp Thr Glu Arg Gln Leu Arg Glu Cys Thr
                   170                 175                 180
   cgc tgg gcc gac gcc gag tgc gag gag atc cct ggc cgt tgg att aca      630
   Arg Trp Ala Asp Ala Glu Cys Glu Glu Ile Pro Gly Arg Trp Ile Thr
               185                 190                 195
   cgg tcc aca ccc cca gag ggc tcg gac agc aca gcc ccc agc acc cag      678
   Arg Ser Thr Pro Pro Glu Gly Ser Asp Ser Thr Ala Pro Ser Thr Gln
           200                 205                 210
   gag cct gag gca cct cca gaa caa gac ctc ata gcc agc acg gtg gca      726
   Glu Pro Glu Ala Pro Pro Glu Gln Asp Leu Ile Ala Ser Thr Val Ala
       215                 220                 225
   ggt gtg gtg acc aca gtg atg ggc agc tcc cag ccc gtg gtg acc cga      774
   Gly Val Val Thr Thr Val Met Gly Ser Ser Gln Pro Val Val Thr Arg
   230                 235                 240                 245
   ggc acc acc gac aac ctc atc cct gtc tat tgc tcc atc ctg gct gct      822
   Gly Thr Thr Asp Asn Leu Ile Pro Val Tyr Cys Ser Ile Leu Ala Ala
                   250                 255                 260
   gtg gtt gtg ggt ctt gtg gcc tac ata gcc ttc aag agg tgg aac agc      870
   Val Val Val Gly Leu Val Ala Tyr Ile Ala Phe Lys Arg Trp Asn Ser
               265                 270                 275
   tgc aag cag aac aag caa gga gcc aac agc cgg cca gtg aac cag acg      918
   Cys Lys Gln Asn Lys Gln Gly Ala Asn Ser Arg Pro Val Asn Gln Thr
           280                 285                 290
   ccc cca cca gag gga gaa aaa ctc cac agc gac agt ggc atc tcc gtg      966
   Pro Pro Pro Glu Gly Glu Lys Leu His Ser Asp Ser Gly Ile Ser Val
       295                 300                 305
   gac agc cag agc ctg cat gac cag cag ccc cac acg cag aca gcc tcg     1014
   Asp Ser Gln Ser Leu His Asp Gln Gln Pro His Thr Gln Thr Ala Ser
   310                 315                 320                 325
   ggc cag gcc ctc aag ggt gac gga ggc ctc tac agc agc ctg ccc cca     1062
   Gly Gln Ala Leu Lys Gly Asp Gly Gly Leu Tyr Ser Ser Leu Pro Pro
                   330                 335                 340
   gcc aag cgg gag gag gtg gag aag ctt ctc aac ggc tct gcg ggg gac     1110
   Ala Lys Arg Glu Glu Val Glu Lys Leu Leu Asn Gly Ser Ala Gly Asp
               345                 350                 355
   acc tgg cgg cac ctg gcg ggc gag ctg ggc tac cag ccc gag cac ata     1158
   Thr Trp Arg His Leu Ala Gly Glu Leu Gly Tyr Gln Pro Glu His Ile
           360                 365                 370
   gac tcc ttt acc cat gag gcc tgc ccc gtt cgc gcc ctg ctt gca agc     1206
   Asp Ser Phe Thr His Glu Ala Cys Pro Val Arg Ala Leu Leu Ala Ser
       375                 380                 385
   tgg gcc acc cag gac agc gcc aca ctg gac gcc ctc ctg gcc gcc ctg     1254
   Trp Ala Thr Gln Asp Ser Ala Thr Leu Asp Ala Leu Leu Ala Ala Leu
   390                 395                 400                 405
   cgc cgc atc cag cga gcc gac ctc gtg gag agt ctg tgc agt gag tcc     1302
   Arg Arg Ile Gln Arg Ala Asp Leu Val Glu Ser Leu Cys Ser Glu Ser
                   410                 415                 420
   act gcc aca tcc cca gtg tgaaagggca attctgcaga tatccagcac             1350
   Thr Ala Thr Ser Pro Val
               425
   agtggcggcc gctcgagtct agagggcccg cggttcgaag gtaagcctat ccctaaccct   1410
   ctcctcggtc tcgattctac gcgtaccggt catcatcacc atcaccattg agtttaaacc 1470
   cgctgatcag cctcgactgt gccttctagt tgccagccat ctgttgtttg cccctccccc 1530
   gtgccttcct tgaccctgga aggtgccact cccactgtcc tttcctaata aaatgaggaa 1590
   attgcatcgc attgtctgag taggtgtcat tctattctgg ggggtggggt ggggcaggac 1650
   agcaaggggg aggattggga agacaatagc aggcatgctg gggatgcggt gggctctatg 1710
   gcttctgagg cggaaagaac cagctggggc tctagggggt atccccacgc gccctgtagc 1770
   ggcgcattaa gcgcggcggg tgtggtggtt acgcgcagcg tgaccgctac acttgccagc 1830
   gccctagcgc ccgctccttt cgctttcttc ccttcctttc tcgccacgtt cgccggcttt 1890
   ccccgtcaag ctctaaatcg gggcatccct ttagggttcc gatttagtgc tttacggcac 1950
   ctcgacccca aaaaacttga ttagggtgat ggttcacgta gtgggccatc gccctgatag 2010
   acggtttttc gccctttgac gttggagtcc acgttcttta atagtggact cttgttccaa 2070
   actggaacaa cactcaaccc tatctcggtc tattcttttg atttataagg gattttgggg 2130
   atttcggcct attggttaaa aaatgagctg atttaacaaa aatttaacgc gaattaattc 2190
   tgtggaatgt gtgtcagtta gggtgtggaa agtccccagg ctccccaggc aggcagaagt 2250
   atgcaaagca tgcatctcaa ttagtcagca accaggtgtg gaaagtcccc aggctcccca 2310
   gcaggcagaa gtatgcaaag catgcatctc aattagtcag caaccatagt cccgccccta 2370
   actccgccca tcccgcccct aactccgccc agttccgccc attctccgcc ccatggctga 2430
   ctaatttttt ttatttatgc agaggccgag gccgcctctg cctctgagct attccagaag 2490
   tagtgaggag gcttttttgg aggcctaggc ttttgcaaaa agctcccggg agcttgtata 2550
   tccattttcg gatctgatca agagacagga tgaggatcgt ttcgcatgat tgaacaagat 2610
   ggattgcacg caggttctcc ggccgcttgg gtggagaggc tattcggcta tgactgggca 2670
   caacagacaa tcggctgctc tgatgccgcc gtgttccggc tgtcagcgca ggggcgcccg 2730
   gttctttttg tcaagaccga cctgtccggt gccctgaatg aactgcagga cgaggcagcg 2790
   cggctatcgt ggctggccac gacgggcgtt ccttgcgcag ctgtgctcga cgttgtcact 2850
   gaagcgggaa gggactggct gctattgggc gaagtgccgg ggcaggatct cctgtcatct 2910
   caccttgctc ctgccgagaa agtatccatc atggctgatg caatgcggcg gctgcatacg 2970
   cttgatccgg ctacctgccc attcgaccac caagcgaaac atcgcatcga gcgagcacgt 3030
   actcggatgg aagccggtct tgtcgatcag gatgatctgg acgaagagca tcaggggctc 3090
   gcgccagccg aactgttcgc caggctcaag gcgcgcatgc ccgacggcga ggatctcgtc 3150
   gtgacccatg gcgatgcctg cttgccgaat atcatggtgg aaaatggccg cttttctgga 3210
   ttcatcgact gtggccggct gggtgtggcg gaccgctatc aggacatagc gttggctacc 3270
   cgtgatattg ctgaagagct tggcggcgaa tgggctgacc gcttcctcgt gctttacggt 3330
   atcgccgctc ccgattcgca gcgcatcgcc ttctatcgcc ttcttgacga gttcttctga 3390
   gcgggactct ggggttcgcg aaatgaccga ccaagcgacg cccaacctgc catcacgaga 3450
   tttcgattcc accgccgcct tctatgaaag gttgggcttc ggaatcgttt tccgggacgc 3510
   cggctggatg atcctccagc gcggggatct catgctggag ttcttcgccc accccaactt 3570
   gtttattgca gcttataatg gttacaaata aagcaatagc atcacaaatt tcacaaataa 3630
   agcatttttt tcactgcatt ctagttgtgg tttgtccaaa ctcatcaatg tatcttatca 3690
   tgtctgtata ccgtcgacct ctagctagag cttggcgtaa tcatggtcat agctgtttcc 3750
   tgtgtgaaat tgttatccgc tcacaattcc acacaacata cgagccggaa gcataaagtg 3810
   taaagcctgg ggtgcctaat gagtgagcta actcacatta attgcgttgc gctcactgcc 3870
   cgctttccag tcgggaaacc tgtcgtgcca gctgcattaa tgaatcggcc aacgcgcggg 3930
   gagaggcggt ttgcgtattg ggcgctcttc cgcttcctcg ctcactgact cgctgcgctc 3990
   ggtcgttcgg ctgcggcgag cggtatcagc tcactcaaag gcggtaatac ggttatccac 4050
   agaatcaggg gataacgcag gaaagaacat gtgagcaaaa ggccagcaaa aggccaggaa 4110
   ccgtaaaaag gccgcgttgc tggcgttttt ccataggctc cgcccccctg acgagcatca 4170
   caaaaatcga cgctcaagtc agaggtggcg aaacccgaca ggactataaa gataccaggc 4230
   gtttccccct ggaagctccc tcgtgcgctc tcctgttccg accctgccgc ttaccggata 4290
   cctgtccgcc tttctccctt cgggaagcgt ggcgctttct caatgctcac gctgtaggta 4350
   tctcagttcg gtgtaggtcg ttcgctccaa gctgggctgt gtgcacgaac cccccgttca 4410
   gcccgaccgc tgcgccttat ccggtaacta tcgtcttgag tccaacccgg taagacacga 4470
   cttatcgcca ctggcagcag ccactggtaa caggattagc agagcgaggt atgtaggcgg 4530
   tgctacagag ttcttgaagt ggtggcctaa ctacggctac actagaagga cagtatttgg 4590
   tatctgcgct ctgctgaagc cagttacctt cggaaaaaga gttggtagct cttgatccgg 4650
   caaacaaacc accgctggta gcggtggttt ttttgtttgc aagcagcaga ttacgcgcag 4710
   aaaaaaagga tctcaagaag atcctttgat cttttctacg gggtctgacg ctcagtggaa 4770
   cgaaaactca cgttaaggga ttttggtcat gagattatca aaaaggatct tcacctagat 4830
   ccttttaaat taaaaatgaa gttttaaatc aatctaaagt atatatgagt aaacttggtc 4890
   tgacagttac caatgcttaa tcagtgaggc acctatctca gcgatctgtc tatttcgttc 4950
   atccatagtt gcctgactcc ccgtcgtgta gataactacg atacgggagg gcttaccatc 5010
   tggccccagt gctgcaatga taccgcgaga cccacgctca ccggctccag atttatcagc 5070
   aataaaccag ccagccggaa gggccgagcg cagaagtggt cctgcaactt tatccgcctc 5130
   catccagtct attaattgtt gccgggaagc tagagtaagt agttcgccag ttaatagttt 5190
   gcgcaacgtt gttgccattg ctacaggcat cgtggtgtca cgctcgtcgt ttggtatggc 5250
   ttcattcagc tccggttccc aacgatcaag gcgagttaca tgatccccca tgttgtgcaa 5310
   aaaagcggtt agctccttcg gtcctccgat cgttgtcaga agtaagttgg ccgcagtgtt 5370
   atcactcatg gttatggcag cactgcataa ttctcttact gtcatgccat ccgtaagatg 5430
   cttttctgtg actggtgagt actcaaccaa gtcattctga gaatagtgta tgcggcgacc 5490
   gagttgctct tgcccggcgt caatacggga taataccgcg ccacatagca gaactttaaa 5550
   agtgctcatc attggaaaac gttcttcggg gcgaaaactc tcaaggatct taccgctgtt 5610
   gagatccagt tcgatgtaac ccactcgtgc acccaactga tcttcagcat cttttacttt 5670
   caccagcgtt tctgggtgag caaaaacagg aaggcaaaat gccgcaaaaa agggaataag 5730
   ggcgacacgg aaatgttgaa tactcatact cttccttttt caatattatt gaagcattta 5790
   tcagggttat tgtctcatga gcggatacat atttgaatgt atttagaaaa ataaacaaat 5850
   aggggttccg cgcacatttc cccgaaaagt gccacctgac gtccgggaga tctcccgatc 5910
   ccctatggtc gactctcagt acaatctgct ctgatgccgc atagttaagc cagtatctgc 5970
   tccctgcttg tgtgttggag gtcgctgagt agtgcgcgag caaaatttaa gctacaacaa 6030
   ggcaaggctt gaccgacaat tgcatgaaga atctgcttag ggttaggcgt tttgcgctgc 6090
   ttcgcgatgt acgggccaga tatacgcgtt gacattgatt attgactagt tattaatagt 6150
   aatcaattac ggggtcatta gttcatagcc catatatgga gttccgcgtt acataactta 6210
   cggtaaatgg cccgcctggc tgaccgccca acgacccccg cccattgacg tcaataatga 6270
   cgtatgttcc catagtaacg ccaataggga ctttccattg acgtcaatgg gtggactatt 6330
   tacggtaaac tgcccacttg gcagtacatc aagtgtatca tatgccaagt acgcccccta 6390
   ttgacgtcaa tgacggtaaa tggcccgcct ggcattatgc ccagtacatg accttatggg 6450
   actttcctac ttggcagtac atctacgtat tagtcatcgc tattaccatg gtgatgcggt 6510
   tttggcagta catcaatggg cgtggatagc ggtttgactc acggggattt ccaagtctcc 6570
   accccattga cgtcaatggg agtttgtttt ggcaccaaaa tcaacgggac tttccaaaat 6630
   gtcgtaacaa ctccgcccca ttgacgcaaa tgggcggtag gcgtgtacgg tgggaggtct 6690
   atataagcag agctctctgg ctaactagag aacccactgc ttactggctt atcgaaatta 6750
   atacgactca ctatagggag acccaagctg gctagttaag cttggtaccg agctcggatc 6810
   cactagtcca gtgtggtgga attgcccttg ccaccatggg ggcaggtgcc accggccgcg 6870
   ccatggacgg gccgcgcctg ctgctgttgc tgcttctggg ggtgtccctt ggaggtgcca 6930
   aggaggcatg ccccacaggc ctgtacacgc acagcggtga gtgctgcaaa gcctgcaacc 6990
   tgggcgaggg tgtggcccag ccttgtggag ccaaccagac cgtgtgtgag ccctgcctgg 7050
   acagcgtgac gttctccgac gtggtgagcg cgaccgagcc gtgcaagccg tgcaccgagt 7110
   gcgtggggct ccagagcatg tcggcgccgt gcgtggaggc cgacgacgcc gtgtgccgct 7170
   gcgcctacgg ctactaccag gatgagacga ctgggcgctg cgaggcgtgc cgcgtgtgcg 7230
   aggcgggctc gggcctcgtg ttctcctgcc aggacaagca gaacaccgtg tgcgaggagt 7290
   gccccgacgg cacgtattcc gacgaggcca accacgtgga cccgtgcctg ccctgcaccg 7350
   tgtgcgagga caccgagcgc cagctccgcg agtgcacacg ctgggccgac gccgagtgcg 7410
   aggagatccc tggccgttgg attacacggt ccacaccccc agagggctcg gacagcacag 7470
   cccccagcac ccaggagcct gaggcacctc cagaacaaga cctcatagcc agcacggtgg 7530
   caggtgtggt gaccacagtg atgggcagct cccagcccgt ggtgacccga ggcaccaccg 7590
   acaacctcat ccctgtctat tgctccatcc tggctgctgt ggttgtgggt cttgtggcct 7650
   acatagcctt caagaggtgg aacagctgca agcagaacaa gcaaggagcc aacagccggc 7710
   cagtgaacca gacgccccca ccagagggag aaaaactcca cagcgacagt ggcatctccg 7770
   tggacagcca gagcctgcat gaccagcagc cccacacgca gacagcctcg ggccaggccc 7830
   tcaagggtga cggaggcctc tacagcagcc tgcccccagc caagcgggag gaggtggaga 7890
   agcttctcaa cggctctgcg ggggacacct ggcggcacct ggcgggcgag ctgggctacc 7950
   agcccgagca catagactcc tttacccatg aggcctgccc cgttcgcgcc ctgcttgcaa 8010
   gctgggccac ccaggacagc gccacactgg acgccctcct ggccgccctg cgccgcatcc 8070
   agcgagccga cctcgtggag agtctgtgca gtgagtccac tgccacatcc ccagtgtgaa 8130
   agggcaattc tgcagatatc cagcacagtg gcggccgctc gagtctagag ggcccgcggt 8190
   tcgaaggtaa gcctatccct aaccctctcc tcggtctcga ttctacgcgt accggtcatc 8250
   atcaccatca ccattgagtt taaacccgct gatcagcctc gactgtgcct tctagttgcc 8310
   agccatctgt tgtttgcccc tcccccgtgc cttccttgac cctggaaggt gccactccca 8370
   ctgtcctttc ctaataaaat gaggaaattg catcgcattg tctgagtagg tgtcattcta 8430
   ttctgggggg tggggtgggg caggacagca agggggagga ttgggaagac aatagcaggc 8490
   atgctgggga tgcggtgggc tctatggctt ctgaggcgga aagaaccagc tggggctcta 8550
   gggggtatcc ccacgcgccc tgtagcggcg cattaagcgc ggcgggtgtg gtggttacgc 8610
   gcagcgtgac cgctacactt gccagcgccc tagcgcccgc tcctttcgct ttcttccctt 8670
   cctttctcgc cacgttcgcc ggctttcccc gtcaagctct aaatcggggc atccctttag 8730
   ggttccgatt tagtgcttta cggcacctcg accccaaaaa acttgattag ggtgatggtt 8790
   cacgtagtgg gccatcgccc tgatagacgg tttttcgccc tttgacgttg gagtccacgt 8850
   tctttaatag tggactcttg ttccaaactg gaacaacact caaccctatc tcggtctatt 8910
   cttttgattt ataagggatt ttggggattt cggcctattg gttaaaaaat gagctgattt 8970
   aacaaaaatt taacgcgaat taattctgtg gaatgtgtgt cagttagggt gtggaaagtc 9030
   cccaggctcc ccaggcaggc agaagtatgc aaagcatgca tctcaattag tcagcaacca 9090
   ggtgtggaaa gtccccaggc tccccagcag gcagaagtat gcaaagcatg catctcaatt 9150
   agtcagcaac catagtcccg cccctaactc cgcccatccc gcccctaact ccgcccagtt 9210
   ccgcccattc tccgccccat ggctgactaa ttttttttat ttatgcagag gccgaggccg 9270
   cctctgcctc tgagctattc cagaagtagt gaggaggctt ttttggaggc ctaggctttt 9330
   gcaaaaagct cccgggagct tgtatatcca ttttcggatc tgatcaagag acaggatgag 9390
   gatcgtttcg catgattgaa caagatggat tgcacgcagg ttctccggcc gcttgggtgg 9450
   agaggctatt cggctatgac tgggcacaac agacaatcgg ctgctctgat gccgccgtgt 9510
   tccggctgtc agcgcagggg cgcccggttc tttttgtcaa gaccgacctg tccggtgccc 9570
   tgaatgaact gcaggacgag gcagcgcggc tatcgtggct ggccacgacg ggcgttcctt 9630
   gcgcagctgt gctcgacgtt gtcactgaag cgggaaggga ctggctgcta ttgggcgaag 9690
   tgccggggca ggatctcctg tcatctcacc ttgctcctgc cgagaaagta tccatcatgg 9750
   ctgatgcaat gcggcggctg catacgcttg atccggctac ctgcccattc gaccaccaag 9810
   cgaaacatcg catcgagcga gcacgtactc ggatggaagc cggtcttgtc gatcaggatg 9870
   atctggacga agagcatcag gggctcgcgc cagccgaact gttcgccagg ctcaaggcgc 9930
   gcatgcccga cggcgaggat ctcgtcgtga cccatggcga tgcctgcttg ccgaatatca 9990
   tggtggaaaa tggccgcttt tctggattca tcgactgtgg ccggctgggt gtggcggacc 10050
   gctatcagga catagcgttg gctacccgtg atattgctga agagcttggc ggcgaatggg 10110
   ctgaccgctt cctcgtgctt tacggtatcg ccgctcccga ttcgcagcgc atcgccttct 10170
   atcgccttct tgacgagttc ttctgagcgg gactctgggg ttcgcgaaat gaccgaccaa 10230
   gcgacgccca acctgccatc acgagatttc gattccaccg ccgccttcta tgaaaggttg 10290
   ggcttcggaa tcgttttccg ggacgccggc tggatgatcc tccagcgcgg ggatctcatg 10350
   ctggagttct tcgcccaccc caacttgttt attgcagctt ataatggtta caaataaagc 10410
   aatagcatca caaatttcac aaataaagca tttttttcac tgcattctag ttgtggtttg 10470
   tccaaactca tcaatgtatc ttatcatgtc tgtataccgt cgacctctag ctagagcttg 10530
   gcgtaatcat ggtcatagct gtttcctgtg tgaaattgtt atccgctcac aattccacac 10590
   aacatacgag ccggaagcat aaagtgtaaa gcctggggtg cctaatgagt gagctaactc 10650
   acattaattg cgttgcgctc actgcccgct ttccagtcgg gaaacctgtc gtgccagctg 10710
   cattaatgaa tcggccaacg cgcggggaga ggcggtttgc gtattgggcg ctcttccgct 10770
   tcctcgctca ctgactcgct gcgctcggtc gttcggctgc ggcgagcggt atcagctcac 10830
   tcaaaggcgg taatacggtt atccacagaa tcaggggata acgcaggaaa gaacatgtga 10890
   gcaaaaggcc agcaaaaggc caggaaccgt aaaaaggccg cgttgctggc gtttttccat 10950
   aggctccgcc cccctgacga gcatcacaaa aatcgacgct caagtcagag gtggcgaaac 11010
   ccgacaggac tataaagata ccaggcgttt ccccctggaa gctccctcgt gcgctctcct 11070
   gttccgaccc tgccgcttac cggatacctg tccgcctttc tcccttcggg aagcgtggcg 11130
   ctttctcaat gctcacgctg taggtatctc agttcggtgt aggtcgttcg ctccaagctg 11190
   ggctgtgtgc acgaaccccc cgttcagccc gaccgctgcg ccttatccgg taactatcgt 11250
   cttgagtcca acccggtaag acacgactta tcgccactgg cagcagccac tggtaacagg 11310
   attagcagag cgaggtatgt aggcggtgct acagagttct tgaagtggtg gcctaactac 11370
   ggctacacta gaaggacagt atttggtatc tgcgctctgc tgaagccagt taccttcgga 11430
   aaaagagttg gtagctcttg atccggcaaa caaaccaccg ctggtagcgg tggttttttt 11490
   gtttgcaagc agcagattac gcgcagaaaa aaaggatctc aagaagatcc tttgatcttt 11550
   tctacggggt ctgacgctca gtggaacgaa aactcacgtt aagggatttt ggtcatgaga 11610
   ttatcaaaaa ggatcttcac ctagatcctt ttaaattaaa aatgaagttt taaatcaatc 11670
   taaagtatat atgagtaaac ttggtctgac agttaccaat gcttaatcag tgaggcacct 11730
   atctcagcga tctgtctatt tcgttcatcc atagttgcct gactccccgt cgtgtagata 11790
   actacgatac gggagggctt accatctggc cccagtgctg caatgatacc gcgagaccca 11850
   cgctcaccgg ctccagattt atcagcaata aaccagccag ccggaagggc cgagcgcaga 11910
   agtggtcctg caactttatc cgcctccatc cagtctatta attgttgccg ggaagctaga 11970
   gtaagtagtt cgccagttaa tagtttgcgc aacgttgttg ccattgctac aggcatcgtg 12030
   gtgtcacgct cgtcgtttgg tatggcttca ttcagctccg gttcccaacg atcaaggcga 12090
   gttacatgat cccccatgtt gtgcaaaaaa gcggttagct ccttcggtcc tccgatcgtt 12150
   gtcagaagta agttggccgc agtgttatca ctcatggtta tggcagcact gcataattct 12210
   cttactgtca tgccatccgt aagatgcttt tctgtgactg gtgagtactc aaccaagtca 12270
   ttctgagaat agtgtatgcg gcgaccgagt tgctcttgcc cggcgtcaat acgggataat 12330
   accgcgccac atagcagaac tttaaaagtg ctcatcattg gaaaacgttc ttcggggcga 12390
   aaactctcaa ggatcttacc gctgttgaga tccagttcga tgtaacccac tcgtgcaccc 12450
   aactgatctt cagcatcttt tactttcacc agcgtttctg ggtgagcaaa aacaggaagg 12510
   caaaatgccg caaaaaaggg aataagggcg acacggaaat gttgaatact catactcttc 12570
   ctttttcaat attattgaag catttatcag ggttattgtc tcatgagcgg atacatattt 12630
   gaatgtattt agaaaaataa acaaataggg gttccgcgca catttccccg aaaagtgcca 12690
   cctgacgtcc gggagatctc ccgatcccct atggtcgact ctcagtacaa tctgctctga 12750
   tgccgcatag ttaagccagt atctgctccc tgcttgtgtg ttggaggtcg ctgagtagtg 12810
   cgcgagcaaa atttaagcta caacaaggca aggcttgacc gacaattgca tgaagaatct 12870
   gcttagggtt aggcgttttg cgctgcttcg cgatgtacgg gccagatata cgcgttgaca 12930
   ttgattattg actagttatt aatagtaatc aattacgggg tcattagttc atagcccata 12990
   tatggagttc cgcgttacat aacttacggt aaatggcccg cctggctgac cgcccaacga 13050
   cccccgccca ttgacgtcaa taatgacgta tgttcccata gtaacgccaa tagggacttt 13110
   ccattgacgt caatgggtgg actatttacg gtaaactgcc cacttggcag tacatcaagt 13170
   gtatcatatg ccaagtacgc cccctattga cgtcaatgac ggtaaatggc ccgcctggca 13230
   ttatgcccag tacatgacct tatgggactt tcctacttgg cagtacatct acgtattagt 13290
   catcgctatt accatggtga tgcggttttg gcagtacatc aatgggcgtg gatagcggtt 13350
   tgactcacgg ggatttccaa gtctccaccc cattgacgtc aatgggagtt tgttttggca 13410
   ccaaaatcaa cgggactttc caaaatgtcg taacaactcc gccccattga cgcaaatggg 13470
   cggtaggcgt gtacggtggg aggtctatat aagcagagct ctctggctaa ctagagaacc 13530
   cactgcttac tggcttatcg aaattaatac gactcactat agggagaccc aagctggcta 13590
   gttaagcttg gtaccgagct cg 13612
<210> 9
   <211> 427
   <212> PRT
   <213> Artificial Sequence
<220>
   <223> Plasmid pcDNA3.1(V5-His)hp75 Nr.16
<400> 9
   Met Gly Ala Gly Ala Thr Gly Arg Ala Met Asp Gly Pro Arg Leu Leu
   1               5                   10                  15
   Leu Leu Leu Leu Leu Gly Val Ser Leu Gly Gly Ala Lys Glu Ala Cys
               20                  25                  30
   Pro Thr Gly Leu Tyr Thr His Ser Gly Glu Cys Cys Lys Ala Cys Asn
           35                  40                  45
   Leu Gly Glu Gly Val Ala Gln Pro Cys Gly Ala Asn Gln Thr Val Cys
       50                  55                  60
   Glu Pro Cys Leu Asp Ser Val Thr Phe Ser Asp Val Val Ser Ala Thr
   65                  70                  75                  80
   Glu Pro Cys Lys Pro Cys Thr Glu Cys Val Gly Leu Gln Ser Met Ser
                   85                  90                  95
   Ala Pro Cys Val Glu Ala Asp Asp Ala Val Cys Arg Cys Ala Tyr Gly
               100                 105                 110
   Tyr Tyr Gln Asp Glu Thr Thr Gly Arg Cys Glu Ala Cys Arg Val Cys
           115                 120                 125
   Glu Ala Gly Ser Gly Leu Val Phe Ser Cys Gln Asp Lys Gln Asn Thr
       130                 135                 140
   Val Cys Glu Glu Cys Pro Asp Gly Thr Tyr Ser Asp Glu Ala Asn His
   145                 150                 155                 160
   Val Asp Pro Cys Leu Pro Cys Thr Val Cys Glu Asp Thr Glu Arg Gln
                   165                 170                 175
   Leu Arg Glu Cys Thr Arg Trp Ala Asp Ala Glu Cys Glu Glu Ile Pro
               180                 185                 190
   Gly Arg Trp Ile Thr Arg Ser Thr Pro Pro Glu Gly Ser Asp Ser Thr
           195                 200                 205
   Ala Pro Ser Thr Gln Glu Pro Glu Ala Pro Pro Glu Gln Asp Leu Ile
       210                 215                 220
   Ala Ser Thr Val Ala Gly Val Val Thr Thr Val Met Gly Ser Ser Gln
   225                 230                 235                 240
   Pro Val Val Thr Arg Gly Thr Thr Asp Asn Leu Ile Pro Val Tyr Cys
                   245                 250                 255
   Ser Ile Leu Ala Ala Val Val Val Gly Leu Val Ala Tyr Ile Ala Phe
               260                 265                 270
   Lys Arg Trp Asn Ser Cys Lys Gln Asn Lys Gln Gly Ala Asn Ser Arg
           275                 280                 285
   Pro Val Asn Gln Thr Pro Pro Pro Glu Gly Glu Lys Leu His Ser Asp
       290                 295                 300
   Ser Gly Ile Ser Val Asp Ser Gln Ser Leu His Asp Gln Gln Pro His
   305 310 315 320
   Thr Gln Thr Ala Ser Gly Gln Ala Leu Lys Gly Asp Gly Gly Leu Tyr
   325 330 335
   Ser Ser Leu Pro Pro Ala Lys Arg Glu Glu Val Glu Lys Leu Leu Asn
   340 345 350
   Gly Ser Ala Gly Asp Thr Trp Arg His Leu Ala Gly Glu Leu Gly Tyr
   355 360 365
   Gln Pro Glu His Ile Asp Ser Phe Thr His Glu Ala Cys Pro Val Arg
   370 375 380
   Ala Leu Leu Ala Ser Trp Ala Thr Gln Asp Ser Ala Thr Leu Asp Ala
   385 390 395 400
   Leu Leu Ala Ala Leu Arg Arg Ile Gln Arg Ala Asp Leu Val Glu Ser
   405 410 415
   Leu Cys Ser Glu Ser Thr Ala Thr Ser Pro Val
   420 425
<210> 10
   <211> 8846
   <212> DNA
   <213> Artificial Sequence
<220>
   <223> Plasmid pIRES hNgR hp75
<220>
   <221> CDS
   <222> (1202)..(2620)
   <223> human NogoR sequence
<220>
   <221> CDS
   <222> (3294)..(4574)
   <223> human p75 sequence
<400> 10
   gtgatgctcg tcaggggggc ggagcctatg gaaaaacgcc agcaacgcgg cctttttacg     60
   gttcctggcc ttttgctggc cttttgctca catggctcga cagatcttca atattggcca    120
   ttagccatat tattcattgg ttatatagca taaatcaata ttggctattg gccattgcat    180
   acgttgtatc tatatcataa tatgtacatt tatattggct catgtccaat atgaccgcca    240
   tgttggcatt gattattgac tagttattaa tagtaatcaa ttacggggtc attagttcat    300
   agcccatata tggagttccg cgttacataa cttacggtaa atggcccgcc tggctgaccg    360
   cccaacgacc cccgcccatt gacgtcaata atgacgtatg ttcccatagt aacgccaata    420
   gggactttcc attgacgtca atgggtggag tatttacggt aaactgccca cttggcagta    480
   catcaagtgt atcatatgcc aagtccgccc cctattgacg tcaatgacgg taaatggccc    540
   gcctggcatt atgcccagta catgacctta cgggactttc ctacttggca gtacatctac    600
   gtattagtca tcgctattac catggtgatg cggttttggc agtacaccaa tgggcgtgga    660
   tagcggtttg actcacgggg atttccaagt ctccacccca ttgacgtcaa tgggagtttg    720
   ttttggcacc aaaatcaacg ggactttcca aaatgtcgta acaactgcga tcgcccgccc    780
   cgttgacgca aatgggcggt aggcgtgtac ggtgggaggt ctatataagc agagctcgtt    840
   tagtgaaccg tcagatcact agaagcttta ttgcggtagt ttatcacagt taaattgcta    900
   acgcagtcag tgcttctgac acaacagtct cgaacttaag ctgcagtgac tctcttaagg    960
   tagccttgca gaagttggtc gtgaggcact gggcaggtaa gtatcaaggt tacaagacag   1020
   gtttaaggag accaatagaa actgggcttg tcgagacaga gaagactctt gcgtttctga   1080
   taggcaccta ttggtcttac tgacatccac tttgcctttc tctccacagg tgtccactcc   1140
   cagttcaatt acagctctta aggctagagt acttaatacg actcactata ggctagagct   1200
   t atg aag agg gcg tcc gct gga ggg agc cgg ctg ctg gca tgg gtg ctg   1249
     Met Lys Arg Ala Ser Ala Gly Gly Ser Arg Leu Leu Ala Trp Val Leu
     1               5                   10                  15
   tgg ctg cag gcc tgg cag gtg gca gcc cca tgc cca ggt gcc tgc gta     1297
   Trp Leu Gln Ala Trp Gln Val Ala Ala Pro Cys Pro Gly Ala Cys Val
               20                  25                  30
   tgc tac aat gag ccc aag gtg acg aca agc tgc ccc cag cag ggc ctg     1345
   Cys Tyr Asn Glu Pro Lys Val Thr Thr Ser Cys Pro Gln Gln Gly Leu
           35                  40                  45
   cag gct gtg ccc gtg ggc atc cct gct gcc agc cag cgc atc ttc ctg     1393
   Gln Ala Val Pro Val Gly Ile Pro Ala Ala Ser Gln Arg Ile Phe Leu
       50                  55                  60
   cac ggc aac cgc atc tcg cat gtg cca gct gcc agc ttc cgt gcc tgc     1441
   His Gly Asn Arg Ile Ser His Val Pro Ala Ala Ser Phe Arg Ala Cys
   65                  70                  75                  80
   cgc aac ctc acc atc ctg tgg ctg cac tcg aat gtg ctg gcc cga att     1489
   Arg Asn Leu Thr Ile Leu Trp Leu His Ser Asn Val Leu Ala Arg Ile
                   85                  90                  95
   gat gcg gct gcc ttc act ggc ctg gcc ctc ctg gag cag ctg gac ctc     1537
   Asp Ala Ala Ala Phe Thr Gly Leu Ala Leu Leu Glu Gln Leu Asp Leu
               100                 105                 110
   agc gat aat gca cag ctc cgg tct gtg gac cct gcc aca ttc cac ggc     1585
   Ser Asp Asn Ala Gln Leu Arg Ser Val Asp Pro Ala Thr Phe His Gly
           115                 120                 125
   ctg ggc cgc cta cac acg ctg cac ctg gac cgc tgc ggc ctg cag gag     1633
   Leu Gly Arg Leu His Thr Leu His Leu Asp Arg Cys Gly Leu Gln Glu
       130                 135                 140
   ctg ggc ccg ggg ctg ttc cgc ggc ctg gct gcc ctg cag tac ctc tac     1681
   Leu Gly Pro Gly Leu Phe Arg Gly Leu Ala Ala Leu Gln Tyr Leu Tyr
   145                 150                 155                 160
   ctg cag gac aac gcg ctg cag gca ctg cct gat gac acc ttc cgc gac     1729
   Leu Gln Asp Asn Ala Leu Gln Ala Leu Pro Asp Asp Thr Phe Arg Asp
                   165                 170                 175
   ctg ggc aac ctc aca cac ctc ttc ctg cac ggc aac cgc atc tcc agc      1777
   Leu Gly Asn Leu Thr His Leu Phe Leu His Gly Asn Arg Ile Ser Ser
               180                 185                 190
   gtg ccc gag cgc gcc ttc cgt ggg ctg cac agc ctc gac cgt ctc cta      1825
   Val Pro Glu Arg Ala Phe Arg Gly Leu His Ser Leu Asp Arg Leu Leu
           195                 200                 205
   ctg cac cag aac cgc gtg gcc cat gtg cac ccg cat gcc ttc cgt gac      1873
   Leu His Gln Asn Arg Val Ala His Val His Pro His Ala Phe Arg Asp
       210                 215                 220
   ctt ggc cgc ctc atg aca ctc tat ctg ttt gcc aac aat cta tca gcg      1921
   Leu Gly Arg Leu Met Thr Leu Tyr Leu Phe Ala Asn Asn Leu Ser Ala
   225                 230                 235                 240
   ctg ccc act gag gcc ctg gcc ccc ctg cgt gcc ctg cag tac ctg agg      1969
   Leu Pro Thr Glu Ala Leu Ala Pro Leu Arg Ala Leu Gln Tyr Leu Arg
                   245                 250                 255
   ctc aac gac aac ccc tgg gtg tgt gac tgc cgg gca cgc cca ctc tgg      2017
   Leu Asn Asp Asn Pro Trp Val Cys Asp Cys Arg Ala Arg Pro Leu Trp
               260                 265                 270
   gcc tgg ctg cag aag ttc cgc ggc tcc tcc tcc gag gtg ccc tgc agc      2065
   Ala Trp Leu Gln Lys Phe Arg Gly Ser Ser Ser Glu Val Pro Cys Ser
           275                 280                 285
   ctc ccg caa cgc ctg gct ggc cgt gac ctc aaa cgc cta gct gcc aat      2113
   Leu Pro Gln Arg Leu Ala Gly Arg Asp Leu Lys Arg Leu Ala Ala Asn
       290                 295                 300
   gac ctg cag ggc tgc gct gtg gcc acc ggc cct tac cat ccc atc tgg      2161
   Asp Leu Gln Gly Cys Ala Val Ala Thr Gly Pro Tyr His Pro Ile Trp
   305                 310                 315                 320
   acc ggc agg gcc acc gat gag gag ccg ctg ggg ctt ccc aag tgc tgc      2209
   Thr Gly Arg Ala Thr Asp Glu Glu Pro Leu Gly Leu Pro Lys Cys Cys
                   325                 330                 335
   cag cca gat gcc gct gac aag gcc tca gta ctg gag cct gga aga cca      2257
   Gln Pro Asp Ala Ala Asp Lys Ala Ser Val Leu Glu Pro Gly Arg Pro
               340                 345                 350
   gct tcg gca ggc aat gcg ctg aag gga cgc gtg ccg ccc ggt gac agc      2305
   Ala Ser Ala Gly Asn Ala Leu Lys Gly Arg Val Pro Pro Gly Asp Ser
           355                 360                 365
   ccg ccg ggc aac ggc tct ggc cca cgg cac atc aat gac tca ccc ttt     2353
   Pro Pro Gly Asn Gly Ser Gly Pro Arg His Ile Asn Asp Ser Pro Phe
       370                 375                 380
   ggg act ctg cct ggc tct gct gag ccc ccg ctc act gca gtg cgg ccc     2401
   Gly Thr Leu Pro Gly Ser Ala Glu Pro Pro Leu Thr Ala Val Arg Pro
   385                 390                 395                 400
   gag ggc tcc gag cca cca ggg ttc ccc acc tcg ggc cct cgc cgg agg     2449
   Glu Gly Ser Glu Pro Pro Gly Phe Pro Thr Ser Gly Pro Arg Arg Arg
                   405                 410                 415
   cca ggc tgt tca cgc aag aac cgc acc cgc agc cac tgc cgt ctg ggc     2497
   Pro Gly Cys Ser Arg Lys Asn Arg Thr Arg Ser His Cys Arg Leu Gly
               420                 425                 430
   cag gca ggc agc ggg ggt ggc ggg act ggt gac tca gaa ggc tca ggt     2545
   Gln Ala Gly Ser Gly Gly Gly Gly Thr Gly Asp Ser Glu Gly Ser Gly
           435                 440                 445
   gcc cta ccc agc ctc acc tgc agc ctc acc ccc ctg ggc ctg gcg ctg     2593
   Ala Leu Pro Ser Leu Thr Cys Ser Leu Thr Pro Leu Gly Leu Ala Leu
       450                 455                 460
   gtg ctg tgg aca gtg ctt ggg ccc tgc tgagaattca cgcgtcgagc           2640
   Val Leu Trp Thr Val Leu Gly Pro Cys
   465                 470
   atgcatctag ggcggccaat tccgcccctc tccctccccc ccccctaacg ttactggccg   2700
   aagccgcttg gaataaggcc ggtgtgcgtt tgtctatatg tgattttcca ccatattgcc   2760
   gtcttttggc aatgtgaggg cccggaaacc tggccctgtc ttcttgacga gcattcctag   2820
   gggtctttcc cctctcgcca aaggaatgca aggtctgttg aatgtcgtga aggaagcagt   2880
   tcctctggaa gcttcttgaa gacaaacaac gtctgtagcg accctttgca ggcagcggaa   2940
   ccccccacct ggcgacaggt gcctctgcgg ccaaaagcca cgtgtataag atacacctgc   3000
   aaaggcggca caaccccagt gccacgttgt gagttggata gttgtggaaa gagtcaaatg   3060
   gctctcctca agcgtattca acaaggggct gaaggatgcc cagaaggtac cccattgtat   3120
   gggatctgat ctggggcctc ggtgcacatg ctttacatgt gtttagtcga ggttaaaaaa   3180
   acgtctaggc cccccgaacc acggggacgt ggttttcctt tgaaaaacac gatgataagc   3240
   ttgccacaac ccgggatcct ctagtccagt gtggtggaat tgcccttgcc acc atg      3296
   Met
   ggg gca ggt gcc acc ggc cgc gcc atg gac ggg ccg cgc ctg ctg ctg      3344
   Gly Ala Gly Ala Thr Gly Arg Ala Met Asp Gly Pro Arg Leu Leu Leu
   475                 480                 485                 490
   ttg ctg ctt ctg ggg gtg tcc ctt gga ggt gcc aag gag gca tgc ccc      3392
   Leu Leu Leu Leu Gly Val Ser Leu Gly Gly Ala Lys Glu Ala Cys Pro
                   495                 500                 505
   aca ggc ctg tac acg cac agc ggt gag tgc tgc aaa gcc tgc aac ctg      3440
   Thr Gly Leu Tyr Thr His Ser Gly Glu Cys Cys Lys Ala Cys Asn Leu
               510                 515                 520
   ggc gag ggt gtg gcc cag cct tgt gga gcc aac cag acc gtg tgt gag      3488
   Gly Glu Gly Val Ala Gln Pro Cys Gly Ala Asn Gln Thr Val Cys Glu
           525                 530                 535
   ccc tgc ctg gac agc gtg acg ttc tcc gac gtg gtg agc gcg acc gag      3536
   Pro Cys Leu Asp Ser Val Thr Phe Ser Asp Val Val Ser Ala Thr Glu
       540                 545                 550
   ccg tgc aag ccg tgc acc gag tgc gtg ggg ctc cag agc atg tcg gcg      3584
   Pro Cys Lys Pro Cys Thr Glu Cys Val Gly Leu Gln Ser Met Ser Ala
   555                 560                 565                 570
   ccg tgc gtg gag gcc gac gac gcc gtg tgc cgc tgc gcc tac ggc tac      3632
   Pro Cys Val Glu Ala Asp Asp Ala Val Cys Arg Cys Ala Tyr Gly Tyr
                   575                 580                 585
   tac cag gat gag acg act ggg cgc tgc gag gcg tgc cgc gtg tgc gag      3680
   Tyr Gln Asp Glu Thr Thr Gly Arg Cys Glu Ala Cys Arg Val Cys Glu
               590                 595                 600
   gcg ggc tcg ggc ctc gtg ttc tcc tgc cag gac aag cag aac acc gtg      3728
   Ala Gly Ser Gly Leu Val Phe Ser Cys Gln Asp Lys Gln Asn Thr Val
           605                 610                 615
   tgc gag gag tgc ccc gac ggc acg tat tcc gac gag gcc aac cac gtg      3776
   Cys Glu Glu Cys Pro Asp Gly Thr Tyr Ser Asp Glu Ala Asn His Val
       620                 625                 630
   gac ccg tgc ctg ccc tgc acc gtg tgc gag gac acc gag cgc cag ctc      3824
   Asp Pro Cys Leu Pro Cys Thr Val Cys Glu Asp Thr Glu Arg Gln Leu
   635                 640                 645                 650
   cgc gag tgc aca cgc tgg gcc gac gcc gag tgc gag gag atc cct ggc      3872
   Arg Glu Cys Thr Arg Trp Ala Asp Ala Glu Cys Glu Glu Ile Pro Gly
                   655                 660                 665
   cgt tgg att aca cgg tcc aca ccc cca gag ggc tcg gac agc aca gcc      3920
   Arg Trp Ile Thr Arg Ser Thr Pro Pro Glu Gly Ser Asp Ser Thr Ala
               670                 675                 680
   ccc agc acc cag gag cct gag gca cct cca gaa caa gac ctc ata gcc      3968
   Pro Ser Thr Gln Glu Pro Glu Ala Pro Pro Glu Gln Asp Leu Ile Ala
           685                 690                 695
   agc acg gtg gca ggt gtg gtg acc aca gtg atg ggc agc tcc cag ccc      4016
   Ser Thr Val Ala Gly Val Val Thr Thr Val Met Gly Ser Ser Gln Pro
       700                 705                 710
   gtg gtg acc cga ggc acc acc gac aac ctc atc cct gtc tat tgc tcc      4064
   Val Val Thr Arg Gly Thr Thr Asp Asn Leu Ile Pro Val Tyr Cys Ser
   715                 720                 725                 730
   atc ctg gct gct gtg gtt gtg ggt ctt gtg gcc tac ata gcc ttc aag      4112
   Ile Leu Ala Ala Val Val Val Gly Leu Val Ala Tyr Ile Ala Phe Lys
                   735                 740                 745
   agg tgg aac agc tgc aag cag aac aag caa gga gcc aac agc cgg cca      4160
   Arg Trp Asn Ser Cys Lys Gln Asn Lys Gln Gly Ala Asn Ser Arg Pro
               750                 755                 760
   gtg aac cag acg ccc cca cca gag gga gaa aaa ctc cac agc gac agt      4208
   Val Asn Gln Thr Pro Pro Pro Glu Gly Glu Lys Leu His Ser Asp Ser
           765                 770                 775
   ggc atc tcc gtg gac agc cag agc ctg cat gac cag cag ccc cac acg      4256
   Gly Ile Ser Val Asp Ser Gln Ser Leu His Asp Gln Gln Pro His Thr
       780                 785                 790
   cag aca gcc tcg ggc cag gcc ctc aag ggt gac gga ggc ctc tac agc      4304
   Gln Thr Ala Ser Gly Gln Ala Leu Lys Gly Asp Gly Gly Leu Tyr Ser
   795                 800                 805                 810
   agc ctg ccc cca gcc aag cgg gag gag gtg gag aag ctt ctc aac ggc      4352
   Ser Leu Pro Pro Ala Lys Arg Glu Glu Val Glu Lys Leu Leu Asn Gly
                   815                 820                 825
   tct gcg ggg gac acc tgg cgg cac ctg gcg ggc gag ctg ggc tac cag      4400
   Ser Ala Gly Asp Thr Trp Arg His Leu Ala Gly Glu Leu Gly Tyr Gln
               830                 835                 840
   ccc gag cac ata gac tcc ttt acc cat gag gcc tgc ccc gtt cgc gcc      4448
   Pro Glu His Ile Asp Ser Phe Thr His Glu Ala Cys Pro Val Arg Ala
           845                 850                 855
   ctg ctt gca agc tgg gcc acc cag gac agc gcc aca ctg gac gcc ctc      4496
   Leu Leu Ala Ser Trp Ala Thr Gln Asp Ser Ala Thr Leu Asp Ala Leu
       860                 865                 870
   ctg gcc gcc ctg cgc cgc atc cag cga gcc gac ctc gtg gag agt ctg    4544
   Leu Ala Ala Leu Arg Arg Ile Gln Arg Ala Asp Leu Val Glu Ser Leu
   875                 880                 885                 890
   tgc agt gag tcc act gcc aca tcc cca gtg tgaaagggca attctgcaga    4594
   Cys Ser Glu Ser Thr Ala Thr Ser Pro Val
                   895                 900
   tatccagcac agtggcggcc gcttcccttt agtgagggtt aatgcttcga gcagacatga    4654
   taagatacat tgatgagttt ggacaaacca caactagaat gcagtgaaaa aaatgcttta    4714
   tttgtgaaat ttgtgatgct attgctttat ttgtaaccat tataagctgc aataaacaag    4774
   ttaacaacaa caattgcatt cattttatgt ttcaggttca gggggagatg tgggaggttt    4834
   tttaaagcaa gtaaaacctc tacaaatgtg gtaaaatccg ataaggatcg atccgggctg    4894
   gcgtaatagc gaagaggccc gcaccgatcg cccttcccaa cagttgcgca gcctgaatgg    4954
   cgaatggacg cgccctgtag cggcgcatta agcgcggcgg gtgtggtggt tacgcgcagc    5014
   gtgaccgcta cacttgccag cgccctagcg cccgctcctt tcgctttctt cccttccttt    5074
   ctcgccacgt tcgccggctt tccccgtcaa gctctaaatc gggggctccc tttagggttc    5134
   cgatttagag ctttacggca cctcgaccgc aaaaaacttg atttgggtga tggttcacgt    5194
   agtgggccat cgccctgata gacggttttt cgccctttga cgttggagtc cacgttcttt    5254
   aatagtggac tcttgttcca aactggaaca acactcaacc ctatctcggt ctattctttt    5314
   gatttataag ggattttgcc gatttcggcc tattggttaa aaaatgagct gatttaacaa    5374
   atatttaacg cgaattttaa caaaatatta acgtttacaa tttcgcctga tgcggtattt    5434
   tctccttacg catctgtgcg gtatttcaca ccgcatacgc ggatctgcgc agcaccatgg    5494
   cctgaaataa cctctgaaag aggaacttgg ttaggtacct tctgaggcgg aaagaaccag    5554
   ctgtggaatg tgtgtcagtt agggtgtgga aagtccccag gctccccagc aggcagaagt    5614
   atgcaaagca tgcatctcaa ttagtcagca accaggtgtg gaaagtcccc aggctcccca    5674
   gcaggcagaa gtatgcaaag catgcatctc aattagtcag caaccatagt cccgccccta    5734
   actccgccca tcccgcccct aactccgccc agttccgccc attctccgcc ccatggctga    5794
   ctaatttttt ttatttatgc agaggccgag gccgcctcgg cctctgagct attccagaag 5854
   tagtgaggag gcttttttgg aggcctaggc ttttgcaaaa agcttgattc ttctgacaca 5914
   acagtctcga acttaaggct agagccacca tgattgaaca agatggattg cacgcaggtt 5974
   ctccggccgc ttgggtggag aggctattcg gctatgactg ggcacaacag acaatcggct 6034
   gctctgatgc cgccgtgttc cggctgtcag cgcaggggcg cccggttctt tttgtcaaga 6094
   ccgacctgtc cggtgccctg aatgaactgc aggacgaggc agcgcggcta tcgtggctgg 6154
   ccacgacggg cgttccttgc gcagctgtgc tcgacgttgt cactgaagcg ggaagggact 6214
   ggctgctatt gggcgaagtg ccggggcagg atctcctgtc atctcacctt gctcctgccg 6274
   agaaagtatc catcatggct gatgcaatgc ggcggctgca tacgcttgat ccggctacct 6334
   gcccattcga ccaccaagcg aaacatcgca tcgagcgagc acgtactcgg atggaagccg 6394
   gtcttgtcga tcaggatgat ctggacgaag agcatcaggg gctcgcgcca gccgaactgt 6454
   tcgccaggct caaggcgcgc atgcccgacg gcgaggatct cgtcgtgacc catggcgatg 6514
   cctgcttgcc gaatatcatg gtggaaaatg gccgcttttc tggattcatc gactgtggcc 6574
   ggctgggtgt ggcggaccgc tatcaggaca tagcgttggc tacccgtgat attgctgaag 6634
   agcttggcgg cgaatgggct gaccgcttcc tcgtgcttta cggtatcgcc gctcccgatt 6694
   cgcagcgcat cgccttctat cgccttcttg acgagttctt ctgagcggga ctctggggtt 6754
   cgaaatgacc gaccaagcga cgcccaacct gccatcacga tggccgcaat aaaatatctt 6814
   tattttcatt acatctgtgt gttggttttt tgtgtgaatc gatagcgata aggatccgcg 6874
   tatggtgcac tctcagtaca atctgctctg atgccgcata gttaagccag ccccgacacc 6934
   cgccaacacc cgctgacgcg ccctgacggg cttgtctgct cccggcatcc gcttacagac 6994
   aagctgtgac cgtctccggg agctgcatgt gtcagaggtt ttcaccgtca tcaccgaaac 7054
   gcgcgagacg aaagggcctc gtgatacgcc tatttttata ggttaatgtc atgataataa 7114
   tggtttctta gacgtcaggt ggcacttttc ggggaaatgt gcgcggaacc cctatttgtt 7174
   tatttttcta aatacattca aatatgtatc cgctcatgag acaataaccc tgataaatgc 7234
   ttcaataata ttgaaaaagg aagagtatga gtattcaaca tttccgtgtc gcccttattc 7294
   ccttttttgc ggcattttgc cttcctgttt ttgctcaccc agaaacgctg gtgaaagtaa 7354
   aagatgctga agatcagttg ggtgcacgag tgggttacat cgaactggat ctcaacagcg 7414
   gtaagatcct tgagagtttt cgccccgaag aacgttttcc aatgatgagc acttttaaag 7474
   ttctgctatg tggcgcggta ttatcccgta ttgacgccgg gcaagagcaa ctcggtcgcc 7534
   gcatacacta ttctcagaat gacttggttg agtactcacc agtcacagaa aagcatctta 7594
   cggatggcat gacagtaaga gaattatgca gtgctgccat aaccatgagt gataacactg 7654
   cggccaactt acttctgaca acgatcggag gaccgaagga gctaaccgct tttttgcaca 7714
   acatggggga tcatgtaact cgccttgatc gttgggaacc ggagctgaat gaagccatac 7774
   caaacgacga gcgtgacacc acgatgcctg tagcaatggc aacaacgttg cgcaaactat 7834
   taactggcga actacttact ctagcttccc ggcaacaatt aatagactgg atggaggcgg 7894
   ataaagttgc aggaccactt ctgcgctcgg cccttccggc tggctggttt attgctgata 7954
   aatctggagc cggtgagcgt gggtctcgcg gtatcattgc agcactgggg ccagatggta 8014
   agccctcccg tatcgtagtt atctacacga cggggagtca ggcaactatg gatgaacgaa 8074
   atagacagat cgctgagata ggtgcctcac tgattaagca ttggtaactg tcagaccaag 8134
   tttactcata tatactttag attgatttaa aacttcattt ttaatttaaa aggatctagg 8194
   tgaagatcct ttttgataat ctcatgacca aaatccctta acgtgagttt tcgttccact 8254
   gagcgtcaga ccccgtagaa aagatcaaag gatcttcttg agatcctttt tttctgcgcg 8314
   taatctgctg cttgcaaaca aaaaaaccac cgctaccagc ggtggtttgt ttgccggatc 8374
   aagagctacc aactcttttt ccgaaggtaa ctggcttcag cagagcgcag ataccaaata 8434
   ctgtccttct agtgtagccg tagttaggcc accacttcaa gaactctgta gcaccgccta 8494
   catacctcgc tctgctaatc ctgttaccag tggctgctgc cagtggcgat aagtcgtgtc 8554
   ttaccgggtt ggactcaaga cgatagttac cggataaggc gcagcggtcg ggctgaacgg 8614
   ggggttcgtg cacacagccc agcttggagc gaacgaccta caccgaactg agatacctac 8674
   agcgtgagct atgagaaagc gccacgcttc ccgaagggag aaaggcggac aggtatccgg 8734
   taagcggcag ggtcggaaca ggagagcgca cgagggagct tccaggggga aacgcctggt 8794
   atctttatag tcctgtcggg tttcgccacc tctgacttga gcgtcgattt tt 8846
<210> 11
   <211> 473
   <212> PRT
   <213> Artificial Sequence
<220>
   <223> Plasmid pIRES hNgR hp75
<400> 11
   Met Lys Arg Ala Ser Ala Gly Gly Ser Arg Leu Leu Ala Trp Val Leu
   1 5 10 15
   Trp Leu Gln Ala Trp Gln Val Ala Ala Pro Cys Pro Gly Ala Cys Val
   20 25 30
   Cys Tyr Asn Glu Pro Lys Val Thr Thr Ser Cys Pro Gln Gln Gly Leu
   35 40 45
   Gln Ala Val Pro Val Gly Ile Pro Ala Ala Ser Gln Arg Ile Phe Leu
   50 55 60
   His Gly Asn Arg Ile Ser His Val Pro Ala Ala Ser Phe Arg Ala Cys
   65 70 75 80
   Arg Asn Leu Thr Ile Leu Trp Leu His Ser Asn Val Leu Ala Arg Ile
   85 90 95
   Asp Ala Ala Ala Phe Thr Gly Leu Ala Leu Leu Glu Gln Leu Asp Leu
   100 105 110
   Ser Asp Asn Ala Gln Leu Arg Ser Val Asp Pro Ala Thr Phe His Gly
   115 120 125
   Leu Gly Arg Leu His Thr Leu His Leu Asp Arg Cys Gly Leu Gln Glu
   130 135 140
   Leu Gly Pro Gly Leu Phe Arg Gly Leu Ala Ala Leu Gln Tyr Leu Tyr
   145 150 155 160
   Leu Gln Asp Asn Ala Leu Gln Ala Leu Pro Asp Asp Thr Phe Arg Asp
   165 170 175
   Leu Gly Asn Leu Thr His Leu Phe Leu His Gly Asn Arg Ile Ser Ser
   180 185 190
   Val Pro Glu Arg Ala Phe Arg Gly Leu His Ser Leu Asp Arg Leu Leu
   195 200 205
   Leu His Gln Asn Arg Val Ala His Val His Pro His Ala Phe Arg Asp
   210 215 220
   Leu Gly Arg Leu Met Thr Leu Tyr Leu Phe Ala Asn Asn Leu Ser Ala
   225 230 235 240
   Leu Pro Thr Glu Ala Leu Ala Pro Leu Arg Ala Leu Gln Tyr Leu Arg
   245 250 255
   Leu Asn Asp Asn Pro Trp Val Cys Asp Cys Arg Ala Arg Pro Leu Trp
   260 265 270
   Ala Trp Leu Gln Lys Phe Arg Gly Ser Ser Ser Glu Val Pro Cys Ser
   275 280 285
   Leu Pro Gln Arg Leu Ala Gly Arg Asp Leu Lys Arg Leu Ala Ala Asn
   290 295 300
   Asp Leu Gln Gly Cys Ala Val Ala Thr Gly Pro Tyr His Pro Ile Trp
   305 310 315 320
   Thr Gly Arg Ala Thr Asp Glu Glu Pro Leu Gly Leu Pro Lys Cys Cys
   325 330 335
   Gln Pro Asp Ala Ala Asp Lys Ala Ser Val Leu Glu Pro Gly Arg Pro
   340 345 350
   Ala Ser Ala Gly Asn Ala Leu Lys Gly Arg Val Pro Pro Gly Asp Ser
   355 360 365
   Pro Pro Gly Asn Gly Ser Gly Pro Arg His Ile Asn Asp Ser Pro Phe
   370 375 380
   Gly Thr Leu Pro Gly Ser Ala Glu Pro Pro Leu Thr Ala Val Arg Pro
   385 390 395 400
   Glu Gly Ser Glu Pro Pro Gly Phe Pro Thr Ser Gly Pro Arg Arg Arg
   405 410 415
   Pro Gly Cys Ser Arg Lys Asn Arg Thr Arg Ser His Cys Arg Leu Gly
   420 425 430
   Gln Ala Gly Ser Gly Gly Gly Gly Thr Gly Asp Ser Glu Gly Ser Gly
   435 440 445
   Ala Leu Pro Ser Leu Thr Cys Ser Leu Thr Pro Leu Gly Leu Ala Leu
   450 455 460
   Val Leu Trp Thr Val Leu Gly Pro Cys
   465 470
<210> 12
   <211> 427
   <212> PRT
   <213> Artificial Sequence
<220>
   <223> Plasmid pIRES hNgR hp75
<400> 12
   Met Gly Ala Gly Ala Thr Gly Arg Ala Met Asp Gly Pro Arg Leu Leu
   1               5                   10                  15
   Leu Leu Leu Leu Leu Gly Val Ser Leu Gly Gly Ala Lys Glu Ala Cys
               20                  25                  30
   Pro Thr Gly Leu Tyr Thr His Ser Gly Glu Cys Cys Lys Ala Cys Asn
           35                  40                  45
   Leu Gly Glu Gly Val Ala Gln Pro Cys Gly Ala Asn Gln Thr Val Cys
       50                  55                  60
   Glu Pro Cys Leu Asp Ser Val Thr Phe Ser Asp Val Val Ser Ala Thr
   65                  70                  75                  80
   Glu Pro Cys Lys Pro Cys Thr Glu Cys Val Gly Leu Gln Ser Met Ser
                   85                  90                  95
   Ala Pro Cys Val Glu Ala Asp Asp Ala Val Cys Arg Cys Ala Tyr Gly
               100                 105                 110
   Tyr Tyr Gln Asp Glu Thr Thr Gly Arg Cys Glu Ala Cys Arg Val Cys
           115                 120                 125
   Glu Ala Gly Ser Gly Leu Val Phe Ser Cys Gln Asp Lys Gln Asn Thr
       130                 135                 140
   Val Cys Glu Glu Cys Pro Asp Gly Thr Tyr Ser Asp Glu Ala Asn His
   145                 150                 155                 160
   Val Asp Pro Cys Leu Pro Cys Thr Val Cys Glu Asp Thr Glu Arg Gln
                   165                 170                 175
   Leu Arg Glu Cys Thr Arg Trp Ala Asp Ala Glu Cys Glu Glu Ile Pro
   180 185 190
   Gly Arg Trp Ile Thr Arg Ser Thr Pro Pro Glu Gly Ser Asp Ser Thr
   195 200 205
   Ala Pro Ser Thr Gln Glu Pro Glu Ala Pro Pro Glu Gln Asp Leu Ile
   210 215 220
   Ala Ser Thr Val Ala Gly Val Val Thr Thr Val Met Gly Ser Ser Gln
   225 230 235 240
   Pro Val Val Thr Arg Gly Thr Thr Asp Asn Leu Ile Pro Val Tyr Cys
   245 250 255
   Ser Ile Leu Ala Ala Val Val Val Gly Leu Val Ala Tyr Ile Ala Phe
   260 265 270
   Lys Arg Trp Asn Ser Cys Lys Gln Asn Lys Gln Gly Ala Asn Ser Arg
   275 280 285
   Pro Val Asn Gln Thr Pro Pro Pro Glu Gly Glu Lys Leu His Ser Asp
   290 295 300
   Ser Gly Ile Ser Val Asp Ser Gln Ser Leu His Asp Gln Gln Pro His
   305 310 315 320
   Thr Gln Thr Ala Ser Gly Gln Ala Leu Lys Gly Asp Gly Gly Leu Tyr
   325 330 335
   Ser Ser Leu Pro Pro Ala Lys Arg Glu Glu Val Glu Lys Leu Leu Asn
   340 345 350
   Gly Ser Ala Gly Asp Thr Trp Arg His Leu Ala Gly Glu Leu Gly Tyr
   355 360 365
   Gln Pro Glu His Ile Asp Ser Phe Thr His Glu Ala Cys Pro Val Arg
   370 375 380
   Ala Leu Leu Ala Ser Trp Ala Thr Gln Asp Ser Ala Thr Leu Asp Ala
   385 390 395 400
   Leu Leu Ala Ala Leu Arg Arg Ile Gln Arg Ala Asp Leu Val Glu Ser
   405 410 415
   Leu Cys Ser Glu Ser Thr Ala Thr Ser Pro Val
   420 425
<210> 13
   <211> 6482
   <212> DNA
   <213> Artificial Sequence
<220>
   <223> Plasmid pcDNA4 (mycHis)A hRhoA wt
<220>
   <221> CDS
   <222> (1058)..(1636)
   <223> human RhoA sequence
<400> 13
   gatctcccga tcccctatgg tcgactctca gtacaatctg ctctgatgcc gcatagttaa 60
   gccagtatct gctccctgct tgtgtgttgg aggtcgctga gtagtgcgcg agcaaaattt 120
   aagctacaac aaggcaaggc ttgaccgaca attgcatgaa gaatctgctt agggttaggc 180
   gttttgcgct gcttcgcgat gtacgggcca gatatacgcg ttgacattga ttattgacta 240
   gttattaata gtaatcaatt acggggtcat tagttcatag cccatatatg gagttccgcg 300
   ttacataact tacggtaaat ggcccgcctg gctgaccgcc caacgacccc cgcccattga 360
   cgtcaataat gacgtatgtt cccatagtaa cgccaatagg gactttccat tgacgtcaat      420
   gggtggacta tttacggtaa actgcccact tggcagtaca tcaagtgtat catatgccaa      480
   gtacgccccc tattgacgtc aatgacggta aatggcccgc ctggcattat gcccagtaca      540
   tgaccttatg ggactttcct acttggcagt acatctacgt attagtcatc gctattacca      600
   tggtgatgcg gttttggcag tacatcaatg ggcgtggata gcggtttgac tcacggggat      660
   ttccaagtct ccaccccatt gacgtcaatg ggagtttgtt ttggcaccaa aatcaacggg      720
   actttccaaa atgtcgtaac aactccgccc cattgacgca aatgggcggt aggcgtgtac      780
   ggtgggaggt ctatataagc agagctctct ggctaactag agaacccact gcttactggc      840
   ttatcgaaat taatacgact cactataggg agacccaagc tggctagtta agcttggtac      900
   cgagctcgga tccactagtc cagtgtggtg gaattcggca cgaggctcgt tagtccacgg      960
   tctggtcttc agctacccgc cttcgtctcc gagtttgcga ctcgcggacc ggcgtccccg     1020
   gcgcgaagag gctggactcg gattcgttgc ctgagca atg gct gcc atc cgg aag     1075
                                           Met Ala Ala Ile Arg Lys
                                           1               5
   aaa ctg gtg att gtt ggt gat gga gcc tgt gga aag aca tgc ttg ctc     1123
   Lys Leu Val Ile Val Gly Asp Gly Ala Cys Gly Lys Thr Cys Leu Leu
               10                  15                  20
   ata gtc ttc agc aag gac cag ttc cca gag gtg tat gtg ccc aca gtg     1171
   Ile Val Phe Ser Lys Asp Gln Phe Pro Glu Val Tyr Val Pro Thr Val
           25                  30                  35
   ttt gag aac tat gtg gca gat atc gag gtg gat gga aag cag gta gag     1219
   Phe Glu Asn Tyr Val Ala Asp Ile Glu Val Asp Gly Lys Gln Val Glu
       40                  45                  50
   ttg gct ttg tgg gac aca gct ggg cag gaa gat tat gat cgc ctg agg     1267
   Leu Ala Leu Trp Asp Thr Ala Gly Gln Glu Asp Tyr Asp Arg Leu Arg
   55                  60                  65                  70
   ccc ctc tcc tac cca gat acc gat gtt ata ctg atg tgt ttt tcc atc     1315
   Pro Leu Ser Tyr Pro Asp Thr Asp Val Ile Leu Met Cys Phe Ser Ile
                   75                  80                  85
   gac agc cct gat agt tta gaa aac atc cca gaa aag tgg acc cca gaa     1363
   Asp Ser Pro Asp Ser Leu Glu Asn Ile Pro Glu Lys Trp Thr Pro Glu
               90                  95                  100
   gtc aag cat ttc tgt ccc aac gtg ccc atc atc ctg gtt ggg aat aag 1411
   Val Lys His Phe Cys Pro Asn Val Pro Ile Ile Leu Val Gly Asn Lys
   105 110 115
   aag gat ctt cgg aat gat gag cac aca agg cgg gag cta gcc aag atg 1459
   Lys Asp Leu Arg Asn Asp Glu His Thr Arg Arg Glu Leu Ala Lys Met
   120 125 130
   aag cag gag ccg gtg aaa cct gaa gaa ggc aga gat atg gca aac agg 1507
   Lys Gln Glu Pro Val Lys Pro Glu Glu Gly Arg Asp Met Ala Asn Arg
   135 140 145 150
   att ggc gct ttt ggg tac atg gag tgt tca gca aag acc aaa gat gga 1555
   Ile Gly Ala Phe Gly Tyr Met Glu Cys Ser Ala Lys Thr Lys Asp Gly
   155 160 165
   gtg aga gag gtt ttt gaa atg gct acg aga gct gct ctg caa gct aga 1603
   Val Arg Glu Val Phe Glu Met Ala Thr Arg Ala Ala Leu Gln Ala Arg
   170 175 180
   cgt ggg aag aaa aaa tct ggg tgc ctt gtc ttg tgaaaccttg ctgcaagcac 1656
   Arg Gly Lys Lys Lys Ser Gly Cys Leu Val Leu
   185 190
   agcccttatg cggttaattt tgaagtgctg tttattaatc ttagtgtatg attactggcc 1716
   tttttcattt atctataatt tacctaagat tacaaatcag aagtcatctt gctaccagta 1776
   tttagaagcc aactatgatt attaacgatg tccaacccgt ctggcccacc agggtccttt 1836
   tgacactgct ctaacagccc tcctctgcac tcccacctga cacaccaggc gctaattcaa 1896
   ggaatttctt aacttcttgc ttctttctag aaagagaaac agttggtaac ttttgtgaat 1956
   taggctgtaa ctactttata actaacatgt cctgcctatt atctgtcagc tgcaaggtac 2016
   tctggtgagt caccacttca gggctttact ccgtaacaga ttttgttggc atagctctgg 2076
   ggtgggcagt tttttgaaaa tgggctcaac cagaaaagcc caagttcatg cagctgtggc 2136
   agagttacag ttctgtggtt tcatgttagt taccttatag ttactgtgta attagtgcca 2196
   cttaatgtat gttaccaaaa ataaatatat ctaccccaga ctagatgtag tattttttgt 2256
   ataattggat ttcctaatac tgtcatcctc aaagaaagtg tattggtttt ttaaaaaaga 2316
   aagtgtattt ggaaataaag tcagatggaa aattcaaaaa aaaaaaaaaa aaaactcgag 2376
   tctagagggc ccttcgaaca aaaactcatc tcagaagagg atctgaatat gcataccggt 2436
   catcatcacc atcaccattg agtttaaacc cgctgatcag cctcgactgt gccttctagt 2496
   tgccagccat ctgttgtttg cccctccccc gtgccttcct tgaccctgga aggtgccact 2556
   cccactgtcc tttcctaata aaatgaggaa attgcatcgc attgtctgag taggtgtcat 2616
   tctattctgg ggggtggggt ggggcaggac agcaaggggg aggattggga agacaatagc 2676
   aggcatgctg gggatgcggt gggctctatg gcttctgagg cggaaagaac cagctggggc 2736
   tctagggggt atccccacgc gccctgtagc ggcgcattaa gcgcggcggg tgtggtggtt 2796
   acgcgcagcg tgaccgctac acttgccagc gccctagcgc ccgctccttt cgctttcttc 2856
   ccttcctttc tcgccacgtt cgccggcttt ccccgtcaag ctctaaatcg gggcatccct 2916
   ttagggttcc gatttagtgc tttacggcac ctcgacccca aaaaacttga ttagggtgat 2976
   ggttcacgta gtgggccatc gccctgatag acggtttttc gccctttgac gttggagtcc 3036
   acgttcttta atagtggact cttgttccaa actggaacaa cactcaaccc tatctcggtc 3096
   tattcttttg atttataagg gattttgggg atttcggcct attggttaaa aaatgagctg 3156
   atttaacaaa aatttaacgc gaattaattc tgtggaatgt gtgtcagtta gggtgtggaa 3216
   agtccccagg ctccccaggc aggcagaagt atgcaaagca tgcatctcaa ttagtcagca 3276
   accaggtgtg gaaagtcccc aggctcccca gcaggcagaa gtatgcaaag catgcatctc 3336
   aattagtcag caaccatagt cccgccccta actccgccca tcccgcccct aactccgccc 3396
   agttccgccc attctccgcc ccatggctga ctaatttttt ttatttatgc agaggccgag 3456
   gccgcctctg cctctgagct attccagaag tagtgaggag gcttttttgg aggcctaggc 3516
   ttttgcaaaa agctcccggg agcttgtata tccattttcg gatctgatca gcacgtgttg 3576
   acaattaatc atcggcatag tatatcggca tagtataata cgacaaggtg aggaactaaa 3636
   ccatggccaa gttgaccagt gccgttccgg tgctcaccgc gcgcgacgtc gccggagcgg 3696
   tcgagttctg gaccgaccgg ctcgggttct cccgggactt cgtggaggac gacttcgccg 3756
   gtgtggtccg ggacgacgtg accctgttca tcagcgcggt ccaggaccag gtggtgccgg 3816
   acaacaccct ggcctgggtg tgggtgcgcg gcctggacga gctgtacgcc gagtggtcgg 3876
   aggtcgtgtc cacgaacttc cgggacgcct ccgggccggc catgaccgag atcggcgagc 3936
   agccgtgggg gcgggagttc gccctgcgcg acccggccgg caactgcgtg cacttcgtgg 3996
   ccgaggagca ggactgacac gtgctacgag atttcgattc caccgccgcc ttctatgaaa 4056
   ggttgggctt cggaatcgtt ttccgggacg ccggctggat gatcctccag cgcggggatc 4116
   tcatgctgga gttcttcgcc caccccaact tgtttattgc agcttataat ggttacaaat 4176
   aaagcaatag catcacaaat ttcacaaata aagcattttt ttcactgcat tctagttgtg 4236
   gtttgtccaa actcatcaat gtatcttatc atgtctgtat accgtcgacc tctagctaga 4296
   gcttggcgta atcatggtca tagctgtttc ctgtgtgaaa ttgttatccg ctcacaattc 4356
   cacacaacat acgagccgga agcataaagt gtaaagcctg gggtgcctaa tgagtgagct 4416
   aactcacatt aattgcgttg cgctcactgc ccgctttcca gtcgggaaac ctgtcgtgcc 4476
   agctgcatta atgaatcggc caacgcgcgg ggagaggcgg tttgcgtatt gggcgctctt 4536
   ccgcttcctc gctcactgac tcgctgcgct cggtcgttcg gctgcggcga gcggtatcag 4596
   ctcactcaaa ggcggtaata cggttatcca cagaatcagg ggataacgca ggaaagaaca 4656
   tgtgagcaaa aggccagcaa aaggccagga accgtaaaaa ggccgcgttg ctggcgtttt 4716
   tccataggct ccgcccccct gacgagcatc acaaaaatcg acgctcaagt cagaggtggc 4776
   gaaacccgac aggactataa agataccagg cgtttccccc tggaagctcc ctcgtgcgct 4836
   ctcctgttcc gaccctgccg cttaccggat acctgtccgc ctttctccct tcgggaagcg 4896
   tggcgctttc tcaatgctca cgctgtaggt atctcagttc ggtgtaggtc gttcgctcca 4956
   agctgggctg tgtgcacgaa ccccccgttc agcccgaccg ctgcgcctta tccggtaact 5016
   atcgtcttga gtccaacccg gtaagacacg acttatcgcc actggcagca gccactggta 5076
   acaggattag cagagcgagg tatgtaggcg gtgctacaga gttcttgaag tggtggccta 5136
   actacggcta cactagaagg acagtatttg gtatctgcgc tctgctgaag ccagttacct 5196
   tcggaaaaag agttggtagc tcttgatccg gcaaacaaac caccgctggt agcggtggtt 5256
   tttttgtttg caagcagcag attacgcgca gaaaaaaagg atctcaagaa gatcctttga 5316
   tcttttctac ggggtctgac gctcagtgga acgaaaactc acgttaaggg attttggtca 5376
   tgagattatc aaaaaggatc ttcacctaga tccttttaaa ttaaaaatga agttttaaat 5436
   caatctaaag tatatatgag taaacttggt ctgacagtta ccaatgctta atcagtgagg 5496
   cacctatctc agcgatctgt ctatttcgtt catccatagt tgcctgactc cccgtcgtgt 5556
   agataactac gatacgggag ggcttaccat ctggccccag tgctgcaatg ataccgcgag 5616
   acccacgctc accggctcca gatttatcag caataaacca gccagccgga agggccgagc 5676
   gcagaagtgg tcctgcaact ttatccgcct ccatccagtc tattaattgt tgccgggaag 5736
   ctagagtaag tagttcgcca gttaatagtt tgcgcaacgt tgttgccatt gctacaggca 5796
   tcgtggtgtc acgctcgtcg tttggtatgg cttcattcag ctccggttcc caacgatcaa 5856
   ggcgagttac atgatccccc atgttgtgca aaaaagcggt tagctccttc ggtcctccga 5916
   tcgttgtcag aagtaagttg gccgcagtgt tatcactcat ggttatggca gcactgcata 5976
   attctcttac tgtcatgcca tccgtaagat gcttttctgt gactggtgag tactcaacca 6036
   agtcattctg agaatagtgt atgcggcgac cgagttgctc ttgcccggcg tcaatacggg 6096
   ataataccgc gccacatagc agaactttaa aagtgctcat cattggaaaa cgttcttcgg 6156
   ggcgaaaact ctcaaggatc ttaccgctgt tgagatccag ttcgatgtaa cccactcgtg 6216
   cacccaactg atcttcagca tcttttactt tcaccagcgt ttctgggtga gcaaaaacag 6276
   gaaggcaaaa tgccgcaaaa aagggaataa gggcgacacg gaaatgttga atactcatac 6336
   tcttcctttt tcaatattat tgaagcattt atcagggtta ttgtctcatg agcggataca 6396
   tatttgaatg tatttagaaa aataaacaaa taggggttcc gcgcacattt ccccgaaaag 6456
   tgccacctga cgtcgacgga tcggga 6482
<210> 14
   <211> 193
   <212> PRT
   <213> Artificial Sequence
<220>
   <223> Plasmid pcDNA4 (mycHis)A hRhoA wt
<400> 14
   Met Ala Ala Ile Arg Lys Lys Leu Val Ile Val Gly Asp Gly Ala Cys
   1 5 10 15
   Gly Lys Thr Cys Leu Leu Ile Val Phe Ser Lys Asp Gln Phe Pro Glu
   20 25 30
   Val Tyr Val Pro Thr Val Phe Glu Asn Tyr Val Ala Asp Ile Glu Val
   35 40 45
   Asp Gly Lys Gln Val Glu Leu Ala Leu Trp Asp Thr Ala Gly Gln Glu
   50 55 60
   Asp Tyr Asp Arg Leu Arg Pro Leu Ser Tyr Pro Asp Thr Asp Val Ile
   65 70 75 80
   Leu Met Cys Phe Ser Ile Asp Ser Pro Asp Ser Leu Glu Asn Ile Pro
   85 90 95
   Glu Lys Trp Thr Pro Glu Val Lys His Phe Cys Pro Asn Val Pro Ile
   100 105 110
   Ile Leu Val Gly Asn Lys Lys Asp Leu Arg Asn Asp Glu His Thr Arg
   115 120 125
   Arg Glu Leu Ala Lys Met Lys Gln Glu Pro Val Lys Pro Glu Glu Gly
   130 135 140
   Arg Asp Met Ala Asn Arg Ile Gly Ala Phe Gly Tyr Met Glu Cys Ser
   145 150 155 160
   Ala Lys Thr Lys Asp Gly Val Arg Glu Val Phe Glu Met Ala Thr Arg
   165 170 175
   Ala Ala Leu Gln Ala Arg Arg Gly Lys Lys Lys Ser Gly Cys Leu Val
   180 185 190
   Leu
<210> 15
   <211> 6853
   <212> DNA
   <213> Artificial Sequence
<220>
   <223> Plasmid pcDNA3 hRhoA wt
<220>
   <221> CDS
   <222> (127)..(705)
   <223> human RhoA sequence
<400> 15
   aattcggcac gaggctcgtt agtccacggt ctggtcttca gctacccgcc ttcgtctccg      60
   agtttgcgac tcgcggaccg gcgtccccgg cgcgaagagg ctggactcgg attcgttgcc     120
   tgagca atg gct gcc atc cgg aag aaa ctg gtg att gtt ggt gat gga       168
          Met Ala Ala Ile Arg Lys Lys Leu Val Ile Val Gly Asp Gly
          1               5                   10
   gcc tgt gga aag aca tgc ttg ctc ata gtc ttc agc aag gac cag ttc      216
   Ala Cys Gly Lys Thr Cys Leu Leu Ile Val Phe Ser Lys Asp Gln Phe
   15                  20                  25                  30
   cca gag gtg tat gtg ccc aca gtg ttt gag aac tat gtg gca gat atc      264
   Pro Glu Val Tyr Val Pro Thr Val Phe Glu Asn Tyr Val Ala Asp Ile
                   35                  40                  45
   gag gtg gat gga aag cag gta gag ttg gct ttg tgg gac aca gct ggg      312
   Glu Val Asp Gly Lys Gln Val Glu Leu Ala Leu Trp Asp Thr Ala Gly
               50                  55                  60
   cag gaa gat tat gat cgc ctg agg ccc ctc tcc tac cca gat acc gat       360
   Gln Glu Asp Tyr Asp Arg Leu Arg Pro Leu Ser Tyr Pro Asp Thr Asp
           65                  70                  75
   gtt ata ctg atg tgt ttt tcc atc gac agc cct gat agt tta gaa aac       408
   Val Ile Leu Met Cys Phe Ser Ile Asp Ser Pro Asp Ser Leu Glu Asn
       80                  85                  90
   atc cca gaa aag tgg acc cca gaa gtc aag cat ttc tgt ccc aac gtg       456
   Ile Pro Glu Lys Trp Thr Pro Glu Val Lys His Phe Cys Pro Asn Val
   95                  100                 105                 110
   ccc atc atc ctg gtt ggg aat aag aag gat ctt cgg aat gat gag cac       504
   Pro Ile Ile Leu Val Gly Asn Lys Lys Asp Leu Arg Asn Asp Glu His
                   115                 120                 125
   aca agg cgg gag cta gcc aag atg aag cag gag ccg gtg aaa cct gaa       552
   Thr Arg Arg Glu Leu Ala Lys Met Lys Gln Glu Pro Val Lys Pro Glu
               130                 135                 140
   gaa ggc aga gat atg gca aac agg att ggc gct ttt ggg tac atg gag       600
   Glu Gly Arg Asp Met Ala Asn Arg Ile Gly Ala Phe Gly Tyr Met Glu
           145                 150                 155
   tgt tca gca aag acc aaa gat gga gtg aga gag gtt ttt gaa atg gct       648
   Cys Ser Ala Lys Thr Lys Asp Gly Val Arg Glu Val Phe Glu Met Ala
       160                 165                 170
   acg aga gct gct ctg caa gct aga cgt ggg aag aaa aaa tct ggg tgc       696
   Thr Arg Ala Ala Leu Gln Ala Arg Arg Gly Lys Lys Lys Ser Gly Cys
   175                 180                 185                 190
   ctt gtc ttg tgaaaccttg ctgcaagcac agcccttatg cggttaattt               745
   Leu Val Leu
   tgaagtgctg tttattaatc ttagtgtatg attactggcc tttttcattt atctataatt     805
   tacctaagat tacaaatcag aagtcatctt gctaccagta tttagaagcc aactatgatt     865
   attaacgatg tccaacccgt ctggcccacc agggtccttt tgacactgct ctaacagccc     925
   tcctctgcac tcccacctga cacaccaggc gctaattcaa ggaatttctt aacttcttgc     985
   ttctttctag aaagagaaac agttggtaac ttttgtgaat taggctgtaa ctactttata    1045
   actaacatgt cctgcctatt atctgtcagc tgcaaggtac tctggtgagt caccacttca    1105
   gggctttact ccgtaacaga ttttgttggc atagctctgg ggtgggcagt tttttgaaaa    1165
   tgggctcaac cagaaaagcc caagttcatg cagctgtggc agagttacag ttctgtggtt 1225
   tcatgttagt taccttatag ttactgtgta attagtgcca cttaatgtat gttaccaaaa 1285
   ataaatatat ctaccccaga ctagatgtag tattttttgt ataattggat ttcctaatac 1345
   tgtcatcctc aaagaaagtg tattggtttt ttaaaaaaga aagtgtattt ggaaataaag 1405
   tcagatggaa aattcaaaaa aaaaaaaaaa aaaactcgag catgcatcta gagggcccta 1465
   ttctatagtg tcacctaaat gctagagctc gctgatcagc ctcgactgtg ccttctagtt 1525
   gccagccatc tgttgtttgc ccctcccccg tgccttcctt gaccctggaa ggtgccactc 1585
   ccactgtcct ttcctaataa aatgaggaaa ttgcatcgca ttgtctgagt aggtgtcatt 1645
   ctattctggg gggtggggtg gggcaggaca gcaaggggga ggattgggaa gacaatagca 1705
   ggcatgctgg ggatgcggtg ggctctatgg cttctgaggc ggaaagaacc agctggggct 1765
   ctagggggta tccccacgcg ccctgtagcg gcgcattaag cgcggcgggt gtggtggtta 1825
   cgcgcagcgt gaccgctaca cttgccagcg ccctagcgcc cgctcctttc gctttcttcc 1885
   cttcctttct cgccacgttc gccggctttc cccgtcaagc tctaaatcgg ggcatccctt 1945
   tagggttccg atttagtgct ttacggcacc tcgaccccaa aaaacttgat tagggtgatg 2005
   gttcacgtag tgggccatcg ccctgataga cggtttttcg ccctttgacg ttggagtcca 2065
   cgttctttaa tagtggactc ttgttccaaa ctggaacaac actcaaccct atctcggtct 2125
   attcttttga tttataaggg attttgggga tttcggccta ttggttaaaa aatgagctga 2185
   tttaacaaaa atttaacgcg aattaattct gtggaatgtg tgtcagttag ggtgtggaaa 2245
   gtccccaggc tccccaggca ggcagaagta tgcaaagcat gcatctcaat tagtcagcaa 2305
   ccaggtgtgg aaagtcccca ggctccccag caggcagaag tatgcaaagc atgcatctca 2365
   attagtcagc aaccatagtc ccgcccctaa ctccgcccat cccgccccta actccgccca 2425
   gttccgccca ttctccgccc catggctgac taattttttt tatttatgca gaggccgagg 2485
   ccgcctctgc ctctgagcta ttccagaagt agtgaggagg cttttttgga ggcctaggct 2545
   tttgcaaaaa gctcccggga gcttgtatat ccattttcgg atctgatcaa gagacaggat 2605
   gaggatcgtt tcgcatgatt gaacaagatg gattgcacgc aggttctccg gccgcttggg 2665
   tggagaggct attcggctat gactgggcac aacagacaat cggctgctct gatgccgccg 2725
   tgttccggct gtcagcgcag gggcgcccgg ttctttttgt caagaccgac ctgtccggtg 2785
   ccctgaatga actgcaggac gaggcagcgc ggctatcgtg gctggccacg acgggcgttc 2845
   cttgcgcagc tgtgctcgac gttgtcactg aagcgggaag ggactggctg ctattgggcg 2905
   aagtgccggg gcaggatctc ctgtcatctc accttgctcc tgccgagaaa gtatccatca 2965
   tggctgatgc aatgcggcgg ctgcatacgc ttgatccggc tacctgccca ttcgaccacc 3025
   aagcgaaaca tcgcatcgag cgagcacgta ctcggatgga agccggtctt gtcgatcagg 3085
   atgatctgga cgaagagcat caggggctcg cgccagccga actgttcgcc aggctcaagg 3145
   cgcgcatgcc cgacggcgag gatctcgtcg tgacccatgg cgatgcctgc ttgccgaata 3205
   tcatggtgga aaatggccgc ttttctggat tcatcgactg tggccggctg ggtgtggcgg 3265
   accgctatca ggacatagcg ttggctaccc gtgatattgc tgaagagctt ggcggcgaat 3325
   gggctgaccg cttcctcgtg ctttacggta tcgccgctcc cgattcgcag cgcatcgcct 3385
   tctatcgcct tcttgacgag ttcttctgag cgggactctg gggttcgaaa tgaccgacca 3445
   agcgacgccc aacctgccat cacgagattt cgattccacc gccgccttct atgaaaggtt 3505
   gggcttcgga atcgttttcc gggacgccgg ctggatgatc ctccagcgcg gggatctcat 3565
   gctggagttc ttcgcccacc ccaacttgtt tattgcagct tataatggtt acaaataaag 3625
   caatagcatc acaaatttca caaataaagc atttttttca ctgcattcta gttgtggttt 3685
   gtccaaactc atcaatgtat cttatcatgt ctgtataccg tcgacctcta gctagagctt 3745
   ggcgtaatca tggtcatagc tgtttcctgt gtgaaattgt tatccgctca caattccaca 3805
   caacatacga gccggaagca taaagtgtaa agcctggggt gcctaatgag tgagctaact 3865
   cacattaatt gcgttgcgct cactgcccgc tttccagtcg ggaaacctgt cgtgccagct 3925
   gcattaatga atcggccaac gcgcggggag aggcggtttg cgtattgggc gctcttccgc 3985
   ttcctcgctc actgactcgc tgcgctcggt cgttcggctg cggcgagcgg tatcagctca 4045
   ctcaaaggcg gtaatacggt tatccacaga atcaggggat aacgcaggaa agaacatgtg 4105
   agcaaaaggc cagcaaaagg ccaggaaccg taaaaaggcc gcgttgctgg cgtttttcca 4165
   taggctccgc ccccctgacg agcatcacaa aaatcgacgc tcaagtcaga ggtggcgaaa 4225
   cccgacagga ctataaagat accaggcgtt tccccctgga agctccctcg tgcgctctcc 4285
   tgttccgacc ctgccgctta ccggatacct gtccgccttt ctcccttcgg gaagcgtggc 4345
   gctttctcaa tgctcacgct gtaggtatct cagttcggtg taggtcgttc gctccaagct 4405
   gggctgtgtg cacgaacccc ccgttcagcc cgaccgctgc gccttatccg gtaactatcg 4465
   tcttgagtcc aacccggtaa gacacgactt atcgccactg gcagcagcca ctggtaacag 4525
   gattagcaga gcgaggtatg taggcggtgc tacagagttc ttgaagtggt ggcctaacta 4585
   cggctacact agaaggacag tatttggtat ctgcgctctg ctgaagccag ttaccttcgg 4645
   aaaaagagtt ggtagctctt gatccggcaa acaaaccacc gctggtagcg gtggtttttt 4705
   tgtttgcaag cagcagatta cgcgcagaaa aaaaggatct caagaagatc ctttgatctt 4765
   ttctacgggg tctgacgctc agtggaacga aaactcacgt taagggattt tggtcatgag 4825
   attatcaaaa aggatcttca cctagatcct tttaaattaa aaatgaagtt ttaaatcaat 4885
   ctaaagtata tatgagtaaa cttggtctga cagttaccaa tgcttaatca gtgaggcacc 4945
   tatctcagcg atctgtctat ttcgttcatc catagttgcc tgactccccg tcgtgtagat 5005
   aactacgata cgggagggct taccatctgg ccccagtgct gcaatgatac cgcgagaccc 5065
   acgctcaccg gctccagatt tatcagcaat aaaccagcca gccggaaggg ccgagcgcag 5125
   aagtggtcct gcaactttat ccgcctccat ccagtctatt aattgttgcc gggaagctag 5185
   agtaagtagt tcgccagtta atagtttgcg caacgttgtt gccattgcta caggcatcgt 5245
   ggtgtcacgc tcgtcgtttg gtatggcttc attcagctcc ggttcccaac gatcaaggcg 5305
   agttacatga tcccccatgt tgtgcaaaaa agcggttagc tccttcggtc ctccgatcgt 5365
   tgtcagaagt aagttggccg cagtgttatc actcatggtt atggcagcac tgcataattc 5425
   tcttactgtc atgccatccg taagatgctt ttctgtgact ggtgagtact caaccaagtc 5485
   attctgagaa tagtgtatgc ggcgaccgag ttgctcttgc ccggcgtcaa tacgggataa 5545
   taccgcgcca catagcagaa ctttaaaagt gctcatcatt ggaaaacgtt cttcggggcg 5605
   aaaactctca aggatcttac cgctgttgag atccagttcg atgtaaccca ctcgtgcacc 5665
   caactgatct tcagcatctt ttactttcac cagcgtttct gggtgagcaa aaacaggaag 5725
   gcaaaatgcc gcaaaaaagg gaataagggc gacacggaaa tgttgaatac tcatactctt 5785
   cctttttcaa tattattgaa gcatttatca gggttattgt ctcatgagcg gatacatatt 5845
   tgaatgtatt tagaaaaata aacaaatagg ggttccgcgc acatttcccc gaaaagtgcc 5905
   acctgacgtc gacggatcgg gagatctccc gatcccctat ggtcgactct cagtacaatc 5965
   tgctctgatg ccgcatagtt aagccagtat ctgctccctg cttgtgtgtt ggaggtcgct 6025
   gagtagtgcg cgagcaaaat ttaagctaca acaaggcaag gcttgaccga caattgcatg 6085
   aagaatctgc ttagggttag gcgttttgcg ctgcttcgcg atgtacgggc cagatatacg 6145
   cgttgacatt gattattgac tagttattaa tagtaatcaa ttacggggtc attagttcat 6205
   agcccatata tggagttccg cgttacataa cttacggtaa atggcccgcc tggctgaccg 6265
   cccaacgacc cccgcccatt gacgtcaata atgacgtatg ttcccatagt aacgccaata 6325
   gggactttcc attgacgtca atgggtggac tatttacggt aaactgccca cttggcagta 6385
   catcaagtgt atcatatgcc aagtacgccc cctattgacg tcaatgacgg taaatggccc 6445
   gcctggcatt atgcccagta catgacctta tgggactttc ctacttggca gtacatctac 6505
   gtattagtca tcgctattac catggtgatg cggttttggc agtacatcaa tgggcgtgga 6565
   tagcggtttg actcacgggg atttccaagt ctccacccca ttgacgtcaa tgggagtttg 6625
   ttttggcacc aaaatcaacg ggactttcca aaatgtcgta acaactccgc cccattgacg 6685
   caaatgggcg gtaggcgtgt acggtgggag gtctatataa gcagagctct ctggctaact 6745
   agagaaccca ctgcttactg gcttatcgaa attaatacga ctcactatag ggagacccaa 6805
   gcttggtacc gagctcggat ccactagtaa cggccgccag tgtgctgg 6853
<210> 16
   <211> 193
   <212> PRT
   <213> Artificial Sequence
<220>
   <223> Plasmid pcDNA3 hRhoA wt
<400> 16
   Met Ala Ala Ile Arg Lys Lys Leu Val Ile Val Gly Asp Gly Ala Cys
   1 5 10 15
   Gly Lys Thr Cys Leu Leu Ile Val Phe Ser Lys Asp Gln Phe Pro Glu
   20 25 30
   Val Tyr Val Pro Thr Val Phe Glu Asn Tyr Val Ala Asp Ile Glu Val
   35 40 45
   Asp Gly Lys Gln Val Glu Leu Ala Leu Trp Asp Thr Ala Gly Gln Glu
   50 55 60
   Asp Tyr Asp Arg Leu Arg Pro Leu Ser Tyr Pro Asp Thr Asp Val Ile
   65 70 75 80
   Leu Met Cys Phe Ser Ile Asp Ser Pro Asp Ser Leu Glu Asn Ile Pro
   85 90 95
   Glu Lys Trp Thr Pro Glu Val Lys His Phe Cys Pro Asn Val Pro Ile
   100 105 110
   Ile Leu Val Gly Asn Lys Lys Asp Leu Arg Asn Asp Glu His Thr Arg
   115 120 125
   Arg Glu Leu Ala Lys Met Lys Gln Glu Pro Val Lys Pro Glu Glu Gly
   130 135 140
   Arg Asp Met Ala Asn Arg Ile Gly Ala Phe Gly Tyr Met Glu Cys Ser
   145 150 155 160
   Ala Lys Thr Lys Asp Gly Val Arg Glu Val Phe Glu Met Ala Thr Arg
   165 170 175
   Ala Ala Leu Gln Ala Arg Arg Gly Lys Lys Lys Ser Gly Cys Leu Val
   180 185 190
   Leu